# FASCICULE DE BREVET EUROPEEN

(11) **EP 1 252 171 B1**
(45) Date de publication et mention de la délivrance du brevet: **15.08.2007**
(21) Numéro de dépôt: 01904046.8
(22) Date de dépôt: 02.02.2001
(51) Int. Cl.: C07H 5/10, C07H 13/02, C07H 15/04, C07H 1/00

(54) **PROCEDE POUR LA PREPARATION DE DERIVES FONCTIONNALISES DE BETA-(1,3)-GLUCANES**
VERFAHREN ZUR HERSTELLUNG VON FUNKTIONALISIERTEN BETA-(1,3)-GLUCANDERIVATEN
METHOD FOR PREPARING FUNCTIONALISED BETA-(1,3)-GLUCAN DERIVATIVES

(30) Priorité: 04.02.2000 FR 0001429
(43) Date de publication de la demande: 30.10.2002
(73) Titulaire: LABORATOIRES GOEMAR S.A., 35403 Saint-Malo (FR)
(72) Inventeur: YVIN, Jean-Claude, F-35400 Saint-Malo (FR); JAMOIS, Frank, F-35000 Rennes (FR); FERRIERES, Vincent, 35140 Saint Aubin du Cormier (FR); PLUSQUELLEC, Daniel, F-35230 Noyal Châtillon sur Seiche (FR)
(74) Mandataire: Hubert, Philippe
(86) Numéro de dépôt international: PCT/FR2001/000329
(87) Numéro de publication internationale: WO 2001/057053

(56) Documents cités:
- K. TAKEO ET AL.: "Synthesis of the Laminara-oligosaccharide beta-glycosides of dp 3-8" CARBOHYDRATE RESEARCH, vol. 245, - 1993 pages 81-96, XP002150503
- P.M. COLLINS, H. ALI: "A new cycloglucohexaose derivative the chemical synthesis of cyclo{-3-[beta-D-Glcp-(1-3)-]5-D-Glcp-(1-} " TETRAHEDRON LETTERS, vol. 31, no. 31, 1990, pages 4517-4520, XP002150504
- H. PAULSEN ET AL.: "Synthese von verzweigten Tetrasaccharid- und Pentasaccharide-Strukturen von N-Glycoproteinen, methyliert an 4'-OH des Verzweigungsgliedes" LIEBIGS ANN. CHEM., 1992, pages 1303-1313, XP002150505
- ZIEGLER T ET AL: "Intramolecular Glycosylation of Prearranged Glycosides Part 5. alpha-(1->4)-Selective Glucosylation of Glucosides and Glucosamines" TETRAHEDRON LETTERS,NL,ELSEVIER SCIENCE PUBLISHERS, AMSTERDAM, vol. 38, no. 21, 26 mai 1997 (1997-05-26), pages 3715-3718, XP004064018 ISSN: 0040-4039
- P. FÜGEDI ET AL.: "Synthesis of a branched heptasaccharide having phytoalexin-elicitor activity" CARBOHYDRATE RESEARCH, vol. 164, 1987, pages 297-312, XP002170062
- P.J. GAREGG ET AL.: "Synthesis of methyl (ethyl 2-O-acyl-3,4-di-O-benzyl-1-thio-beta-D-glu copyranosid)uronates and evaluation of their use as reactive beta-selective glucuronic acid donore" J. ORG. CHEM., vol. 60, 1995, pages 2200-2204, XP002170063
- XIA J ET AL: "Use of 1,2-dichloro 4,5-dicyanoquinone (DDQ) for cleavage of the 2-naphthylmethyl (NAP) group" TETRAHEDRON LETTERS,NL,ELSEVIER SCIENCE PUBLISHERS, AMSTERDAM, vol. 41, no. 2, janvier 2000 (2000-01), pages 169-173, XP004185441 ISSN: 0040-4039

## Description

La présente invention concerne d'une façon générale un nouveau procédé de préparation par voie chimique de dérivés fonctionnalisés de β-(1,3)-glucanes, permettant d accéder à des oligosaccharides libres ou comportant des groupements spécifiques en des positions préalablement définies.

La présente invention trouve notamment application pour la préparation de composés biologiquement actifs utilisables dans le domaine agricole, cosmétique ou pharmaceutique.

On sait que de nombreux oligosaccharides présentent une activité biologique généralement liée à la présence de groupements spécifiques (c'est-à-dire non hydroxyles), tels que par exemple des groupements sulfate, phosphate, méthyle... sur des positions bien définies.

L'activité biologique est également liée, dans certains cas, à la longueur de l'oligosaccharide.

Les oligosaccharides libres biologiquement actifs sont habituellement obtenus par hydrolyse ou acétolyse de polysaccharides naturels d'origine végétale et se présentent généralement sous la forme de mélanges complexes extrêmement difficiles à purifier.

On a déjà proposé, dans le document FR 98.04610 (FR 2777281) un procédé de préparation par voie chimique du disaccharide libre communément appelé Laminaribiose, connu pour ses diverses activités biologiques.

Ce procédé qui permet d'obtenir un rendement global élevé en Laminaribiose, et qui comprend généralement la réaction entre un donneur de glycosyle et un accepteur de glycosyle, est essentiellement caractérisé par un choix judicieux de ces composés, ainsi que du promoteur utilisé lors de la réaction de couplage.

Cependant, ce procédé est spécifique du seul Laminaribiose.

Les oligosaccharides biologiquement actifs comportant des groupements spécifiques sont généralement obtenus à partir d'oligosaccharides libres qui conduisent inévitablement à des mélanges complexes difficiles à purifier, y compris dans le cas où l'oligosaccharide de départ est de structure parfaitement définie. D'une façon générale, plus l'oligosaccharide est long et plus le mélange obtenu est complexe et difficile à purifier.

La préparation par voie chimique de dérivés de β-(1,3)-glucanes sélectivement fonctionnalisés permettant d'accéder à des oligosaccharides libres ou comportant des groupements spécifiques en des positions préalablement définies se heurte notamment à la difficulté de différencier les fonctions hydroxyles tant au niveau de chaque unité monosaccharidique, et plus particulièrement au niveau des positions 2 et 3, qu'au niveau du polysaccharide.

Des procédés permettant de réaliser une liaison β-(1,3) entre des unités glucidiques par réaction entre un donneur et un accepteur de glycosyle ont notamment été décrits dans les documents CARBOHYDRATE RESEARCH, vol. 245; 1993 pages 81-96 ; TETRAHEDRON, 1993, Vol. 49, no. 33, pages 7301 à 7316 et JOURNAL OF ORGANIC CHEMISTRY, 1993, Vol. 58, pages 1090 à 1099.

Le document CARBOHYDRATE RESEARCH, vol. 245, 1993 pages. 81-96 est le seul, parmi les documents précités, qui concerne exclusivement la synthèse de dérivés de β-(1,3)-glucane. Le procédé de synthèse de décrit dans ce document antérieur fait intervenir des composés très différents des composés utilisés dans le cadre de la présente invention et ne permet en aucun cas d'accéder à des dérivés variés sous forme sensiblement pure.

Le document TETRAHEDRON, 1993, Vol. 49, no. 33, pages 7301 à 7316 concerne la synthèse de composés très particuliers, comportant essentiellement des liaisons de type β-(1,6).

La formation d'une liaison β-(1,3) également envisagée dans ce document antérieur fait appel à un donneur de glycosyle (composé 17, page 7304) qui est un imidate (composé de type Ib selon l'invention) dont tous les groupements protecteurs sont identiques.

Cet enseignement n'est donc d'aucune façon susceptible d'apporter une quelconque solution au problème de la fonctionnalisation et de la différenciation des groupements présents sur les diverses positions de l'unité glucosidique.

Le document JOURNAL OF ORGANIC CHEMISTRY, 1993, Vol. 58, pages 1090 à 1099 concerne également la préparation d'un composé très spécifique (composé de formule 45) qui comporte divers types de liaison, et en particulier des liaisons β-(1,4) et α-(1,1).

La réalisation de la liaison β-(1,3) décrite dans ce document antérieur est également réalisée avec des composés différents de ceux utilisés dans le cadre de la présente invention.

Les documents LIEBIGS ANN. CHEM., 1992, pages 1303-1313 ; J. ORG. CHEM., vol. 60,1995, pages 2200-2204 ; JOURNAL OF CHEMICAL SOCIETY PERKIN TRANS I, 1998, Vol., no. 10 pages 1699 à 1704 ; CARBOHYDRATE RESEARCH, 1993, Vol. 246, pages 161 à 172 ; TETRAHEDRON, 1993, Vol. 49, no. 33, pages 7301 à 7316 ; TETRAHEDRON LETTERS, 1996, Vol. 37, no. 48, pages 8795 à 8798 ; CARBOHYDRATE RESEARCH, 1998, Vol. 311, pages 171 à 181. et JOURNAL OF ORGANIC CHEMISTRY, 1993, Vol. 58, pages 1090 à 1099 révèlent des composés qui ne sont pas utilisés pour la synthèse de dérivés de β-(1,3)-glucane, mais dont les structures chimiques présentent des analogies plus ou moins marquées avec les synthons donneur de glycosyle utilisés dans le cadre de la présente invention.

Tous les composés décrits dans ces documents se différencient des composés revendiqués de formule (**Ia**) en ce qu'ils portent en position 3 un groupe benzyle ou paraméthoxybenzyle.

Le composé 27 du document LIEBIGS ANN. CHEM., 1992, pages 1303-1313, à savoir le 2-O-acétyl-3-*O*-allyl-*4*,*6*-benzylidène-α-*D-*glucopyranosyltrichloroacétimidate, est utilisé pour créer une liaison β-(1-4) avec un composé (28) qui comporte en position 1, 3 et 6 des groupements protecteurs identiques, de sorte que le disaccharide résultant de l'association des composés 27 et 28 ne peut en aucun cas être fonctionnalisé.

Le composé 15 du document J. CARBOHYDRATE CHEMISTRY (1989), Vol. 8, pages 629 à 644 se différencie des composés revendiqués de formule (la) en ce qu'il porte en position 3 un groupe méthyle. En outre, ce composé est utilisé selon ce document antérieur pour créer une liaison β-(1-4) en association avec un accepteur de glycosyle tout à fait distinct des composés décrits dans cette fonction selon l'invention.

Des composés présentant des structures chimiques plus éloignées de celles des synthons donneurs de glycosyle utilisés dans le cadre de la présente invention sont décrits dans les documents CARBOHYDRATE LETTERS (1994), vol. 1, pages 47-54 et CARBOHYDRATE RESEARCH (1999), vol. 317, pages 63-84. Les composés décrits dans ces deux documents sont effet dérivés de L-idopyranose.

Les documents CARBOHYDRATE RESEARCH, vol. 164, 1987, pages 297-312 et SYNLETT, 1997, pages 1397 à 1399 révèlent des composés qui ne sont pas utilisés pour la synthèse de dérivés de β-(1,3)-glucane, mais dont les structures chimiques présentent des analogies plus ou moins marquées avec les synthons accepteurs de glycosyle utilisés dans le cadre de la présente invention.

Les composés révélés par ces documents antérieurs se différencient des composés revendiqués de formule (II) en ce qu'ils comportent tous un groupement (correspondant au groupement Y) de type -S-R, dans lequel R représente un groupe méthyle (composé 4 de CARBOHYDRATE RESEARCH, vol. 164, 1987, pages 297-312) éthyle (composé 16 de SYNLETT, 1997, pages 1397 à 1399) ou phényle (composé 17 de SYNLETT, 1997, pages 1397 à 1399).

Des composés présentant des structures chimiques relativement éloignées de celles des synthons accepteurs de glycosyle utilisés dans le cadre de la présente invention sont décrits dans les documents CARBOHYDRATE RESEARCH (1983), vol. 122, pages 69-79 et BIOORGANIC & MEDICINAL CHEMISTRY (1996), vol. 4, pages 1963-1977.

Ainsi, le composé N°3 révélé à la page 71 du document CARBOHYDRATE RESEARCH (1983), vol. 122, pages 69-79 est un manopyranoside de configuration anomérique alpha, à la différence des composés de formule (II) selon l'invention qui sont dérivés de D-glucopyranose de configuration anomérique bêta. Il en est de même des composés 22 et 25 décrits à la page 1966 dans le document BIOORGANIC & MEDICINAL CHEMISTRY (1996), vol. 4, pages 1963-1977.

Il existe donc un besoin non satisfait à ce jour, de disposer d'un procédé général permettant la préparation de dérivés fonctionnalisés de β-(1,3)-glucanes variés, présentant un nombre limité d'étapes, compatible avec un vaste choix de groupes protecteurs de différents types, d'une mise en oeuvre aisée et permettant d'obtenir les produits recherchés sous forme pure.

L'expression "dérivés sélectivement fonctionnalisés" utilisée dans le cadre de la présente description vise donc à couvrir des composés dont chaque position peut être sélectivement transformée par voie chimique en un groupement spécifique tel que par exemple un groupement sulfate, phosphate ou méthyle.

Il a été découvert, et ceci constitue le fondement de la présente invention, qu'il était possible de répondre à ce besoin par l'utilisation de donneurs de glycosyle et d'accepteurs de glycosyle originaux susceptibles en outre d'être préparés par une seule et même voie de synthèse à partir du glucose.

Ainsi, selon un premier aspect, la présente invention a pour objet un procédé pour la préparation de dérivés fonctionalisés de β-(1,3)-glucanes comprenant une réaction entre un donneur de glycosyle et un accepteur de glycosyle, caractérisé en ce que le donneur de glycosyle est choisi dans le groupe constitué des composés de formules générales **Ia** et **Ib:** 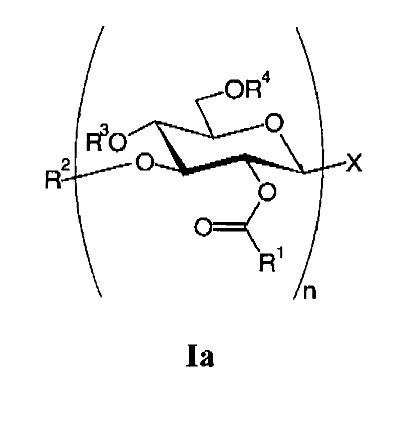 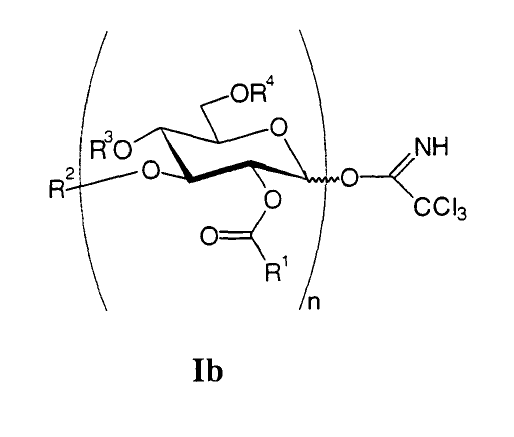 dans lesquelles :
X représente un groupe partant choisi parmi :
   - un groupement de formule S(O)ₚRₐ, dans laquelle Rₐ représente un radical alkyle ayant 1 à 18 atomes de carbone, un radical 1, 1-dicyclohexylméthyle, un radical aryle non substitué ou substitué par un groupement alkyle ou alcoxy ayant de 1 à 6 atomes de carbone, un groupement nitro ou acétamide et p est un nombre entier égal à 0 ou 1 :
R¹ représente :
   - un radical alkyle, halogénoalkyle ou cétoalkyle ayant de 1 à 6 atomes de carbone :

   - un radical aryle non substitué ou substitué par un ou plusieurs groupements choisis parmi un atome d'halogène, un radical alcoxy ayant de 1 à 6 atomes de carbone ou un groupement nitro ;
R³ et R⁴, différents de -CO-R¹ et de R², représentent indépendamment un radical benzyle, chlorobenzyle, méthoxybenzyle, nitrobenzyle, allyle, méthylnaphtyle, chloroacétyle, trialkylsilyle ou triarylméthyle;
   ou forment ensemble un radical éthylidyle, isopropylidyle, hexafluoroisopropylidyle, cyclopentylidyle, cyclohexylidyle, cycloheptylidyle, butylidyle, 1-tertiobutyléthylidyle, benzylidyle, méthoxybenzylidyle, 1-phénylbenzylidyle.
R² représente :
   - un groupement différent de -COR¹ et choisi parmi un radical méthyle, allyle, méthylnaphthyle, benzyle, paraméthoxybenzyle ;
n est un nombre entier compris entre 1 et 4 ; étant précisé que dans le cas où n est supérieur à 1, -COR¹, R³ et R⁴ peuvent être différents d'une unité glucosyle à l'autre ;
et en ce que l'accepteur de glycosyle est choisi dans le groupe constitué des composés de formule générale **II**: 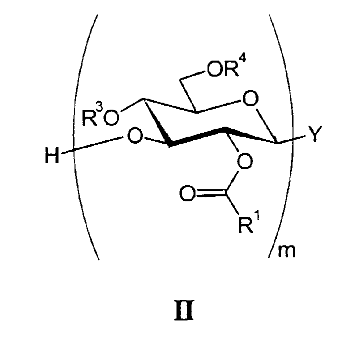 dans laquelle :
Y représente un groupe choisi parmi :
   - un groupement de formule -O-R_{b} dans laquelle R_{b} représente un radical alkyle ayant de 1 à 24 atomes de carbone, alcényle ayant de 2 à 24 atomes de carbone ou un radical arylalkylaryle ou arylalkyle ayant de 6 à 18 atomes de carbone ;
   - un résidu de sérine ou de thréonine ;
   - un résidu de stérol ;
   - un résidu de glycérolipide ;
   - un groupement de formule -S- Rₐ dans laquelle Rₐ est tel que défini précédemment ;
R¹, R³ et R⁴ sont tels que définis précédemment et ;
m est un nombre entier compris entre 1 et 8, étant précisé que dans le cas
où m est supérieur à 1, -COR¹, R³ et R⁴ peuvent être différents d'une unité glucosyle à l'autre.

Dans la description et les revendications, on entend :
- par radical alkyle, toute chaîne hydrocarbonée, linéaire ou ramifiée, un radical ayant de 1 à 6 atomes de carbone étant par exemple un radical méthyle, éthyle, propyle, isopropyle, butyle, isobutyle, tertiobutyle, pentyle, isopentyle, hexyle, isohexyle ;
- par radical alcényle, toute chaîne hydrocarbonée, linéaire ou ramifiée, comportant une double liaison :
- par radical alcoxy, tout radical de formule -O-R dans laquelle R est un radical alkyle tel que défini ci-dessus ;
- par radical halogénoalkyle ayant de 1 à 6 atomes de carbone, tout radical alkyle dont 1 à 7 atomes d'hydrogène ont été substitués par 1 à 7 atomes d'halogène, comme par exemple un radical chlorométhyle, un radical bromométhyle, un radical trifluorométhyle, un radical trifluoro-2,2,2-éthyle, un radical pentafluoroéthyle, un radical heptafluoropropyle ;
- par radical aryle, un cycle aromatique ayant 5 ou 6 atomes de carbone ou hétéroatomes, comme par exemple un radical phényle, pyridyle, thiényle, furannyle, pyrimidyle.

Le procédé selon l'invention est particulièrement intéressant puisqu'il permet d'accéder aisément à une multitude de composés sélectivement fonctionnalisés dans des positions prédéterminées.

Les groupements spécifiques susceptibles d'être introduits dans ces positions prédéterminées selon le procédé de l'invention peuvent être de nature variée et seront généralement des groupements sulfate, phosphate, méthyle, notamment dans les positions 2, 3, 4 et 6. Ce procédé permet également de fixer des résidus originaux particulièrement intéressants d'un point de vue biologique sur la position anomérique de l'unité réductrice de l'oligosaccharide notamment dans le cas où Y représente un résidu de sérine ou de thréonine, de stérol, de glycérolipide.

Selon une caractéristique particulière du procédé selon l'invention, le donneur de glycosyle précité est choisi dans le groupe constitué des composés de formules générales (Ia) ou (Ib) précitées dans lesquelles :
X représente un groupe partant choisi parmi :
   - un groupement de formule S(O)ₚRₐ, dans laquelle Rₐ représente un radical alkyle ayant 1 à 5 atomes de carbone, un radical aryle non substitué, de préférence un radical phényle ou un radical aryle substitué par un groupement alkyle ayant de 1 à 6 atomes de carbone, de préférence un radical toluyle, et p est un nombre entier égal à 0 ou 1 ;
R¹ représente :
   - un radical alkyle ayant de 1 à 6 atomes de carbone, de préférence méthyle, ou un groupement lévulinyle ;
   - un radical aryle non substitué, de préférence un radical phényle ;
R³ et R⁴, différents de -CO-R¹ et de R², représentent indépendamment un radical benzyle, chlorobenzyle, méthoxybenzyle, nitrobenzyle, allyle, méthylnaphtyle, chloroacétyle, trialkylsilyle ou triarylméthyle;
   ou forment ensemble un radical éthylidyle, isopropylidyle, benzylidyle ;
R² représente :
   - un groupement différent de -COR¹ et choisi parmi un radical méthyle, allyle, méthylnaphthyle, benzyle, paraméthoxybenzyle ;
   n est un nombre entier égal à 1, 2 ou 3; étant précisé que dans le cas où n est égal à 2 ou 3, -COR¹, R³ et R⁴ peuvent être différents d'une unité glucosyle à l'autre.

Avantageusement, le donneur de glycosyle précité est choisi dans le groupe constitué des composés de formules générales (Ia) ou (Ib) précitées dans lesquelles :
X représente un groupe partant choisi parmi:
   - un groupement de formule SRₐ dans laquelle Rₐ représente un radical éthyle, propyle, butyle, phényle ou toluyle, de préférence un radical éthyle ou phényle ;
R¹ représente un radical méthyle ou phényle;
R³ et R⁴ différents de -CO-R¹ et de R² représentent indépendamment un radical benzyle, méthoxybenzyle
   ou forment ensemble un radical benzylidyle;
R² représente un groupement différent de -COR¹ et choisi parmi un radical allyle, méthylnaphtyle, de préférence un radical allyle ou méthlylnaphtyle;
n est un nombre entier égal à 1 ou 2; de préférence égal à 1 ; étant précisé que dans le cas où n est égal à 2, -COR¹, R³ et R⁴ peuvent être différents d'une unité glucosyle à l'autre.

Selon une autre caractéristique particulière du procédé selon l'invention, l'accepteur de glycosyle précité est choisi dans le groupe constitué des composés de formule générale (II) précitée dans laquelle :
Y représente un groupe choisi parmi :
   - un groupement de formule -O-R_{b} dans laquelle R_{b} représente un radical alkyle ayant de 1 à 24 atomes de carbone, alcényle ayant de 2 à 24 atomes de carbone ou un radical arylalkylaryle ou arylalkyle ayant de 6 à 18 atomes de carbone ;
   - un résidu de sérine ou de thréonine ;
   - un résidu de stérol ;
   - un résidu de glycérolipide ;
   - un groupement de formule -S- Rₐ dans laquelle Rₐ représente un radical alkyle ayant 1 à 5 atomes de carbone, un radical aryle non substitué, de préférence un radical phényle ou un radical aryle substitué par un groupement alkyle ayant de 1 à 6 atomes de carbone ;
R¹ représente :
   - un radical alkyle ayant de 1 à 6 atomes de carbone, de préférence méthyle ;
   - un radical aryle non substitué, de préférence un radical phényle ;
R³ et R⁴, différents de -CO-R¹ et de R², représentent indépendamment un radical benzyle, chlorobenzyle, méthoxybenzyle, nitrobenzyle, allyle, méthylnaphtyle, chloroacétyle, trialkylsilyle ou triarylméthyle;
ou forment ensemble un radical éthylidyle, isopropylidyle, benzylidyle ;ou forment ensemble un radical éthylidyle, isopropylidyle, benzylidyle ;
m est un nombre entier compris entre 1 et 8, étant précisé que dans le cas où m est supérieur à 1, -COR¹, R³ et R⁴ peuvent être différents d'une unité glucosyle à l'autre.

Avantageusement, l'accepteur de glycosyle précité est choisi dans le groupe constitué des composés de formule générale (II) précitée dans laquelle l'une au moins des conditions suivantes est réalisée :
Y représente un groupe choisi parmi :
   - un groupement de formule -O-R_{b} dans laquelle R_{b} représente un radical alkyle ayant de 1 à 24 atomes de carbone, alcényle ayant de 2 à 24 atomes de carbone ou un radical benzyle ;
R¹ représente un radical méthyle ou phényle ;
R³ et R⁴ différents de -CO-R¹ représentent indépendamment un radical benzyle, methoxybenzyle
   ou forment ensemble un radical benzylidyle ;
m est un nombre entier compris entre 1 et 8, étant précisé que dans le cas où m est supérieur à 1, -COR¹, R³ et R⁴ peuvent être différents d'une unité glucosyle à l'autre.

Comme on le comprend, le procédé selon l'invention comporte une ou plusieurs étapes de couplage, selon le nombre d'unités de l'oligosaccharide.

D'une façon générale, chaque étape de couplage entre un donneur de glycosyle et un accepteur de glycosyle sera réalisée en solution dans un solvant organique anhydre à une température comprise entre -80°C et 40°C, pendant une durée de 1 minute à 8 heures en présence d'un promoteur approprié choisi parmi :
- une source d'ions halonium, associée ou non à un acide de Lewis ou un sel d'acide fort dans le cas des composés de formule générale (Ia) dans laquelle X représente un groupement S(O)ₚRₐ tel que défini ci-dessus et dans lequel p est égal à 0 ;
- un acide de Lewis associé à une amine, dans le cas des composés de formule générale (Ia) dans laquelle X représente un groupement S(O)ₚRₐ tel que défini ci-dessus et dans lequel p est égal à 1 ;
- un acide de Brönstedt ou un acide de Lewis, dans le cas des composés de formule (Ib).

La nature chimique du promoteur, les quantités respectives de donneur de glycosyle, d'accepteur de glycosyle et de promoteur ainsi que les conditions réactionnelles de chaque étape de couplage pourront être facilement déterminées par l'homme de métier qui pourra se reporter notamment à la description du document FR 98.04610.

Les composés de formule (Ia), (Ib) et (II) pourront être préparés par diverses voies de synthèse connues dans la chimie des sucres.

Avantageusement, et ceci constitue une caractéristique originale de l'invention, tous ces composés pourront être préparés par une voie de synthèse unique à partir du glucose, ce qui constitue un avantage particulièrement intéressant du point de vue industriel.

Cette voie de synthèse, illustrée ci-dessous par le schéma réactionnel (I) dans le cas des monosaccharides, comprend dans l'ordre suivant :
- la préparation 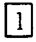 d'un dérivé du glucose sous forme furanosique (X) dont les positions 1.2 et 5,6 sont protégées par exemple par des groupements acétals ;
- la fonctionnalisation sélective 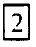 du composé ainsi obtenu en position 3 par un groupement correspondant au groupement R² défini précédemment pour former un composé (IX);
- le clivage 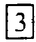 des groupements protecteurs des positions 1,2 et 5,6 dudit composé (IX) pour former un composé (VIII);
- l'introduction 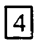 dans ledit composé (VIII) de groupes esters, de préférence acétyle ou benzoyle en position 1,2 (introduction du groupement COR¹ en position 2), 4 et 6 pour former un composé (VII);
- l'introduction 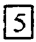 en position anomérique d'un groupement thio de formule S-Rₐ, de préférence un groupement thioéthyle ou thiophényle, pour former un composé de formule (VI) ;
- la déestérification 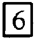 des positions 2, 4 et 6 ou 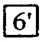 des positions 4 et 6 du composé (VI) pour former ainsi les composés (IV) ou (V) ;
- l'introduction 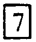 , 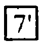 et 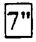 dans lesdits composés (IV) ou (V) des groupements R³ et R⁴ tels que définis précédemment pour conduire au composé (III) ou au monosaccharide (Ia) recherché ;
- le cas échéant, l'estérification 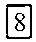 en position 2 dudit composé (III) pour conduire au monosaccharide voulu (Ia).

On obtient ainsi un composé sélectivement fonctionnalisé par l'introduction successive des groupements en position 3 puis 4 et 6 puis 2, ou en position 3 puis 2 puis 4 et 6 qui permet de conduire au composé recherché de formule (Ia), lequel permet de conduire subséquemment en une ou deux étapes de glycosylation et/ou de déprotection et/ou d'activation en position anomérique à un donneur de glycosyle ou un accepteur de glycosyle de formules (Ib) et (II) dans lesquelles n=1 et m=1.

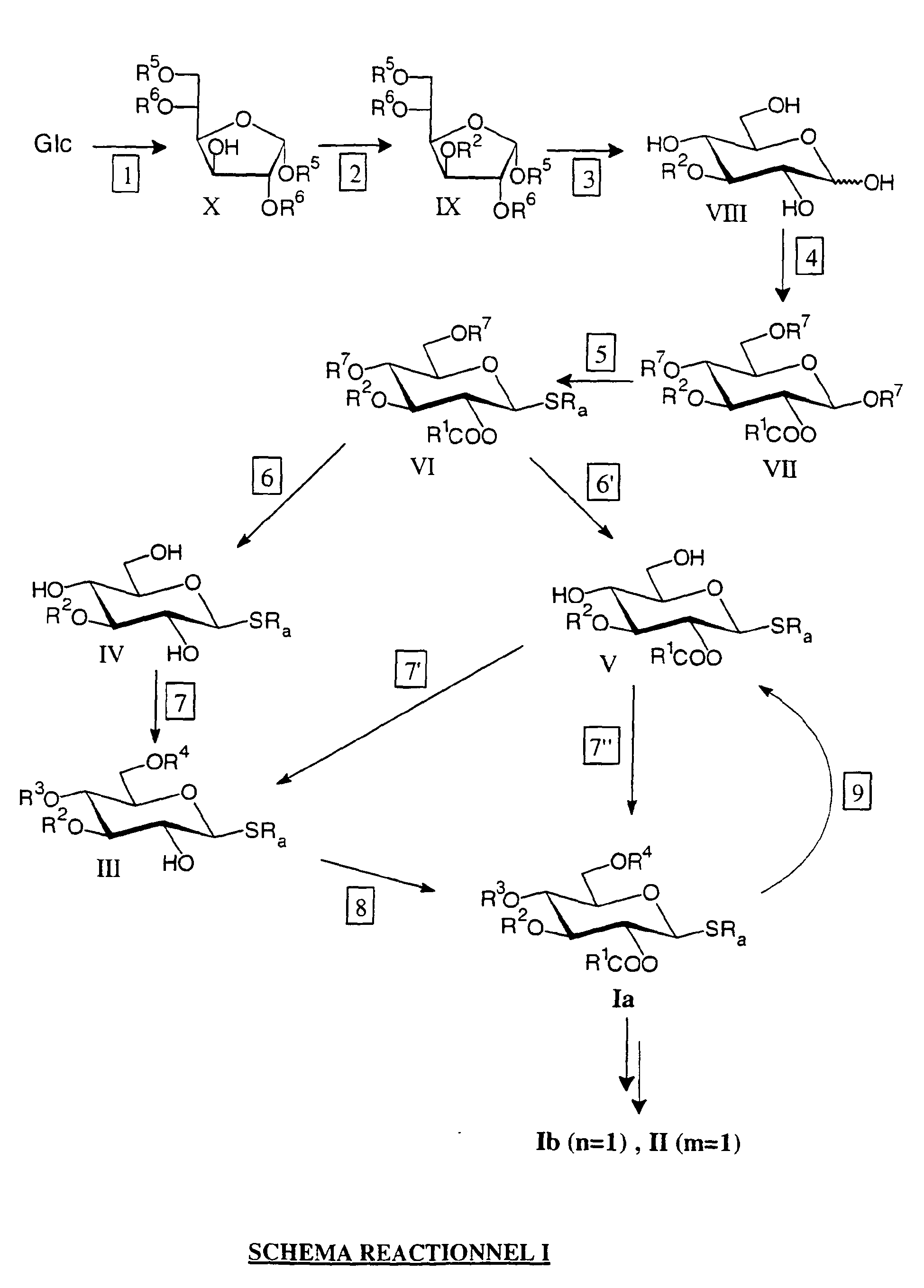

Si le schéma réactionnel 1 précité est original dans certaines de ses séquences réactionnelles, l'homme de métier n'aura toutefois aucune difficulté pour déterminer les conditions réactionnelles les plus appropriées pour mettre en oeuvre chacune des étapes mentionnées de ce schéma.

Les monosaccharides ainsi obtenus permettent d'accéder aisément aux autres oligosaccharides (di-, tri-, etc..) de formule (Ia), (Ib) et (II).

De préférence, un tel oligosaccharide sera préparé par une réaction de couplage entre un donneur de glycosyle monosaccharidique tel qu'obtenu selon le schéma réactionnel (I) ou disaccharidique et un accepteur de glycosyle monosaccharidique tel qu'obtenu selon le schéma réactionnel (I), ou le cas échéant polysaccharidique obtenu à partir de monosaccharides par une ou plusieurs opérations antérieures de couplage, et déprotection sélective du groupe R².

Selon un second aspect, la présente demande vise à couvrir, en tant que produit nouveau, les synthons donneurs ou accepteurs de glycosyle de formule (Ia), (1b) ou (II) tels que définis aux revendications 8 à 11.

L'invention sera maintenant illustrée par les exemples non limitatifs suivants préparés en suivant le schéma réactionnel 1 donné précédemment :

**EXEMPLE 1**

**Préparation du 2,4,6-tri-*O*-acétyl-3-*O-*(2-méthylnaphtyl)-1-thio-β-D-glucopyranoside d'éthyle.**

Etape 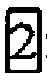 : préparation du 1,2:5,6-di-*O*-isopropylidene-3-*O*-(2-méthylnaphtyl)-α-D-glucofuranose.

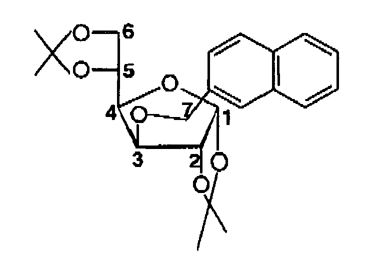

Dans un réacteur de 2 L sont introduits 117,7 g (1 éq.) de 1,2:5,6-di-*O-*isopropylidene-α-D-glucofuranose commercial (M = 260,28) et 100 g de bromure de 2-méthylnaphtyle (1 éq.) à une température de 2°C. 400 mL de dyméthylformamide sont alors ajoutés (dilution 1 dans 2) et le milieu réactionnel est additionné progressivement de 21,7 g (1,2 éq.) d'hydrure de sodium à 60% pendant 10 minutes. On laisse ensuite remonter doucement la température. Au bout de 2h20', l'excès de NaH est neutralisé par du méthanol et le produit est précipité par addition de 2 L d'eau glacé sous vive agitation. 2 heures plus tard, le surnageant est éliminé et le précipité repris au dichlorométhane ( L). Après décantation, la phase organique est séchée et concentrée. Le produit brut (M = 400,5) est directement utilisé pour l'étape suivante.
CCM: Rf = 0,5 [toluène/acétate d'éthyle (9/1; v/v)].
Solide blanc.
RMN ¹³C (CDCl₃, 100 MHz): 135,12; 133,29; 133,11 (C quat. arom.); 128,29, 127,95, 127,78, 126,54, 126,25, 126,07, 125,71 (C arom.); 111,89, 109,011 (C quat. acetal.); 105,39 (C1); 82,73 (C2); 81,65 (C3); 81,41 (C4); 72,59 (C5); 72,49 (C7); 67,50 (C6); 26,90, 26,32, 25,54 (CH₃).
RMN ¹H (CDCl₃, 400 MHz): 7,84-7,80 (m, 4H, H arom.); 7,49-7,45 (m, 3H, H arom.); 5,93 (d, 1H, H1, J_{H1-H2} = 3,7 Hz); 4,85 (d, 1H, H7a, J_{H7a-H7b} = 12,0 Hz); 4,79 (d, 1H, H7b, J_{H7b-H7a} = 12,0 Hz); 4,63 (d, 1H, H2, J_{H2-H1} = 3,7 Hz); 4,42 (td, 1H, H5, J_{H5-H4} = 7,8 Hz, J_{H5-H6a} = J_{H5-H6b} = 6,0 Hz); 4,16 (dd, 1H, H4, J_{H4-H3} = 3,0 Hz, J_{H4-H5} = 7,7 Hz); 4,14 (dd, 1H, H6a, J_{H6a-H5} = 6,1 Hz, J_{H6a-H6b} = 8,6 Hz); 4,08 (d, 1H, H3, J_{H3-H4} = 3,0 Hz); 4,03 (dd, 1H, H6b, J_{H6b-H5} = 5,8 Hz; J_{H6b-H6a} = 8,6 Hz); 1,49, 1,43, 1,40, 1,31 (4s, 12H, CH₃).

Etape 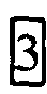 : préparation du 3-*O*-(2-méthylnaphtyl)-D-glucopyranose.

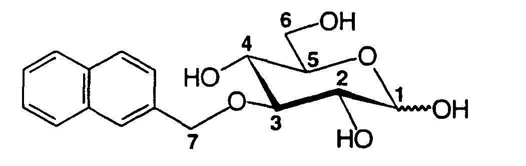

Le produit obtenu précédemment (M = 400,5; 135 g théoriques) est dissous dans 270 mL d'acétone et introduit dans un réacteur de 2 L. 270 mL d'eau (dilution 1 dans 3 au total) puis 420 g de résine IR 120 (H⁺) sont alors ajoutés. Le milieu réactionnel est chauffé à 60°C pendant 1 à 2 jours (au départ le mélange est hétérogène puis devient homogène au cours de la réaction). La réaction terminée, la résine est filtrée, rincée avec du méthanol et le filtrat est neutralisé par addition de quelques mL de solution aqueuse de bicarbonate de sodium à 5%. On évapore à sec et on lave 2 fois le produit en réalisant une suspension de ce dernier dans 1 L de toluène (agitation pendant 15 min à 40 °C puis filtration après retour à température ambiante). Finalement, 116 g de 3-*O*-(2-méthylnaphtyl)-D-glucopyranose recherché (M = 320,3) sont recueillis.
CCM: Rf = 0,2 [dichlorométhane/méthanol (9/1; v/v)].
Solide blanc.
Rendement (% ) = 80 pour les étapes 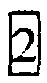 et 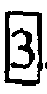 .
RMN ¹³C (CD₃OD, 100 MHz): 137,95, 134,77, 134,44 (C quat. arom.); 128,91, 128,76, 128,62, 127,49, 127,37, 127,32, 126,94, 126,75 (C arom.); anomère β: 98,29 (Cl); 86,40 (C3); 78,01, 76,50 (C2, C5); 73,03 (C7); 71,57 (C4); 62,80 (C6); anomère α: 94,16 (Cl); 83,64 (C3); 76,25, 74,02, 73,10, 71,66 (C2, C4, C5, C7); 62,66 (C6).
RMN ¹H (CD₃OD, 400 MHz): 7,78-7,31 (3 m, H arom.); 5,02 (d, 1H, H1a, J_{H1α-H2α} = 3,6 Hz); 4,97 (d, 1H, H7aβ, J_{H7aβ-H7bβ} = 11,3 Hz); 4,93 (d, 1H, J_{H7bβ-H7aβ} = 11,3 Hz); 4,42 (d, 1H, H1b, J_{H1β-H2β} = 7,8 Hz); 3,77 (dd, 1H, H6aβ, J_{H6aβ-H5β} = 2,4 Hz, J_{H6aβ-H6bβ} = 11,9 Hz); 3,57 (dd, 1H, H6bβ, J_{H6bβ-H5β} = 5,9 Hz, J_{H6bβ-H6aβ} = 11,8 Hz); 3,38 (t, 1H, H4b, J_{H4β-H3β} = J_{H3β-H5β} = 9,1 Hz): 3,32 (t, 1H, J_{H3β-H2β} = J_{H3β-H4β} = 8,8 Hz); 3,24-3,19 (m, 2H, H2β, H5β).

Etape 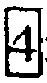 : préparation du 1,2,4,6-tétra-*O*-acétyl-3-*O*-(2-méthylnaphtyl)-D-glucopyranose.

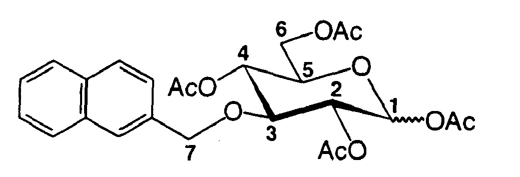

Dans un ballon de 1 L sont introduits successivement 116 g (1 éq.) de 3-*O-*(2-méthylnaphtyl)-D-glucopyranose (M = 320,3), 59,4 g (2 éq.) d'acétate de sodium et 680 mL (20 éq.) d'anhydride acétique. Le mélange est porté au reflux dans un bain d'eau bouillante pendant 2 heures puis coulé sur 5 L d'eau à température ambiante. Après une nuit d'agitation, le précipité obtenu est filtré puis lavé avec 6 L d'une solution aqueuse de bicarbonate de sodium à 5% pendant 10 min, filtré de nouveau et rincé à l'eau jusqu'à neutralité et séché. On obtient ainsi très majoritairement le produit de configuration β recherché (M = 488,5) sous la forme d'un mélange d'anomères β/α = 86/14.
CCM: Rf = 0,3 [éther de pétrole/acétate d'éthyle (7/3; v/v)].
Solide blanc.
RMN ¹³C (CDCl₃, 100 MHz): anomère α: 170,88, 169,70, 169,37, 168,88 (C=O); 135,43, 133,26, 133,01 (C quat. arom.); (128,28, 127,91, 127,76, 126,37, 126,26, 126,15, 125,72, 125,54 (C arom.); 89,57 5 (C1); 77,03 (C3); 74,89 (C7); 71,62 (C2); 70,33 (C5); 69,08 (C4); 61,81 (C6); 21,01, 20,83, 20,82, 20,71 (CH₃).
anomère β: 170,82, 169,38, 169,33, 169,18 (C=O); 135,01, 133,21, 133,03 (C quat. arom.); 128,33, 127,94, 127,72, 126,62, 126,35, 126,20, 125,70 (C arom.); 92,01 (C1); 79,86 (C3); 74,34 (C7); 73,02 (C5); 71,61 (C2); 69,06 (C4); 61,79 (C6); 20,94, 20,82, 20,80, 20,77 (CH₃).
RMN ¹H (CDCl₃, 400 MHz): anomère α: 7,83-7,81 (m, 3H, H arom.); 7,70 (s, 1H, H arom.); 7,51-7,41 (m, 2H, H arom.); 7,37-7,35 (m, 1H, H arom.); 6,32 (d, 1H, H1, J_{H1-H2} = 3,7 Hz); 5,20 (t, 1H, H4, J_{H4-H3} = J_{H4-H5} = 9,8 Hz); 5,11 (dd, 1H, H2, J_{H2-H1} = 3,7 Hz, J_{H2-H3} = 10,0 Hz); 4,87 (d, 1H, H7a, J_{H7a-H7b} = 12,1 Hz); 4,78 (d, 1H, H7b, J_{H7b-H7a} = 12,1 Hz); 4,21 (dd, 1H, H6a, J_{H6a-H5} = 4,3 Hz, J_{H6a-H6b} = 12,5 Hz); 4,07 (dd, 1H, H6b, J_{H6b-H5} = 2,4 Hz, J_{H6b-H6a} = 12,5 Hz); 4,02 (t, 1H, H3, J_{H3-H2} = J_{H3-H4} = 9,7 Hz); 4,01 (ddd, 1H, H5, J_{H5-H4} = 10,2 Hz, J_{H5-H6a} = 4,2 Hz, J_{H5-H6b} = 2,4 Hz); 2,16, 2,09, 1,98, 1,92 (4s, 12H, CH₃).
anomère β: 7,83-7,80 (m, 3H, H arom.); 7,69 (s, 1H, H arom.); 7,51-7,46 (m, 2H, H arom.); 7,36-7,33 (m, 1H. H arom.); 5,66 (d, 1H, H1, J_{H1-H2} = 8,2 Hz); 5,21 (dd, 1H, H2, J_{H2-H1} = 8,2 Hz, J_{H2-H3} = 9,5 Hz); 5,20 (t, 1H. H4, J_{H4-H3} = J_{H4-H5} = 9,5 Hz); 4,78 (s, 2H, H7a, H7b); 4,23 (dd, 1H, H6a, J_{H6a-H5} = 4,8 Hz, J_{H6a-H6b} = 12,5 Hz); 4,09 (dd, 1H, H6b, J_{H6b-H5} = 2,4 Hz, J_{H6b-H6a} = 12,2 Hz); 3,81 (t, 1H, H3, J_{H3-H2} = J_{H3-H4} = 9,3 Hz); 3,74 (ddd, 1H, H5, J_{H5-H4} = 10,0 Hz, J_{H5-H6a} = 4,8 Hz, J_{H5-H6b} = 2,3 Hz); 2,10, 2,08, 1,94, 1,93 (4s, 12H, CH₃).

Etape 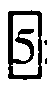 : préparation du 2,4,6-tri-*O*-acétyl-3-*O*-(2-méthylnaphtyl)-1-thio-β-D-glucopyranoside d'éthyle.

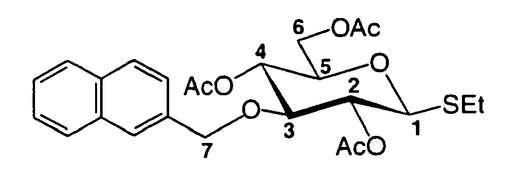

Dans un réacteur de 2 L sont dissous 177 g (1 éq.) théoriques de 1,2,4,6-tétra-*O*-acétyl-3-*O*-(2-méthylnaphtyl)-D-glucopyranose obtenu précédemment (M = 488,5) puis 885 mL (dilution 1 dans 5) de dichlorométhane. Le milieu est refroidi à 0°C puis on ajoute 20,5 mL (1,1 éq.) d'éthanethiol et doucement goutte à goutte 50,1 mL (1,1 éq.) d'éthérate de trifluorure de bore (addition pendant 20 min). Après 2 heures à 0°C, on lave 2 fois avec 1 L d'une solution aqueuse de bicarbonate de sodium à 5% (la solution de couleur rouge devient jaune pâle), une fois avec 500 mL d'eau puis la phase organique est séchée (MgSO₄) et évaporée. Le produit brut isolé (M = 490,6) est utilisé comme tel pour la suite.
CCM: Rf = 0,4 [éther de pétrole/acétate d'éthyle (7/3; v/v)].
Solide blanc.
RMN ¹³C (CDCl₃, 100 MHz): 170,89, 149,46, 169,43 (C=O); 135,27, 133,25, 133,02 (3 C quat. arom.); 128,29, 127,95, 127,74, 126,52, 126,32, 126,14, 125,73 (7 C quat. arom.); 83,75 (C1); 81,51 (C3); 76,25, 74,32, 71,31, 69,70 (C2, C4, C5, C7); 62,54 (C6); 24,03 (CH₂[SEt]); 21,03, 20,87 (CH₃[Ac]); 14,85 (CH₃[SEt]).
RMN ¹H (CDCl₃, 400 MHz): 7,83-7,80 (m, 3H, H arom.); 7,68 (s, 1H, H arom.); 7,50-7,46 (m, 2H, H arom.); 7,35-7,33 (m, 1H, H arom.); 5,14 (t, 1H, H2 ou H4, J = 9,9 Hz); 5,12 (t, 1H, H2 ou H4, J = 9,7 Hz); 4,80 (d, 1H, H7a, J_{H7a-H7b} = 12,0 Hz); 4,75 (d, 1H, H7b, J_{H7b-H7a} = 12,1 Hz); 4,40 (d, 1H, H1, J_{H1-H2} = 10,0 Hz); 4,19 (dd, 1H, H6a, J_{H6a-H5} = 5,1 Hz, J_{H6a-H6b} = 12,3 Hz); 4,11 (dd, 1H, H6b, J_{H6b-H5} = 2,4 Hz, J_{H6b-H6a} = 12,2 Hz); 3,76 (t, 1H, H3, J_{H3-H2} = J_{H3-H4} = 9,2 Hz); 3,60 (ddd, 1H, H5, J_{H5-H4} = 10,0 Hz, J_{H5-H6a} = 5,1 Hz, J_{H5-H6b} = 2,5 Hz); 2,77-2,63 (m, 2H, CH₂[SEt]); 2,07, 1,98, 1,92 (3s, 9H, CH₃); 1,24 (t, 3H, CH₃[SEt], J = 7,4 Hz).

**EXEMPLE 2**

**Préparation d'un donneur de glycosyle de formule générale (la) selon l'invention produit de l'exemple 1 puis des étapes** 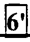 **et** 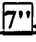 **.**

Etape 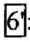 : préparation du 2-*O*-acétyl-3-*O*-(2-méthylnaphtyl)-1-thio-β-D-glucopyranoside d'éthyle.

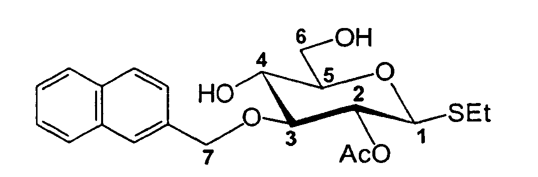

178 g (1 éq.) de 2,4,6-tri-*O*-acétyl-3-*O*-(2-méthylnaphtyl)-1-thio-β-D-glucopyranoside d'éthyle (M = 490,6) théoriques sont dissous à chaud (40°C) dans 180 mL de toluène et coulés sur 900 mL (dilution 1 dans 5) de méthanol. La solution est limpide. Le retour à T.A. s'accompagne de la précipitation progressive du produit jusqu'à obtention d'une suspension. On ajoute alors doucement 0,05 éq. de sodium sous forme d'une solution de méthylate de sodium obtenue en dissolvant 417 mg de sodium dans 50 mL de méthanol. Au bout d'une demi-heure, la solution redevient parfaitement limpide. Après 3h30' de réaction, le milieu est neutralisé par de la résine IR 120 (H⁺), filtré et concentré. Le résidu huileux obtenu est coulé sur 1,5 L d'heptane: le produit précipite. Après filtration, le résidu est repris par 1 L de dichlorométhane et les coproduits de réaction sont éliminés par 2 lavages à chaud (35-40°C) avec 1 L d'eau. La phase organique est récupérée, séchée et évaporée pour conduire à 95 g de 2-*O*-acétyl-3-*O*-(2-méthylnaphtyl)-1-thio-β-D-glucopyranoside d'éthyle recherché (M = 406,5).
CCM: Rf = 0,5 [dichlorométhane/méthanol (9/1; v/v)].
Solide blanc.
Rendement (%) = 65 pour les étapes 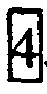 , 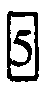 et 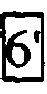 .
RMN ¹³C (CDCl₃, 100 MHz): 169,81 (C=O); 135,48, 133,27, 133,07 (3 C quat. arom.); 128,54, 127,99, 127,78, 126,70, 126,37, 126,18, 125,69 (C arom.); 83,86 (C3); 83,77 (Cl); 79,52 (C5); 74,85 (C7); 71,63 (C2); 70,46 (C4); 62,46 (C6); 24,17 (CH₂[SEt]); 21,17 (CH₃[Ac]); 14,90 (CH₃[SEt]).
RMN ¹H (CDCl₃, 400 MHz): 7,85-7,81 (m, 3H, H arom.); 7,74 (s, 1H, H arom.); 7,50-7,45 (m, 3H, H arom.); 5,01 (dd, 1H, H2, J_{H2-H1} = 9,9 Hz, J_{H2-H3} = 9,2 Hz); 4,97 (d, 1H, H7a, J_{H7a-H7b} = 11,9 Hz); 4,85 (d, 1H, H7b, J_{H7b-H7a} = 11,8 Hz); 4,40 (d, 1H, H1, J_{H1-H2} = 10,0 Hz); 3,88 (dd, 1H, H6a, J_{H6a-H5} = 3,2 Hz, J_{H6a-H6b} = 12,0 Hz); 3,78 (dd, 1H, H6b, J_{H6b-H5} = 4,7 Hz, J_{H6b-H6a} = 12,0 Hz); 3,72 (t, 1H, H4, J_{H4-H3} = J_{H4-H5} = 9,3 Hz); 3,59 (t, 1H, H3, J_{H3-H2} = J_{H3-H4} = 9,1 Hz); 3,37 (ddd, 1H, H5, J_{H5-H4} = 9,5 Hz, J_{H5-H6a} = 3,4 Hz, J_{H5-H6b} = 4,6 Hz); 2,99 (sl, 1H, OH); 2,68 (qd, 2H, CH₂[SEt], J = ); 2,41 (sl, 1H, OH); 2,00 (s, 3H, CH₃[Ac]); 1,24 (t, 3H, CH₃[SEt], J = 7,4 Hz).

Etape 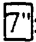 : préparation du 2-*O*-acéty-1,6-*O*-benzylidène-3-*O*-(2-méthylnaphtyl)-1-thio-β-D-glucopyranoside d'éthyle.

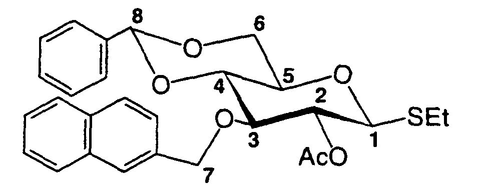

Dans un réacteur de 2 L, 56 g (1 éq.) de 2-*O*-acétyl-3-*O*-(2-méthylnaphtyl)-1-ihio-β-D-glucopyranoside d'éthyle (M = 406,5) sont dissous dans 300 mL (dilution 1 dans 5) d'acétonitrile puis additionnés de 31,0 mL (1,5 éq.) de benzaldéhyde diméthylacétal et 6,4 g (0,2 éq.) d'acide camphosulfonique anhydre (le milieu rosit). Le mélange est chauffé à 55°C pendant 2 heures puis refroidi à T.A. et neutralisé avec 3,8 mL de triéthylamine (décoloration: le milieu devient jaune pâle). Après concentration, le résidu est dissous dans le minimum de dichlorométhane et coulé sur 1 L de méthanol: le produit précipite. Après une nuit au congélateur, le produit est filtré, rincé avec du méthanol glacé puis séché. 53 g de 2-*O*-acétyl-4,6-*O*-benzylidène-3-*O*-(2-méthylnaphtyl)-1-thio-β-D-glucopyranoside d'éthyle attendu (M = 494,6) sont ainsi isolés.
CCM: Rf = 0,6 [toluène/acétate d'éthyle (17/3; v/v)].
Solide blanc.
Rendement (%) = 78.
RMN ¹³C (CDCl₃, 100 MHz): 169,59 (C=O); 137,20, 135,61, 133,24, 133,03 (4 C quat. arom.); 129,15, 128,39, 128,13, 127,97, 127,72, 126,79, 126,17, 126,10, 126,02, 125,99 (C arom.); 101,36 (C8); 84,25 (C1); 81,64, 79,48 (C3, C4); 74,37 (C7); 71,28 (C2); 70,70 (C5); 68,67 (C6); 23,98 (CH₂[SEt]); 21,04 (CH₃[Ac]); 14,86 (CH₃[SEt]).
RMN ¹H (CDCl₃, 400 MHz): 7,83-7,73 (m, 4H, H arom.); 7,53-7,45 (m, 4H, H arom.); 7,42-7,39 (m, 4H, H arom.); 5,61 (s, 1H, H8); 5,12-5,07 (m, 1H, H2); 5,03 (d, 1H, H7a, J_{H7a-H7b} = 12,2 Hz); 4,87 (d, 1H, H7b, J_{H7b-H7a} = 12,3 Hz); 4,45 (d, 1H, H1, J_{H1-H2} = 10,0 Hz); 4,39 (dd, 1H, H6a, J_{H6a-H5} = 5,0 Hz, J_{H6a-H6b} = 10,5 Hz); 3,83-3,78 (m, 3H, H3, H4, H6b); 3,50 (ddd, 1H, H5, J_{H5-H4} = 9,5 Hz, J_{H5-H6a} = 4,8 Hz, J_{H5-H6b} = 9,4 Hz); 2,77-2,64 (m, 2H, CH₂[SEt]); 2,00 (s, 3H, CH₃[Ac]); 1,25 (t, 3H, CH₃[SEt], J = 7,4 Hz).

**EXEMPLE 3**

**Préparation d'un donneur de glycosyle de formule générale (Ia) selon l'invention.**

Etape 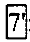 : préparation du 4,6-di-*O*-benzyl-3-*O*-(2-méthylnaphtyl)-1-thio-β-D-glucopyranoside d'éthyle.

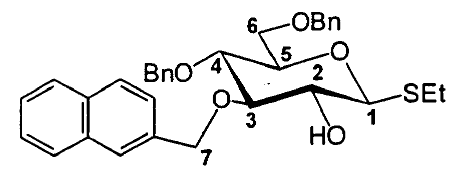

20 g (1 éq.) de 2-*O*-acétyl-3-*O*-(2-méthylnaphtyl)-1-thio-β-D-glucopyranoside d'éthyle préparé à l'étape 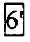 de l'exemple 2 (M = 406,5) sont dissous dans 200 mL (dilution 1 dans 20) de diméthylformamide à 0°C et additionnés de 13 mL (2,2 éq.) de bromure de benzyle. On ajoute alors très progressivement 4,4 g (2,2 éq.) d'hydrure de sodium à 60% à 0°C. Après 2 heures de réaction à température ambiante, l'excès de NaH est détruit par addition de méthanol et le milieu est dilué avec 250 mL d'éther éthylique puis lavé 2 fois avec 30 mL d'eau, séché et concentré. La purification sur gel de silice [flash; éluant: éther de pétrole/acétate d'éthyle (9/1; v/v)] permet de recueillir 21,4 g de 4,6-di-*O*-benzyl-3-*O*-(2-méihylnaphtyl)-1-thio-β-D-glucopyranoside d'éthyle recherché (M =544,7). CCM: Rf= 0,3 [éther de pétrole/acétate d'éthyle (8/2; v/v)].
Solide blanc.
Rendement (%) = 80.
RMN ¹³C (CDCl₃, 100 MHz): 138,19, 138,10, 136,08, 133,36, 133,03 (5 C quat. arom.); 128,43, 128,38, 128,29, 127,98, 127,79, 127,72, 127,64, 126,69, 126,09, 126,04, 125,90 (C arom.); 86,16 (Cl); 85,98 (C3); 79,46 (C5); 77,46 (C4); 75,28 (CH₂[Bn]); 75,12 (C7); 73,42 (CH₂[Bn]); 73,34 (C2); 69,05 (C6); 24,32 (CH₂); 15,46 (CH₃).
RMN ¹H (CDCl₃, 400 MHz): 7,73-7,67 (m, 4H, H arom.); 7,46-7,35 (m, 3H, H arom.); 7,26-7,17 (m, 8H, H arom.); 7,09-7,07 (m, 2H, H arom.); 5,02 (d, 1H, CH₂[Bn], J = 11,5 Hz); 4,92 (d, 1H, CH₂[Bn], J = 11,6 Hz); 4,78 (d, 1H, H7a, J_{H7a-H7b} = 10,9 Hz); 4,52 (d, 1H, CH₂[Bn], J = 11,9 Hz); 4,49 (d, 1H, H7b, J_{H7b-H7a} = 9,7 Hz); 4,45 (d, 1H, CH₂[Bn], J = 12,2 Hz); 4,23 (d, 1H, H1, J_{H1-H2} = 10,2 Hz); 3,67 (dd, 1H, H6a, J_{H6a-H5} = 1,9 Hz, J_{H6a-H6b} = 10,9 Hz); 3,62 (dd, 1H, H6b, J_{H6b-H5} = 4,5 Hz, J_{H6b-H6a} = 10,9 Hz); 3,59-3,47 (m, 3H, H2, H3, H4); 3,43 (m, 1H, H5); 2,72-2,58 (m, 2H, CH₂); 2,40 (s, 1H, OH); 1,23 (t, 3H, CH₃, J = 7,4 Hz).

Etape 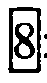 : préparation du 4,6-di-*O*-benzyl-3-*O*-méthylnaphtyl-1-thio-β-D-glucopyranoside d'éthyle.

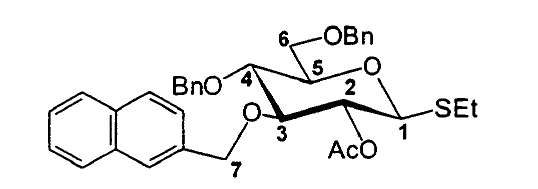

21 g (1 éq.) de 4,6-di-*O*-benzyl-3-*O*-(2-méthylnaphtyl)-1-thio-β-D-glucopyranoside d'éthyle recherché (M = 544,7) sont dissous dans 100 mL de pyridine et additionnés de 10,9 mL (3 éq.) d'anhydride acétique. La réaction terminée, le milieu est dilué à l'eau: le produit recherché précipite. Après filtration, ce dernier est repris au dichlorométhane, lavé avec une solution d'acide chlorhydrique à 10%, une solution de bicarbonate de sodium à 5%, puis à l'eau. 21 g de 4,6-di-*O*-benzyl-3-*O*-méthylnaphtyl-1-thio-(3-D-glucopyranoside d'éthyle (M = 586,8) sont ainsi recueillis.
CCM: Rf = 0,4 [éther de pétrole/acétate d'éthyle (8/2; v/v)].
Solide blanc.
Rendement (%) = 95.
RMN ¹³C (CDCl₃, 100 MHz): 169,76 (C=O); 138,20, 137,95, 135,71, 133,31, 133.02 (5 C quat. arom.); 128,50, 128,43, 128,25, 128,09, 127,99, 127,92, 127,77, 127,73, 127,67, 126,53, 126,20, 126,01, 125,89 (C arom.); 84,40 (C3 ou C4); 83,46 (Cl); 79,55 (C5); 78,00 (C3 ou C4); 75,31 (CH₂[Bn]); 75,19 (C7); 73,52 (CH₂[Bn]); 71,82 (C2); 68,91 (C6); 23,83 (CH₂[SEt]); 21,07 (CH₃[Ac]); 14,97 (CH₃[SEt]).
RMN ¹H (CDCl₃, 400 MHz): 7,75-7,69 (m, 3H, H arom.); 7,63 (s, 1H, H arom.); 7,41-7,38 (m, 2H, H arom.); 7,32-7,30 (s, 1H, H arom.); 7,26-7,19 (m, 10H, H arom.); 7,11-7,08 (m, 2H, H arom.); 5,02-4,97 (m, 1H, H2); 4,88 (d, 1H, CH₂[Bn], J = 11,7 Hz); 4,77 (d, 1H, CH₂[Bn], J = 11,7 Hz); 4,73 (d, 1H, H7a, J_{H7a-H7b} = 10,8 Hz); 4,54 (d, 1H, CH₂[Bn], J = 12,0 Hz); 4,51 (d, 1H, H7b, J_{H7b-H7a} = 9,4 Hz); 4,48 (d, 1H, CH₂[Bn], J = 12,2 Hz); 4,28 (d, 1H, H1, J_{H1-H2} = 10,0 Hz); 3,69 (dd, 1H, H6a, J_{H6a-H5} = 2,0 Hz, J_{H6a-H6b} = 10,9 Hz); 3,68-3,63 (m, 3H, H3, H4, H6b); 3,44 (ddd, 1H, H5, J_{H5-H4} = 9,2 Hz, J_{H5-H6a} = 2,1 Hz, J_{H5-H6b} = 4,1 Hz); 2,71-2,56 (m, 2H, CH₂[SEt]); 1,84 (s, 3H, CH₃[Ac]); 1,18 (t, 3H, CH₃[SEt], J = 7,4 Hz).

**EXEMPLE 4**

**Préparation d'un donneur de glycosyle de formule générale (la) selon l'invention, produit de l'exemple 1 puis des étapes** 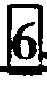 **,** 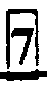 **et** 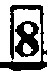 **.**

Etape 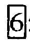 : préparation du 3-*O*-(2-méthylnaphtyl)-1-thio-β-D-glucopyranoside d'éthyle.

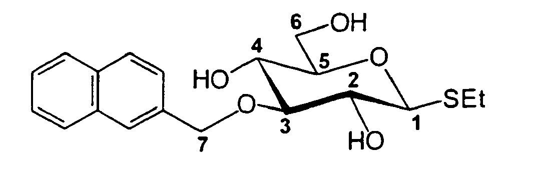

A 100 g de 2,4,6-tri-*O*-acétyl-3-*O*-(2-méthylnaphtyl)-1-thio-β-D-glucopyranoside d'éthyle préparé à l'exemple 1 (M = 490,6) théoriques dissous dans 100 mL de toluène, sont progressivement ajoutés 2 L de méthanol contenant 7,03 g (1,5 éq.). Après 4 heures de réaction à température ambiante, le milieu est neutralisé par de la résine IR 120 (H⁺), filtré et concentré. Le résidu huileux obtenu est coulé sur 1 L d'heptane: le produit précipite. Après filtration, le résidu est repris par 0,6 L de dichlorométhane et les coproduits de réaction sont éliminés par 2 lavages à chaud (35-40°C) avec 0,6 L d'eau. La phase organique est récupérée, séchée et évaporée pour conduire à 49,8 g de 3-*O*-(2-méthylnaphtyl)-1-thio-β-D-glucopyranoside d'éthyle (M = 364,5).
CCM: Rf = 0,5 [dichlorométhane/méthanol (9/1; v/v)].
Solide blanc.
Rendement (%) = 67 pour les étapes 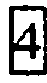 , 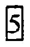 et 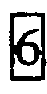 .
RMN ¹³C (CDCl₃, 100 MHz): 135,83, 133,36, 133,12 (3 C quat. arom.); 128,62, 128,01, 127,83, 126,99, 126,34, 126,15, 125,91 (C arom.); 86,70 (Cl); 85,00 (C3); 79,48 (C5); 74,91 (C7); 73,23 (C2); 70,13 (C4); 62,74 (C6); 24,74 (CH₂[SEt]); 15,49 (CH₃[SEt]).
RMN ¹H (CDCl₃, 400 MHz): 7,86-7,82 (m, 4H, H arom.); 7,51-7,47 (m, 3H, H arom.); 5,17 (d, 1H, H7a, J_{H7a-H7b} = 11,8 Hz); 4,94 (d, 1H, H7b, J_{H7b-H7a} = 11,8 Hz); 4,36 (d, 1H, H1, J_{H1-H2} = 9,6 Hz); 3,87 (dd, 1H, H6a, J_{H6a-H5} = 3,3 Hz, J_{H6a-H6b} = 12,0 Hz); 3,75 (dd, 1H, H6b, J_{H6b-H5} = 4,9 Hz, J_{H6b-H6a} = 12,0 Hz); 3,62 (t, 1H, H4, J_{H4-H3} = J_{H4-H5} = 9,2 Hz); 3,53 (t, 1H, H2, J_{H2-H1} = J_{H2-H3} = 9,0 Hz); 3,46 (t, 1H, H3, J_{H3-H2} = J_{H3-H4} = 8,6 Hz); 3,37 (ddd, 1H, H5, J_{H5-H4} = 9,2 Hz, J_{H5-H6a} = 3,5 Hz, J_{H5-H6b} = 4,8 Hz); 2,73 (qd, 2H, CH₂[SEt)); 2,68 (sl, 1H, OH); 2,56 (s, 1H, OH); 2,22 (sl, 1H, OH); 1,33 (t, 3H, CH₃[SEt], J = 7,4 Hz).

Etape 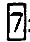 : préparation du 4,6-*O*-benzylidène-3-*O*-(2-méthylnaphtyl)-1-thio-β-D-glucopyranoside d'éthyle.

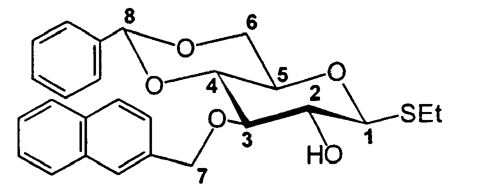

Dans un réacteur de 2 L, 50 g (1 éq.) de 3-*O*-(2-méthylnaphtyl)-1-thio-β-D-glucopyranoside d'éthyle (M = 364,5) sont dissous dans 300 mL (dilution 1 dans 5) d'acétonitrile puis additionnés de 31,0 mL (1,5 éq.) de benzaldéhyde diméthylacétal et 6,4 g (0,2 éq.) d'acide camphosulfonique anhydre (le milieu rosit). Le mélange est chauffé à 55°C pendant 2 heures puis refroidi à T.A. et neutralisé avec 3,8 mL de triéthylamine (décoloration: le milieu devient jaune pâle). Après concentration, le résidu est dissous dans le minimum de dichlorométhane et coulé sur 1 L de méthanol: le produit précipite. Après une nuit au congélateur, le produit est filtré, rincé avec du méthanol glacé puis séché. 48,5 g de 4,6-*O*-benzylidène-3-*O*-(2-méthylnaphtyl)-1-thio-β-D-glucopyranoside d'éthyle attendu (M = 452,6) sont ainsi isolés.
CCM: Rf = 0,4 [toluène/acétate d'éthyle (17/3; v/v)].
Solide blanc.
Rendement (%) = 78.
RMN ¹³C (CDCl₃, 100 MHz): 137,30, 135,80, 133,33, 133,10 (4 C quat. arom.); 129,10, 128,35, 128,30, 128,01, 127,74, 126,90, 126,14, 126,11, 126,05, 125,95 (C arom.); 101,41 (C8); 86,71 (Cl); 81,47, 81,25 (C3, C4); 74,74 (C7); 73,19 (C2); 70,83 (C5); 68,72 (C6); 24,64 (CH₂); 15,31 (CH₃).
RMN ¹H (CDCl₃, 400 MHz): 7,82-7,74 (m, 4H, H arom.); 7,53-7,39 (m, 8 H, H arom.); 5,60 (s, 1H, H8); 5,14 (d, 1H, H7a, J_{H7a-H7b} = 12,0 Hz); 5,01 (d, 1H, H7b, J_{H7b-H7a} = 12,0 Hz); 4,46 (d, 1H, H1, J_{H1-H2} = 9,7 Hz); 4,37 (dd, 1H, H6a, J_{H6a-H5 =} 5,0 Hz, J_{H6a-H6b} = 10,4 Hz); 3,79 (t, 1H, H6b, J_{H6b-H5} = J_{H6b-H6a} = 10,3 Hz); 3,76-3,71 (m, 2H, H3, H4); 3,63 (dd, 1H, H2, J_{H2-H1} = 9,0 Hz, J_{H2-H3} = 8,4 Hz); 3,50 (ddd, 1H. H5, J_{H5-H4} = J_{H5-H6b} = 9,5 Hz, J_{H5-H6a} = 5,1 Hz); 2,82-2,69 (m, 2H, CH₂); 2,61 (s, 1H, OH); 1,32 (t, 3H, CH₃, J = 7,4 Hz).

Etape 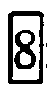 : préparation du 2-*O*-benzoyl-4,6-*O*-benzylidène-3-*O*-(2-méthylnaphtyl)-1-thio-β-D-glucopyranoside d'éthyle.

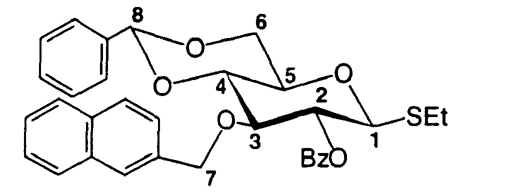

A 48,5 g de 4,6-*O*-benzylidène-3-*O*-(2-méthylnaphtyl)-1-thio-β-D-glucopyranoside d'éthyle (1 éq.) (M = 452,6) dans 300 mL de pyridine (dilution 1 dans 6) sont additionnés doucement en une seule fois 37 ml (3 éq.) de chlorure de benzoyle et la réaction est laissée une nuit à TA. La solution est ensuite coulée sur 1 1 de méthanol sous vive agitation. Le produit précipite doucement puis est filtré et rincé avec du méthanol glacé. 50 g de 2-*O*-benzoyl-4,6-*O*-benzylidène-3-*O*-(2-méthylnaphtyl)-1-thio-β-D-glucopyranoside d'éthyle recherché (M = 556,7) sont ainsi recueillis.
CCM: Rf = 0,6 [toluène/acétate d'éthyle (9/1; v/v)].
Solide blanc.
Rendement (%) = 84.
RMN ¹³C (CDCl₃, 100 MHz): 165,25 (C=O); 137,27, 135,28, 133,25, 133,09, 132,95 (5 C quat. arom.); 129,95, 129,18, 128,42, 128,11, 127,92, 127,67, 127,06, 126,25, 126,14, 125,95, 125,83 (C arom.); 101,42 (C8); 84,38 (Cl); 81,81 (C4), 79,00 (C3); 74,29 (C7); 71,86 (C2); 70,76 (C5); 68,73 (C6); 24,12 (CH₂[SEt]); 14,88 (CH₃[SEt]).
RMN ¹H (CDCl₃, 400 MHz): 7,95-7,92 (m, 2H, H arom.); 7,69-7,36 (3m, 12H, H arom.); 7,22 (dd, 1H, H arom.); 5,64 (s, 1H, H8); 5,36 (dd, 1H, H2, J_{H2-H1} = 10,0 Hz, J_{H2-H3} = 8,5 Hz); 4,98 (d, 1H, H7a, J_{H7a-H7b} = 12,2 Hz); 4,86 (d, 1H, H7b, J_{H7b-H7a} = 12,2 Hz); 4,59 (d, 1H, H1, J_{H1-H2} = 10,0 Hz); 4,41 (dd, 1H, H6a, J_{H6a-H5} = 5,0 Hz, J_{H6a-H6b} = 10,5 Hz); 3,93 (t, 1H, H3, J_{H3-H2} = J_{H3-H4} = 9,2 Hz); 3,88 (dd, 1H, H4, J_{H4-H3} = 8,8 Hz, J_{H4-H5} = 9,2 Hz); 3,84 (t, 1H, H6b, J_{H6b-H5} = J_{H6b-H6a} = 10,3 Hz); 3,56 (ddd, 1H, H5, J_{H5-H4} = 9,1 Hz, J_{H5-H6a} = 5,0 Hz, J_{H5-H6b} = 9,9 Hz); 2,77-2,64 (m, 2H, CH₂[SEt]); 1,21 (t, 3H, CH₃[SEt], J = 7,4 Hz).

### EXEMPLE 5

### Synthèse d'un synthon accepteur de glycosyle de formule générale (II) selon l'invention.

### Etape 5A: préparation du précurseur 2-O-benzoyl-4,6-O-benzylidène-3-O-(2-méthylnaphtyl)-β-D-glucopyranoside de benzyle.

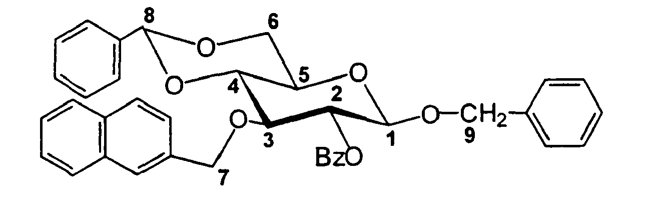

Dans un ballon, à 0°C, sont introduits successivement 31,2 g (1 éq) de 2-*O*-benzoyl-4,6-*O*-benzylidène-3-*O*-(2-méthylnaphtyl)-1-thio-β-D-glucopyranoside d'éthyle (M = 556,7), 13,9 g (1,1 éq.) de N-iodosuccinimide, 10 g de tamis moléculaire 4Å, 200 mL (dilution 1 dans 5) de dichlorométhane anhydre et enfin 6,96 mL (1,2 éq.) d'alcool benzylique. On ajoute alors 1,27 mL (0,1 éq.) de triflate de triéthylsilyle. Après 1 heure de réaction à 0°C, le milieu est neutralisé par quelques gouttes de triéthylamine, filtré sur verre fritté et concentré. La purification sur gel de silice [flash; éluant: toluène/acétate d'éthyle (97/3; v/v)] permet de recueillir 30 g de 2-*O*-benzoyl-4,6-*O*-benzylidène-3-*O*-(2-méthylnaphtyl)-β-D-glucopyranoside de benzyle attendu (M = 602,7).
CCM: Rf = 0,6 [toluène/acétate d'éthyle (9/1; v/v)].
Solide blanc.
Rendement (%) = 89.
RMN ¹³C (CDCl₃, 100 MHz): 165,14 (C=O); 137,28, 136,75, 135,32, 133,13, 133,04, 132,89 (6 C quat. arom.); 129,93, 129,77, 129,13, 128,37, 128,32, 128,29, 128,05, 127,87, 127,77, 127,67, 127,61, 126,97, 126,20, 126,13, 125,89, 125,76 (C arom.); 101,38 (C8): 99,96 (Cl); 81,78 (C4); 77,57 (C3); 73,96 (C7); 73,31 (C2): 70.46 (C9); 68,76 (C6); 66,28 (C5).
RMN ¹H (CDCl₃, 400 MHz): 7,89-7,86 (m, 2H, H arom.); 7,67-7,07 (m, 17H, H arom.); 5,62 (s, 1H, H8); 5,40 (t, 1H, H2, J_{H2-H1} = J_{H2-H3} = 8,3 Hz); 4,95 (d, 1H, H7a, J_{H7a-H7b} = 12,4 Hz); 4,84 (d, 2H, H7b, H9a, J_{H7b-H7a} = J_{H9a-H9b} = 12,6 Hz); 4,58 (d, 1H, H9b, J_{H9b}-_{H9a} = 13,6 Hz); 4,58 (d, 1H, H1, J_{H1-H2} = 7,8 Hz); 4,40 (dd, 1H, H6a, J_{H6a-H5} = 5,0 Hz, J_{H6a-H6b} = 10,5 Hz); 3,90 (t, 1H, H4, J_{H4-H3} = J_{H4-H5} = 9,1 Hz); 3,86 (t, 1H, H6b, J_{H6b-H5} = J_{H6b-H6a} = 10,2 Hz); 3,84 (dd, 1H, H3, J_{H3-H2} = 8,5 Hz, J_{H3-H4} = 9,1 Hz); 3,45 (ddd, 1H, H5, J_{H5-H4} = 9,2 Hz, J_{H5-H6a} = 5,0 Hz, J_{H5}-_{H6b} = 9,8 Hz).

### Etape 5B: préparation du synthon accepteur 2-O-benzoyl-4,6-O-benzylidène-β-D-glucopyranoside de benzyle (OH en position 3).

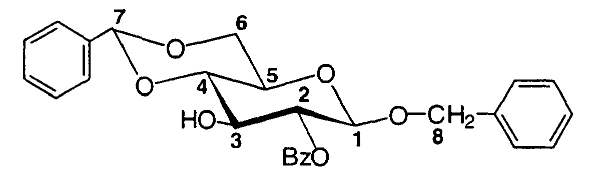

Dans 850 mL d'un mélange dichlorométhane/méthanol (4/1; v/v) sont introduits 42,0 g (1 éq.) de 2-*O*-benzoyl-4,6-benzylidène-3-*O*-(2-méthylnaphtyl)-β-D-glucopyranoside de benzyle (M = 602,7) et 47,5 g (3 éq.) de 2,3-dichloro-5,6-dicyano-1,4-benzoquinone (DDQ) à température ambiante. Au bout de 5h30', le milieu est dilué avec 1 L de dichlorométhane et lavé 2 fois avec une solution aqueuse de bicarbonate de sodium à 5%. Après séchage et évaporation de la phase organique, le résidu est purifié sur gel de silice [flash; éluant: toluène/acétate d'éthyle (9/1; v/v)]. On obtient ainsi 27,4 g de 2-*O*-benzoyl-4,6-*O*-benzylidène-β-D-glucopyranoside de benzyle attendu (M = 462,5).
CCM: Rf = 0,3 [toluène/acétate d'éthyle (9/1; v/v)].
Solide blanc.
Rendement (%) = 85.
RMN ¹³C (CDCl₃, 100 MHz): 165,97 (C=O); 136,97, 136,73, 133,37 (3 C quat. arom.); 130,05, 129,59, 129,37, 128,41, 127,93, 127,84, 126,37 (C arom.); 101,96 (C7); 99,75 (C1); 80,94 (C4); 74,82 (C2); 72,41 (C3); 70,63(C8); 68,66 (C6); 66,26 (C5).
RMN ¹H (CDCl₃, 400 MHz): 8,03-8,00 (m, 2H, H arom.); 7,62-7,58 (m, 1H, H arom.); 7,52-7,43 (m, 4H, H arom.); 7,41-7,36 (m, 3H, H arom.); 7,23-7,19 (m, 5H, H arom.); 5,57 (s, 1H, H7); 5,26 (dd, 1H, H2, J_{H2-H1} = 7,9 Hz, J_{H2-H3} = 9,1 Hz); 4,89 (dd, 1H, H8a, J_{H8a-H8b} = 12,5 Hz); 4,70 (d, 1H, H1, J_{H1-H2} = 7,9 Hz); 4,66 (d, 1H, H8b, J_{H8b-H8a} = 12,6 Hz); 4,42 (dd, 1H, H6a, J_{H6a-H5} = 5,0 Hz, J_{H6a-H6b} = 10,5 Hz); 3,99 (t, 1H, H3, J_{H3-H2} = J_{H3-H4} = 9,2 Hz); 3,85 (t, 1H, H6b, J_{H6b-H5} = J_{H6b-H6a} = 10,3 Hz); 3,68 (t, 1H, H4, J_{H4-H3} = J_{H4-H5} = 9,4 Hz); 3,49 (ddd, 1H, H5, J_{H5-H4} = J_{H5-H6b} = 9,7 Hz, J_{H5-H6a} = 5,1 Hz,); 2,84 (s, 1H, OH).

### EXEMPLE 6

### Synthèse d'un disaccharide selon l'invention.

Cet exemple illustre l'intérêt du procédé conforme à l'invention pour la préparation d'un disaccharide par réaction de couplage entre deux monosaccharides, le disaccharide obtenu pouvant lui-même servir de précurseur d'un disaccharide accepteur de glycosyle (voir exemple 7) pour la synthèse d'un trisaccharide (voir exemple 8).

### Synthèse du 2-O-benzoyl-4,6-O-benzylidène-3-O-(2-méthylnaphtyl)-β-D-glucopyranosyl-(1 → 3)- 2-O-benzoyl-4,6-O-benzylidène-β-D-glucopyranoside de benzyle.

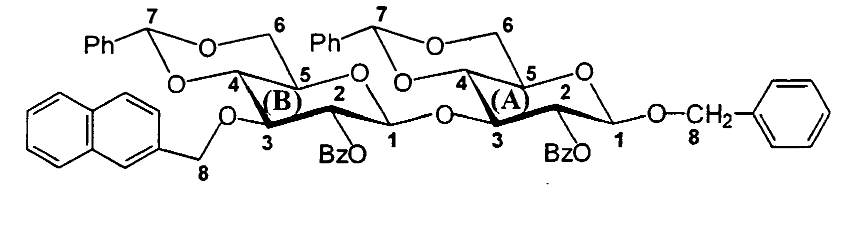

8,02 g (1,1 éq.) de 2-*O*-benzoyl-4,6-*O*-benzylidène-3-*O*-(2-méthylnaphtyl)-1-thio-β-D-glucopyranoside d'éthyle préparé à l'exemple 4 (M = 556,7) et 6,06 g (1 éq.) de 2-*O*-benzoyl-4,6-*O*-benzylidène-β-D-glucopyranoside de benzyle préparé à l'exemple 5 (M = 462,5) sont dissous dans 70 mL de dichlorométhane anhydre en présence de 2 g de tamis moléculaire 4Å à 0° C puis additionnés de 3,24 g (1,1 à 1,2 éq.) de N-iodosuccinimide (NIS) et 296 µL (0,1 éq.) de trifluorométhanesulfonate de triéthylsilyle (TESOTf). Après 50 min de réaction, le milieu est neutralisé par de la triéthylamine, filtré puis lavé par un solution de thiosulfate de sodium à 10% puis à l'eau. Après séchage et évaporation de la phase organique, le produit attendu est purifié par chromatographie sur gel de silice [flash; éluant: toluène/acétate d'éthyle (95/5; v/v)]. 10,36 g de 2-*O*-benzoyl-4,6-*O-*benzylidène-3-*O*-(2-méthylnaphtyl)-β-D-glucopyranosyl-( 1→3)-2-*O*-benzoyl-4,6-*O*-benzylidène-β-D-glucopyranoside de benzyle (M =957,1) sont ainsi obtenus.
CCM: Rf = 0,6 [toluène/acétate d'éthyle (8/2; v/v)].
Solide blanc.
Rendement (%) = 83%
RMN ¹³C (CDCl₃, 100 MHz): 164,74, 164,58 (C=O); 137,32, 137,19, 136,65, 135,33, 133,04, 132,96, 132,85, 132,72 (8 C quat. arom.); 129,82-125,67 (C arom.); 101,50, 101,08 (C7A, C7B); 100,58 (C1B); 99,62 (C1A); 80,89 (C4B); 79,30 (C4A); 78,22 (C3B); 77,79 (C3A); 73,69 (C2B); 73,52, 73,49 (C2A, C8B); 70,28 (C8A); 68,77, 68,73 (C6A, C6B); 66,48 (C5A); 66,00 (C5B).
RMN ¹H (CDCl₃, 400 MHz): 7,74-7,02 (m, 32H, H arom.); 5,53 (s, 1H, H7A ou H7B); 5,34 (s, 1H, H7A ou H7B); 5,31 (dd, 1H, H2A, J_{H2A-H1A} = 7,7 Hz, J_{H2A-H3A} = 9,2 Hz); 5,28 (t, 1H, H2B, J_{H2B-H1B} = J_{H2B-H3B} = 7,4 Hz); 4,85 (d, 1H, H1B, J_{H1B-H2B} = 7,0 Hz); 4,84 (d, 1H, H8aB, J_{H8aB-H8bB} = 12,4 Hz); 4,74 (d, 1H, H8bB, J_{H8bB-H8aB} = 12,2 Hz); 4,74 (d, 1H, H8aA, J_{H8aA-H8bA} = 12,2 Hz); 4,56 (d, 1H, H1A, J_{H1A-H2A} = 7,6 Hz); 4,50 (d, 1H, H8bA, J_{H8bA-H8aA} = 12,6 Hz); 4,36 (dd, 1H, H6aA, J_{H6aA-H5A} = 4,9 Hz, J_{H6aA-H6bA} = 10,5 Hz); 4,16 (dd, 1H, H6aB, J_{H6aB-H5B} = 4,9 Hz, J_{H6aB-H6bB} = 10,4 Hz); 4,11 (t, 1H, H3A, J_{H3A-H2A} = J_{H3A-H4A} = 8,8 Hz); 3,90 (t, 1H, H4B, J_{H4B-H3B} = J_{H4B-H5B} = 9,4 Hz); 3,84 (t, 1H, H4A, J_{H4A-H3A} = J_{H4A-H5A} = 9,3 Hz); 3,80 (dd, 1H, H6bA, J_{H6bA-H5A} = J_{H6bA-H6aA} = 10,3 Hz); 3,74 (dd, 1H, H3B, J_{H3B-H2B} = 7,9 Hz, J_{H3B-H4B} = 8,9 Hz); 3,68 (t, 1H, H6bB, J_{H6bB-H5B} = J_{H6bB-H6aB} = 10,3 Hz); 3,49 (ddd, 1H, H5A, J_{H5A-H4A} = J_{H5A-H6bA} = 9,7 Hz, J_{H5A-H6aA} = 4,9 Hz,); 3,37 (ddd, 1H, H5B, J_{H5B-H4B} = J_{H5B-H6bB} = 9,7 Hz, J_{H5B-H6aB} = 4,9 Hz).

### EXEMPLE 7

### Synthèse d'un disaccharide accepteur de glycosyle selon l'invention.

### Synthèse du 2-O-benzoyl-4,6-O-benzylidène-β-D-glucopyranosyl-(1→3)-2-O-benzoyl-4,6-O-benzylidène-β-D-glucopyranoside de benzyle

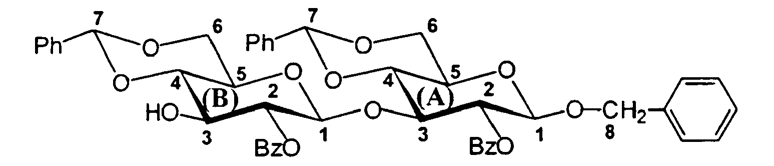

Après dissolution de 2,17 g (1éq.) de 2-*O*-benzoyl-4,6-*O*-benzylidène-3-*O-*(2-méthylnaphtyl)-β-D-glucopyranosyl-(1→3)-2-*O*-benzoyl-4,6-*O*-*b*enzylidène-β D-glucopyranoside de benzyle (M = 957,1) dans 43 mL d'un mélange dichlorométhane/méthanol (4/1; v/v), nous ajoutons 1,54 g (3 éq.) de 2,3-dichloro-5,6-dicyano-1,4-benzoquinone (DDQ) et on laisse évoluer 4h30 à température ambiante. Le milieu est ensuite dilué au dichlorométhane, lavé par un solution d'hydrogénocarbonate de sodium à 5% puis à l'eau. La phase organique est séchée, évaporée et le produit recherché est obtenu après purification sur gel de silice [flash; éluant: toluène/acétate d'éthyle (9/1; v/v)]. 1,46 g de 2-*O-*benzoyl-4,6-*O-*benzylidène-β-D-glucopyranosyl-(1→3)-2-*O*-benzoyl-4,6-*O*-benzylidène-β-D-glucopyranoside de benzyle (M = 815,9) sont ainsi recueillis.
CCM: Rf = 0,4 [toluène/acétate d'éthyle (8/2; v/v)].
Solide blanc.
Rendement (%) = 79%
RMN ¹³C (CDCl₃, 100 MHz): 165,64, 164,67 (C=O); 137,17, 136,97, 136,64, 133,10, 133,00 (5 C quat. arom.); 129,81-126,15 (C arom.); 101,69, 101,49 (C7A, C7B); 100,38 (C1B); 99,58 (C1A); 80,54 (C4B); 79,31 (C4A); 78,00 (C3A); 75,27 (C2B); 73,51 (C2A); 72,60 (C3B); 70,33 (C8A); 68,77 (C6A); 68,65 (C6B); 66,46 (C5A); 66,05 (C5B).
RMN ¹H (CDCl₃, 400 MHz): 7,70-6,96 (m, 25H, H arom.); 5,47 (s, 1H, H7A ou H7B); 5,27 (s, 1H, H7A ou H7B); 5,25 (t, 1H, H2A, J_{H2A-H1A}, = J_{H2A-H3A} = 7,6 Hz); 5,03 (dd, 1H, H2B, J_{H2B-H1B} = 7,4 Hz, J_{H2B-H3B} = 8,0 Hz); 4,83 (d, 1H, H1B, J_{H1B-H2B} = 7,2 Hz); 4,69 (d, 1H, H8aA, J_{H8aA-H8bA} = 12,5 Hz); 4,52 (d, 1H, H1A, J_{H1A-H2A} = 7,5 Hz); 4,44 (d, 1H, H8bA, J_{H8bA-H8aA} = 12,6 Hz); 4,30 (dd, 1H, H6aA, J_{H6aA-H5A} = 4,8 Hz, J_{H6aA-H6bA} = 10,4 Hz); 4,10 (dd, 1H, H6aB, J_{H6aB-H5B} = 4,8 Hz, J_{H6aB-H6bB} = 10,5 Hz); 4,08 (t, 1H, H3A, J_{H3A-H2A} = J_{H3A-H4A} = 8,7 Hz); 3,77 (t, 1H, H4A, J_{H4A-H3A} = J_{H4A-H5A} = 9,3 Hz); 3,76 (t, 1H, H3B, J_{H3B-H2B} = J_{H3B}-_{H4B} = 10,2 Hz); 3,73 (t, 1H, H6bA, J_{H6bA-H5A} = J_{H6bA-H6aA} = 10,3 Hz); 3,60 (t, 1H, H6bB, J_{H6bB}-_{H5B} = h_{16bB}-_{H6aB} = 10,3 Hz); 3,57 (t, 1H, H4B, J_{H4B-H3B} = J_{H4B-H5B} = 9,4 Hz); 3,44 (ddd, 1H, H5A, J_{H5A-H4A} = J_{H5A-H6bA} = 9,7 Hz, J_{H5A-H6aA} = 4,8 Hz,); 3,28 (ddd, 1H, H5B, J_{H5B-H4B} = J_{H58-H6bB} = 9,7 Hz, J_{H5B-H6aB} = 4,9 Hz,); 2,55 (s, 1H, OH).

### EXEMPLE 8

### Synthèse d'un trisaccharide selon l'invention.

Cet exemple illustre l'intérêt du procédé conforme à la présente invention pour la préparation d'un trisaccharide par réaction de couplage entre un monosaccharide et un disaccharide, le trisaccharide obtenu pouvant lui-même servir de précurseur d'accepteur de glycosyle (voir exemple 9) pour la synthèse d'un tétrasaccharide (voir exemple 10).

### Synthèse du 2-O-benzoyl-4,6-O-benzylidène-3-O-(2-méthylnaphtyl)-β-D-glucopyranosyl-(1→3)-2-O-benzoyl-4,6-O-benzylidène-β-D-glucopyranosyl-(1→3)-2-O-benzoyl-4,6-O-benzylidène-β-D-glucopyranoside de benzyle.

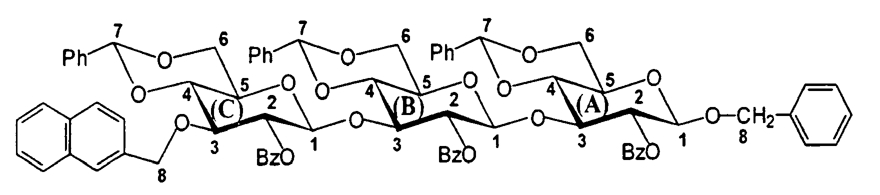

5,03 g (1,1 éq.) de 2-*O*-benzoyl-4,6-*O*-benzylidène-3-*O*-(2-méthylnaphtyl)-1-thio-β-D-glucopyranoside d'éthyle préparé à l'exemple 4 (M = 556,7) et 6,70 g (1 éq.) de 2-*O*-benzoyl-4,6-*O*-benzylidène-β-D-glucopyranosyl-(1→3)-2-*O-*benzoyl-4,6-*O*-benzylidène-β-D-glucopyranoside de benzyle préparé à l'exemple 7 (M = 815,9) sont dissous dans 100 mL de dichlorométhane anhydre en présence de 10 g de tamis tamis moléculaire 4Å à 0°C puis additionnés de 2,03 g de NIS (1,1 à 1,2 éq.) et 190 µL de TESOTf (0,1 éq.). Après 50 min de réaction, le milieu est neutralisé par de la triéthylamine, filtré puis lavé par un solution de thiosulfate de sodium à 10% puis à l'eau. Après séchage et évaporation de la phase organique, le produit attendu est purifié par chromatographie sur gel de silice [flash; éluant: [flash; éluant: toluène/acétate d'éthyle (95/5 puis 9/1; v/v). 9,28 g de 2-*O*-benzoyl-4,6-*O*-benzylidène-3-*O*-méthylnaphtyl-β-D-glucopyranosyl-(1→3)-2-*O*-benzoyl-4,6-*O*-benzylidène-β-D-glucopyranosyl-(1→3)-2-*O*-benzoyl-4,6-*O*-benzylidène-β-D-glucopyranoside de benzyle (M = 1310,5) sont ainsi isolés.
CCM: Rf = 0,5 [toluène/acétate d'éthyle (17/3; v/v)].
Solide blanc.
Rendement (%) = 86%
RMN ¹³C (CDCl₃, 100 MHz): 165,12, 164,77, 164,53 (3 C=O); 137,40, 137,36, 137,20, 136,78, 135,42, 133,43, 133,16 (2 C), 133,09, 132,90 (10 C quat. arom.); 129,93-125,37 (C arom.); 101,94, 101,22, 100,56 (C7A,B,C); 99,49 (C1A); 98,32 (C1C); 97,90 (C1B); 81,38 (C4C); 78,67 (C4A); 78, 19 (C3C); 77,61 (C4B); 76,22 (C3B); 74,31 (C2A); 74,11 (C3A); 73,87 (C8C); 73,28 (C2C); 72,58 (C2B); 70,22 (C8A); 68,81 (C6B); 68,77 (C6C), 68,67 (C6A); 66,49 (C5A); 66,14 (C5C); 65,37 (C5B).
RMN ¹H (CDCl₃, 400 MHz): 7,90-7,05 (m, 42H, H arom.); 5,47 (s, 1H, H7); 5,46 (s, 1H, H7); 5,34 (t, 1H, H2C, J_{H2C-H1C} = J_{H2C-H3C} = 7,8 Hz); 5,08 (t, 1H, H2B, J_{H2B-H1B} = J_{H2B-H3B} = 4,2 Hz); 5,06 (d, 1H, H1C, J_{H1C-H2C} = 7,4 Hz); 4,91 (d, 1H, H8aC, J_{H8aC-H8bC} = 12,3 Hz); 4,86 (d, 1H, H1B, J_{H1B-H2B} = 4,4 Hz); 4,86 (dd, 1H, H2A, J_{H2A-H1A} = 8,0 Hz, J_{H2A-H3A} = 8,6 Hz); 4,80 (d, 1H, H8bC, J_{H8bC-H8aC} = 12,4 Hz); 4,75 (d, 1H, H8aA, J_{H8aA-H8bA} = 12,5 Hz); 4,57 (s, 1H, H7); 4,49 (d, 1H, H8bA, J_{H8bA-H8aA} = 12,6 Hz); 4,45 (d, 1H, H1A, J_{H1A-H2A} = 7,7 Hz); 4,31 (dd, 1H, H6aA, J_{H6aA-H5A} = 4,8 Hz, J_{H6aA-H6bA} = 10,4 Hz); 4,22 (dd, 1H, H6aC, J_{H6aC-H5C} = 4,9 Hz, J_{H6aC-H6bC} = 10,4 Hz); 4,11 (dd, 1H, H6aB, J_{H6aB-H5B} = 3,5 Hz, J_{H6aB-H6bB} = 8,9 Hz); 4,07 (t, 1H, H3A, J_{H3A-H2A} = J_{H3A-H4A} = 9,1 Hz); 4,07 (dd, 1H, H4B, J_{H4B-H3B} = 8,1 Hz, J_{H4B-H5B} = 9,6 Hz); 3,97 (dd, 1H, H3B, J_{H3B-H2B} = 3,9 Hz, J_{H3B-H4B} = 8,1 Hz); 3,91 (t, 1H, H4C, J_{H4C-H3C} = J_{H4C}-_{H5C} = 9,1 Hz); 3,85 (dd, 1H, H3C, J_{H3C-H2C} = 8,1 Hz, J_{H3C-H4C} = 9,0 Hz); 3,72 (dd, 1H, H6bC, J_{H6bC-H5C} = J_{H6bC-H6aC} = 10,3 Hz); 3,64 (t, 1H, H6bA, J_{H6bA-H5A} = J_{H6bA-H6aA} = 10,2 Hz); 3,55 (ddd, ¹H, HSB, J_{H5B-H4B} = J_{H5B-H6bB} = 10,0 Hz, J_{H5B-H6aB} = 4,3 Hz,); 3,52 (t, 1H, H6bB, J_{H6bB-H5B} = J_{H6bB-H6aB} = 9,3 Hz); 3,50 (ddd, 1H, H5C, J_{H5C-H4C} = J_{H5C-H6bC} = 9,8 Hz, J_{H5C-H6aC} = 3,8 Hz,); 3,37 (ddd, 1H, HSA, J_{H5A-H4A} = J_{H5A-H6bA} = 9,8 Hz, J_{H5A-H6aA} = 4,9 Hz,); 3,18 (t, 1H, H4A, J_{H4A-H3A} = J_{H4A-H5A} = 9,4 Hz).

### EXEMPLE 9

### Synthèse d'un trisaccharide accepteur de glycosyle selon l'invention.

### Synthèse du 2-O-benzoyl-4,6-O-benzylidène-β-D-glucopyranosyl-(1→3)-2-O-benzoyl-4,6-O-benzylidène-β-D-glucopyranosyl-(1→3 )-2-O-benzoyl-4,6-O-benzylidène-β-D-glucopyranoside de benzyle.

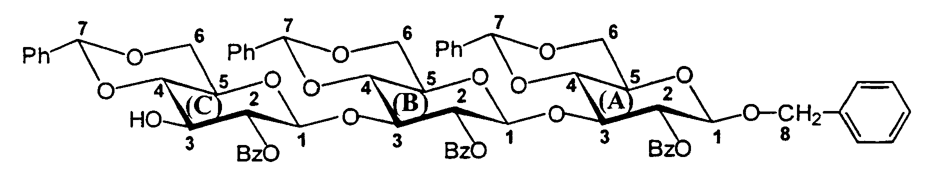

Après dissolution de 6,21 g (1éq.) de 2-*O*-benzoyl-4,6-*O*-benzylidène-3-*O-*(2-méthylnaphtyl)-(β-D-glucopyranosyl-(1→3)-2-*O*-benzoyl-4,6-*O*-benzylidène-(β-D-glucopyranosyl-(1→3)-2-*O*-benzoyl-4,6-*O*-benzylidène-β-D-glucopyranoside de benzyle (M = 1310,5) dans 125 mL d'un mélange dichlorométhane/méthanol (4/1; v/v), nous ajoutons 3,23 g (3 éq.) de DDQ et on laisse évoluer 7 h à température ambiante. Le milieu est ensuite dilué au dichlorométhane, lavé par un solution d'hydrogénocarbonate de sodium à 5% puis à l'eau. La phase organique est séchée, évaporée et le produit recherché est obtenu après purification sur gel de silice [flash; éluant: toluène/acétate d'éthyle (9/1 puis 17/3; v/v)]. 8,89 g de 2-*O-*benzoyl-4,6-*O*-benzylidène-β-D-glucopyranosyl-(1→3)-2-*O*-benzoyl-4,6-*O-*benzylidène-β-D-glucopyranosyl-(1→3)-2-*O*-benzoyl-4,6-*O*-benzylidène-β-D-glucopyranoside de benzyle (M = 1170,3) sont ainsi obtenus.
CCM: Rf= 0,3 [toluène/acétate d'éthyle (17/3; v/v)].
Solide blanc.
Rendement (%) = 78%
RMN ¹³C (CDCl₃, 100 MHz): 165,87, 164,81, 164,58 (3 C=O); 137,32, 137,18, 137,03, 136,75, 133,64, 133,23, 133,20 (7 C quat. arom.); 130,06-125,36 (C arom.); 101,96, 101,84, 100,57 (C7A,B,C); 99,48 (C1A); 98,18 (C1C); 98,03 (C1B); 80,85 (C4C); 78,72 (C4A); 77,64 (C4B); 76,31 (C3B); 74,73 (C2C); 74,40 (C3A); 74,28 (C2A); 72,66 (C2B); 72,54 (C3C); 70,26 (C8A); 68,78 (C6B); 68,67 (C6A, C6C), 66,48 (C5A); 66,09 (C5C); 65,40 (C5B).
RMN ¹H (CDCl₃, 400 MHz): 8,02-7,06 (m, 35H, H arom.); 5,47 (s, 1 H, H7); 5,42 (s, 1H, H7); 5,20 (dd, 1H, H2C, J_{H2C-H1C} = 7,6 Hz, J_{H2C-H3C} = 8,4 Hz); 5,13 (t, 1H, H2B, J_{H2B-H1B} = J_{H2B-H3B} = 4,2 Hz); 5,11 (d, 1H, H1C, J_{H1C-H2C} = 7,5 Hz); 4,95 (dd, 1H, H2A, J_{H2A-H1A} = 8,0 Hz, J_{H2A-H3A} = 8,6 Hz); 4,90 (d, 1H, H1B, J_{H1B-H2B} = 4,4 Hz); 4,76 (d, 1H, H8aA, J_{H8aA-H8bA} = 12,6 Hz); 4,62 (s, 1H, H7); 4,32 (d, 1H, H8bA, J_{H8bA-H8aA} = 12,6 Hz); 4,47 (d, 1H, H1A, J_{H1A-H1A} = 7,8 Hz); 4,31 (dd, 1H, H6aA, J_{H6aA-H5A} = 4,8 Hz, J_{H6aA-H6bA} = 10,4 Hz); 4,22 (dd, 1H, H6aC, J_{H6aC-H5C} = 4,8 Hz, J_{H6aC-H6bC} = 10,4 Hz); 4,12 (dd, 1H, H6aB, J_{H6aB-H5B} = 3,3 Hz, J_{H6aB-H6bB} = 8,8 Hz); 4,09 (t, 1H, H3A, J_{H3A-H2A} = J_{H3A-H4A} = 9,0 Hz); 4,07 (dd, 1H, H4B, J_{H4B-H3B} = J_{H4B-H5B} = 8,9 Hz); 4,01 (dd, 1H, H3B, J_{H3B-H2B} = 3,8 Hz, J_{H3B-H4B} = 8,1 Hz); 3,96 (ddd, 1H, H3C, J_{H3C-H2C} = J_{H3C-H4C} = 8,7 Hz, J_{H3C-OH} = 3,6 Hz); 3,69 (dd, 1H, H6bC, J_{H6bC-H5C} = J_{H6bC-H6aC} = 10,2 Hz); 3,67 (t, 1H, H4C, J_{H4C-H3C} = J_{H4C-H5C} = 9,4 Hz); 3,65 (t, 1H, H6bA, J_{H6bA-H5A} = J_{H6bA-H6aA} = 10,6 Hz); 3,56 (ddd, 1H, H5B, J_{H5B-H4B} = J_{H5B-H6bB} = 9,7 Hz, J_{H5B-H6aB} = 4,1 Hz,); 3,55-3,47 (m, 2H, H6bB, H5C); 3,38 (ddd, 1H, H5A, J_{H5A-H4A} = J_{H5A-H6bA} = 10,0 Hz, J_{H5A-H6aA} = 4,9 Hz,); 3,21 (t, 1H, H4A, J_{H4A-H3A} = J_{H4A-H5A} = 9,4 Hz); 2,64 (d, 1H, OH, J_{OH-H3C} = 3,7 Hz).

### EXEMPLE 10

### Synthèse d'un tétrasaccharide selon l'invention.

Cet exemple illustre l'intérêt du procédé conforme à la présente invention pour la préparation d'un tétrasaccharide par réaction de couplage entre un monosaccharide et un trisaccharide, le tétrasaccharide obtenu pouvant lui-même servir de précurseur d'accepteur de glycosyle tétrasaccharidique (voir exemple 11) pour la synthèse d'un pentasaccharide, et ainsi de suite.

### Synthèse du 2-O-benzoyl-4,6-O-benzylidène-3-O-(2-méthylnaphtyl)-β-D-glucopyranosyl-(1→3)-2-O-benzoyl-4,6-O-benzylidène-β-D-glucopyranosyl-(1→3)-2-O-benzoyl-4,6-O-benzylidène-β-D-glucopyranosyl-(1→3)-2-O-benzoyl-4,6-O-benzylidène-β-D-glucopyranoside de benzyle.

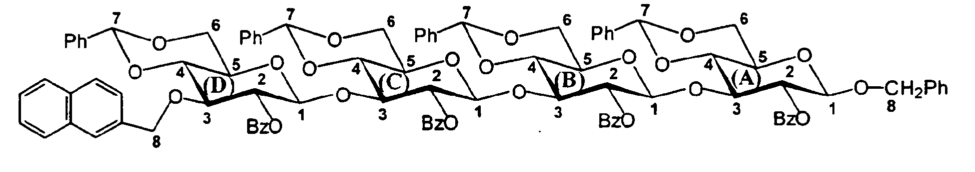

3,23 g (1,1 éq.) de 2-*O*-benzoyl-4,6-*O*-benzylidène-3-*O*-(2-méthylnaphtyl)-1-thio-β-D-glucopyranoside d'éthyle préparé à l'exemple 4 (M = 556,7) et 6,17 g (1 éq.) de 2-*O*-benzoyl-4,6-*O*-benzylidène-β-D-glucopyranosyl-(1→3)-2-*O-*benzoyl-4,6-*O*-benzylidène-β-D-glucopyranosyl-(1→3)-2-*O*-benzoyl-4,6-*O-*benzylidène-β-D-glucopyranoside de benzyle préparé à l'exemple 9 (M = 1170,3) sont dissous dans 100 mL de dichlorométhane anhydre en présence de 10 g de tamis moléculaire 4Å à 0° C puis additionnés de 1,42 g (1,2 éq.) de NIS et 100 µL (0,1 éq.) de trifluorométhanesulfonate de triméthylsilyle (TMSOTf). Après 1 h de réaction, le milieu est neutralisé par de la triéthylamine, filtré puis lavé par un solution de thiosulfate de sodium à 10% puis à l'eau. Après séchage et évaporation de la phase organique, le produit attendu est purifié par chromatographie sur gel de silice [flash; éluant: toluène/acétate d'éthyle (95/5 puis 925/75 puis 9/1; v/v). 7,70 g de 2-*O*-benzoyl-4,6-*O*-benzylidène-3-*O*-(2-méthylnaphtyl)-β-D-glucopyranosyl-( 1→3)-2-*O*-benzoyl-4,6-*O*-benzylidène-*O*-D-glucopyranosyl-(1→3 )-2-*O*-benzoyl-4,6-*O*-benzylidène-β-D-glucopyranosyl-(1→3)-2-*O*-benzoyl-4,6-*O*-benzylidène-β-D-glucopyranoside de benzyle (M = 1664,9) sont ainsi recueillis.
CCM: Rf = 0,5 [toluène/acétate d'éthyle (8/2; v/v)].
Solide blanc.
Rendement (%) = 88%
RMN ¹³C (CDCl₃, 100 MHz): 165,05, 164,68, 164,58, 164,55 (4 C=O); 137,36, 137,32, 137,24, 137,17, 136,69, 135,41, 133,41, 133,36, 133,09, 133,05 (2 C), 132,85 ( 12 C quat. arom.); 129,80-125,33 (C arom.); 101,82, 101,16, 101,08, 100,77 (C7A,B,C,D); 99,49 (C1A); 99,04 (C1D); 98,42 (C1B); 96,94 (C1C); 81,28 (C4D); 78,82 (C4A); 78,28, 78,16 (C3D, C4C); 77,41 (C4B); 76,90 (C3C); 75,07 (C3A); 74,16 (C3B); 73,96 (C2A); 73,77 (C8D); 73,47 (C2B); 73,39 (C2D); 72,54 (C2C); 70,24 (C8A); 68,71, 68,67 (C6A,B,C,D); 66,44, 66,04, 65,52 (C5A,B,C,D).
RMN ¹H (CDCl₃, 400 MHz): 7,78-7,04 (m, 52H, H arom.); 5,53 (s, 1H, H7); 5,43 (s, 1H, H7); 5,33 (t, 1H, H2D, J_{H2D-H1D} = J_{H2D-H3D} = 7,8 Hz); 5,12 (t, 1H, H2C, J_{H2C-H1C} = J_{H2C-H3C} = 5,3 Hz); 4,98 (d, 1H, H1D, J_{H1D-H2D} = 7,4 Hz); 4,98 (dd, 1H, H2A, J_{H2A-H1A} = 8,0 Hz, J_{H2A-H3A} = 8,5 Hz); ); 4,95 (d, 1H, H1C, J_{H1C-H2C} = 5,4 Hz); 4,89 (d, 1H, H8aD, J_{H8aD-H8bD} = 12,5 Hz); 4,82 (s, 1H, H7); 4,79 (d, 1H, H8bD, J_{H8bD-H8aD} = 11,8 Hz); 4,76 (d, 1H, H8aA, J_{H8aA-H8bA} = 11,4 Hz); 4,75 (d, 1 H, H1B, J_{H1B-H2B} = 3,1 Hz); 4,75 (m, 1H, H2B); 4,74 (s, 1H, H7); 4,50 (d, 1H, H8bA, J_{H8bA-H8aA} = 12,6 Hz); 4,47 (d, 1H, H1A, J_{H1A-H2A} = 7,8 Hz); 4,34 (dd, 1H, H6a, J_{H6a-H5} = 4,6 Hz, J_{H6a-H6b} = 10,3 Hz); 4,19 (dd, 1H, H6a, J_{H6a-H5} = 4,9 Hz, J_{H6a-H6b} = 10,4 Hz); 4,12-4,09 (m, 2H, H6); 4,04 (t, 1H, H3A, J_{H3A-H2A} = J_{H3A-H4A} = 8,7 Hz); 4,03-4,00 (m, 1H, H3C); 3,95 (t, 1H, H4C, J_{H4C-H3C} = J_{H4C-H5C} = 8,8 Hz); 3,92-3,89 (m, 1H, H3B); 3,87 (dd, 1H, H4D, J_{H4D-H3D} = 9,3 Hz, J_{H4D-H5D} = 8,7 Hz); 3,82 (dd, 1H, H3D, J_{H3D-H2D} = 8,0 Hz, J_{H3D-H4D} = 9,1 Hz);3,75-3,68 (m, 2H, H6); 3,59-3,37 (m, 5H, H4B, 4 H5, 2 H6); 3,35 (t, 1H, H4A, J_{H4A-H3A} = J_{H4A-H5A} = 9,2 Hz).

### EXEMPLE 11

### Synthèse d'un tétrasaccharide accepteur selon l'invention.

### Synthèse du 2-O-benzoyl-4,6-O-benzylidène-β-D-glucopyranosyl-(1→3)-2-O-benzoyl-4,6-O-benzylidène-β-D-glucopyranosyl-(1→3)-2-O-benzoyl-4,6-O-benzylidène-β-D-glucopyranosyl-(1→3)-2-O-benzoyl-4,6-O-benzylidène-β-D-glucopyranoside de benzyle

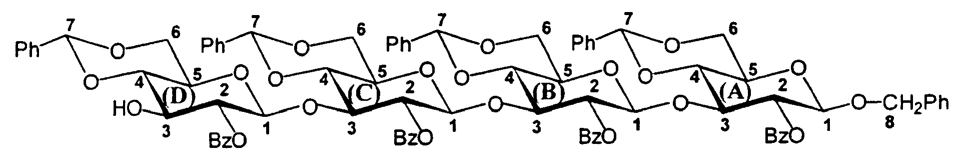

Après dissolution de 3,79 g (1éq.) de 2-*O*-benzoyl-4,6-*O*-benzylidène-3-*O-*méthylnaphtyl-β-D-glucopyranosyl-(1→3)-2-*O*-benzoyl-4,6-*O*-benzylidène-β-D-glucopyranosyl-( 1 →3)-2-*O*-benzoyl-4,6-*O*-benzylidène-β-D-glucopyranosyl-(1→3)-2-*O*-benzoyl-4,6-*O*-benzylidène-β-D-glucopyranoside de benzyle (M = 1664,9) dans 55 mL d'un mélange dichlorométhane/méthanol (4/1; v/v), nous ajoutons 1,55 g (3 éq.) de DDQ et on laisse évoluer 7h30 à température ambiante. Le milieu est ensuite dilué au dichlorométhane, lavé par un solution d'hydrogénocarbonate de sodium à 5% puis à l'eau. La phase organique est séchée, évaporée et le produit recherché est obtenu après purification sur gel de silice [flash; éluant: toluène/acétate d'éthyle (17/3; v/v)]. 2,78 g de 2-*O*-benzoyl-4,6-*O-*benzylidène-β-D-glucopyranosyl-(1→3)-2-*O*-benzoyl-4,6-D-benzylidène-β-D-glucopyranosyl-( 1→3)-2-*O*-benzoyl-4,6-*O*-benzylidène-β-D-glucopyranosyl-(1→3)-2-*O*-benzoyl-4,6-*O*-benzylidène-β-D-glucopyranoside de benzyle (M = 1523,7) sont ainsi isolés.
CCM: Rf = 0,3 [toluène/acétate d'éthyle (8/2; v/v)].
Solide blanc.
Rendement (%) = 80%
RMN ¹³C (CDCl₃, 100 MHz): 165,80, 164,66, 164,61, 164,59 (4 C=O); 137,28, 137,20, 137,13, 137,00, 136,65, 133,62, 133,42, 133,10, 133,06 (9 C quat. arom.); 129,87-125,31 (C arom.); 101,80, 101,71, 101,10, 100,71 (C7A,B,C,D); 99,46 (C1A); 98,76 (C1D); 98,45 (C1B); 96,98 (C1C); 80,73 (C4D); 78,80 (C4A); 78,20 (C4C); 77,42 (C4B); 76,84 (C3C); 75,14 (C3A); 74,75 (C2D); 74,30 (C3B); 73,93 (C2A); 73,47 (C2B); 72,53 (C2C); 72,38 (C3D); 70,22 (C8A); 68,62 (C6A,B,C,D); 66,41, 66,00, 65,50 (C5A,B,C,D).
RMN ¹H (CDCl₃, 400 MHz): 7,91-7,04 (m, 45H, H arom.); 5,51 (s, 1H, H7); 5,39 (s, 1H, H7); 5,16 (dd, 1H, H2D, J_{H2D-H1D} = 7,6 Hz, J_{H2D-H3D} = 8,4 Hz); 5,15 (t, 1H, H2C, J_{H2C-H1C} = J_{H2C-H3C} = 5,3 Hz); 5,02 (d, 1H, H1D, J_{H1D-H2D} = 7,6 Hz); 4,99 (dd, 1H, H2A, J_{H2A-H1A} = 8,0 Hz, J_{H2A-H3A} = 9,6 Hz); 4,97 (d, 1H, H1C, J_{H1C-H2C} = 5,3 Hz); 4,83 (s, 1H, H7); 4,82 (t, 1H, H2B, J_{H2B-H1B} = J_{H2B-H3B} = 5,2 Hz); 4,76 (d, 1H, H1B, J_{H1B-H2B} = 5,6 Hz); 4,75 (d, 1H, H8aA, J_{H8aA-H8bA} = 10,8 Hz); 4,74 (s, 1H, H7); 4,50 (d, 1H, H8bA, J_{H8bA-H8aA} = 13,4 Hz); 4,47 (d, 1H, H1A, J_{H1A-H2A} = 7,9 Hz); 4,34 (dd, 1H, H6a, J_{H6a-H5} = 4,6 Hz, J_{H6a-H6b} = 10,4 Hz); 4,18 (dd, 1H, H6a, J_{H6a-H5} = 4,9 Hz, J_{H6a-H6b} = 10,4 Hz); 4,12-4,09 (m, 2H, H6a); 4,05 (dd, 1H, H3C, J_{H3C-H2C} = 4,9 Hz, J_{H3C-H4C} = 8,3 Hz); 4,04 (t, 1H, H3A, J_{H3A-H2A} = J_{H3A-H4A} = 8,8 Hz); 3,95 (dd, 1H, H4C, J_{H4C-H3C} 8,7 Hz, J_{H4C-H5C} = 9,1 Hz); ); 3,91 (dd, 1H, H3B, J_{H3B-H2B} = 5,6 Hz, J_{H3B-H4B} = 8,5 Hz); 3,89 (ddd, 1H, H3D, J_{H3D-H2D} = JH3D-H4D = 8,9 Hz, J_{H3D-OH} = 4,0 Hz); 3,72 (t, 1H, H6b, J_{H6b-H5} = J_{H6b-H6a} = 9,9 Hz); 3,67 (t, 1H, H6b, J_{H6b-H5} = J_{H6b-H6a} = 10,1 Hz); 3,60 (t, 1H, H4D, J_{H4D-H3D} = J_{H4D-H5D} = 9,4 Hz); 3,58-3,54 (m, 1H, H5C); 3,51 (t, 1H, H6b, J_{H6b-H5} = J_{H6b-H6a} = 10,0 Hz); 3,47-3,37 (m, 5H, H4B, H5A,B,D, H6b); 3,35 (t, 1H, H4A, J_{H4A-H3A} = J_{H4A-H5A} = 9,2 Hz); 2,73 (d, 1H, OH, J_{OH-H3C} = 3,8 Hz).

### EXEMPLE 12

### Autre exemple de synthèse d'un synthon donneur de glycosyle de formule générale (Ia) selon l'invention.

Préparation du 2-*O*-acétyl-3-*O*-allyl-4,6-di-*O*-benzyl-1-thio-β-D-glucopyranoside d'éthyle selon les étapes 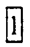 à 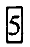 , 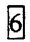 à 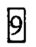 puis 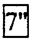 dont l'homme de métier pourra déterminer aisément les conditions réactionnelles les plus appropriées, notamment en suivant l'enseignement des exemples décrits précédemment. 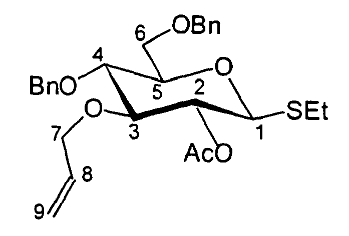 M = 486 g/mol.
CCM: Rf = 0,6 [éther de pétrole/acétate d'éthyle (8/2; v/v)].
Huile incolore.
RMN ¹³C (CDCl₃, 100 MHz) δ (ppm): 169,68 (C=O); 138,21, 137,97 (C quat. arom.); 134,74 (C8); 128,52, 128,44, 128,17, 127,96, 127,78, 127,68 (C arom.); 117,02 (C9) 84,17 (C3); 83,39 (C1); 79,49 (C5); 77,66 (C4); 75,17 (CH₂-Ar); 74,12 (C7); 73,51 (CH₂-Ar); 71,83 (C2); 68,92 (C6); 23,85 (S-CH₂); 21,18 (CH3-CO); 14,97 (S-CH₂-CH₃).
RMN ¹H (CDCl₃, 400 MHz) δ (ppm): 7,34-7,21 (m, 10H, H arom.); 5,87 (ddt, 1H, H8, J_{H8-H7a} = J_{H8-H7b} = 5,7 Hz, J_{H8-H9a} = 17,2 Hz, J_{H8-H9b} = 10,4 Hz); 5,24 (ddt, 1H, H9a, J_{H9a-H7} = 1,6 Hz, J_{H9a-H8} = 17,2 Hz, J_{H9a-H9b} = 1,7 Hz); 5,15 (ddt, 1H, H9b, J_{H9b-H7} = 1,2 Hz, J_{H9b-H8} = 10,4 Hz, J_{H9b-H9a} = 1,5 Hz); 4,97 (dd, 1H, H2, J_{H2-H1} = 9,9 Hz, J_{H2-H3} = 9,2 Hz); 4,80 (d, 1H, C*H*_{*2*-}Ar_{,} ²J = 10,7 Hz); 4,60 (d, 1H, C*H₂*-Ar, ²J = 12,2 Hz); 4,56 (d, 1H, C*H₂*-Ar, ²J = 10,8 Hz); 4,54 (d, 1H, C*H₂*-Ar, ²J = 12,1 Hz); 4,35 (d, 1H, H1, J_{H1-H2} = 10,0 Hz); 4,27 (ddt, 1H, H7a, J_{H7a-H7b} = 12,6 Hz, J_{H7a-H8} = 5,6 Hz, J_{H7a-H9} = 1,4 Hz); 4,16 (ddt, 1H, H7b, J_{H7b-H7a} = 12,6 Hz, J_{H7b-H8} = 5,9 Hz, J_{H7b-H9} = 1,4 Hz); 3,74 (dd, 1H, H6a, J_{H6a-H5} = 2,0 Hz, J_{H6a-H6b} = 11,1 Hz); 3,69 (dd, 1H, H6b, J_{H6b-H5} = 4,5 Hz, J_{H6b-H6a} = 11,0 Hz); 3,63 (t, 1H, H4, J_{H4-H3} = J_{H4-H5} = 9,4 Hz); 3,54 (t, 1H, H3, J_{H3-H2} = J_{H3-H4} = 9,0 Hz); 3,47 (ddd, 1H, H5, J_{H5-H4} = 9,7 Hz, J_{H5-H6a} = 1,9 Hz, J_{H5-H6b} = 4,4 Hz); 2,69 (qd, 2H, S-C*H*₂-CH₃, J = 7,4 Hz, J = 9,6 Hz); 2,11 (s, 3H, CH₃-CO); 1,26 (t, 3H, S-CH₂-C*H*₃, J = 7,4 Hz).

### EXEMPLE 13

### Synthèse d'un synthon selon l'invention.

Cet exemple illustre la préparation d'un synthon selon l'invention qui peut être donneur de glycosyle, ou accepteur de glycosyle par réaction avec un donneur plus actif comme par exemple un trichloroacétimidate. Synthèse du 2-*O*-acétyl-4,6-di-*O*-benzyl-1-thio-β-D-glucopyranoside d'éthyle.

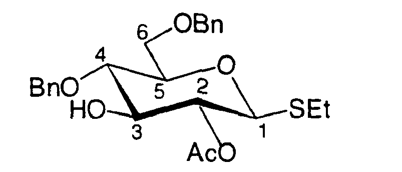

A 1,58 g de 2-*O*-acétyl-3-*O*-allyl-4,6-di-*O*-benzyl-1-thio-β-3-D-glucopyranoside d'éthyle (M = 486) dans 56 mL d'un mélange éthanol/toluène/eau (8/3/1; v/v/v) sont ajoutés 2,74 g (7,5 éq.) de Dabco et 450 mg (0,15 éq.) de catalyseur de Wilkinson. Le milieu est porté à reflux pendant 2 heures puis concentré. Le mélange est repris au dichlorométhane, lavé à l'eau glacée, avec HCl 5% glacé, NaHCO₃ 5% glacé, à l'eau glacée, séché (MgSO₄) et mis à sec.

Le résidu est dissous dans 30 mL d'un mélange acétone/HCl aq. 10% (19/1; v/v) qui est porté à reflux pendant 8 min, puis refroidi à T.A., neutralisé avec NaHCO₃ 5% et concentré. Après reprise au dichlorométhane et lavages à l'eau, le produit est purifié sur gel de silice [flash; éluant: toluène/acétate d'éthyle (9/1; v/v)] et on isole 1 g de 2-*O*-acétyl-4,6-di-*O*-benzyl-1-thio-β-D-glucopyranoside d'éthyle (M = 446).
CCM: Rf = 0,2 [toluène/acétate d'éthyle (9/1; v/v)].
Huile incolore.
Rendement (%) = 69.
RMN ¹³C (CDCl₃, 100 MHz) δ (ppm): 170,62 (C=O); 138,11, 138,04 (C quat. arom.); 128,65, 128,44, 128,14, 128,10, 127,84, 127,73 (C arom.); 83,12 (C1); 79,20 (C5); 78,04 (C4); 76,92 (C3); 74,90 (CH₂-Ar); 73,56 (CH₂-Ar); 72,54 (C2); 68,90 (C6); 23,97 (S-CH₂); 21,10 (CH3-CO); 15,00 (S-CH₂-CH₃).
RMN ¹H (CDCl₃, 400 MHz) δ (ppm): 7,28-7,09 (m, 10H, H arom.); 4,80 (dd, 1H, H2, J_{H2-H1} = 9,9 Hz, J_{H2-H3} = 9,2 Hz); 4,68 (d, 1H, C*H*₂-Ar, ²J = 11,2 Hz); 4,56 (d, 1H, CH,-Ar, ²j = 11.2 Hz); 4.56 (d, 1H, C*H*₂-Ar, ²j = 12,0 Hz); 4,46 (d, 1H, CH₂-Ar, ²J = 12,0 Hz); 4,31 (d, 1H, H1, J_{H1-H2} = 10,0 Hz); 3,72-3,64 (m, 3H, H3, H6a, H6b); 3,51 (dd, 1H, H4, J_{H4-H3} = J_{H4-H5} = 9,3 Hz); 3,40 (m, 1H, H5); 2,64 (qd, 2H, S-CH₂, J = 7,4 Hz, J = 9,6 Hz); 2,34 (d, 1H, OH, J = 3,9 Hz); 2,06 (s, 3H, CH₃-CO); 1,20 (t, 1H, C*H*₃-CH₂-S, J = 7,4 Hz).

### EXEMPLE 14

### Synthèse d'un synthon donneur de glycosyle de formule générale (Ib) selon l'invention.

### Synthèse du 2-O-acétyl-3-O-allyl-4,6-di-O-benzyl-α-D-glucopyranosyl trichloroacétimidate.

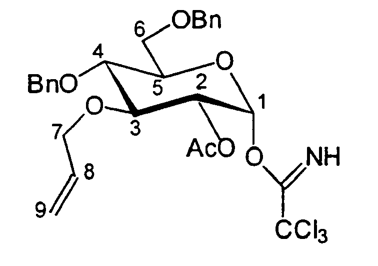

### Etape 14A

A 4,68 g (1 éq.) de 2-*O*-acétyl-3-*O*-allyl-4,6-di-*O*-benzyl-1-thio-β-D-glucopyranoside d'éthyle (M = 486) dans 100 mL d'un mélange acétone/eau (8/2; v/v) sont additionnés de 3,41 g (2 éq.) de *N*-bromosuccinimide. La réaction terminée, le milieu est dilué au dichlorométhane, lavé avec une solution d'hydrogénocarbonate de sodium à 5% puis à l'eau, séché (MgSO₄) et concentré. CCM: Rf = 0,3 [éther de pétrole/acétate d'éthyle (7/3; v/v)].

### Etape 14B

Le résidu précédemment obtenu (M = 442) est alors dissous dans 40 mL de dichlorométhane anhydre et sont introduits 4,83 mL (5 éq.) de trichloroacétonitrile et 290 µL (0,2 éq.) goutte à goutte de DBU. Après 1 h de réaction à température ambiante, le milieu est concentré et la purification sur gel de silice [flash; éluant: éther de pétrole/acétate d'éthyle/triéthylamine (85/15/1; v/v/v) permet d'isoler 4,38 g de 2-*O*-acétyl-3-*O*-allyl-4,6-di-*O*-benzyl-α-D-glucopyranosyl trichloroacétimidate (M = 586,5).
CCM: Rf = 0,7 [éther de pétrole/acétate d'éthyle (8/2; v/v)].
Huile incolore.
Rendement (%) = 78.
RMN ¹³C (CDCl₃, 100 MHz) δ (ppm): 170,11 (C=O); 161,10 (C=NH); 137,92, 137,91 (C quat. arom.); 134,84 (C8); 128,55, 128,49, 128,30, 128,23, 128,17, 128,05, 128,00, 127,83 (C arom.); 116,92 (C9); 94,09 (Cl); 79,30 (C3); 76,86 (C5); 75,52 (CH₂-Ar); 74,37 (C7); 73,58 (CH₂-Ar); 73,45 (C4); 72,49 (C2); 67,94 (C6); 20,82 (CH3).
RMN ¹H (CDCl₃, 400 MHz) δ (ppm): 8,57 (s, 1H, NH); 7,34-7,20 (m, 10H, H arom.); 6,52 (d, 1H, H1, J_{H1-H2} = 3,6 Hz); 5,91 (ddt, 1H, H8, J_{H8-H7a} = J_{H8-H7b} = 5,7 Hz, J_{H8-H9a} = 17,2 Hz, J_{H8-H9b} = 10,4 Hz); 5,27 (ddt, 1H, H9a, J_{H9a-H7} = 1,6 Hz, J_{H9a-H8} = 17,2 Hz, J_{H9a-H9b} = 1,7 Hz); 5,16 (ddt, 1H, H9b, J_{H9b-H7} = 1,2 Hz, J_{H9b-H8} = 10,4 Hz, J_{H9b-H9a} = 1,5 Hz); 5,02 (dd, 1H, H2, J_{H2-H1} = 3,6 Hz, J_{H2-H3} = 9,9 Hz); 4,84 (d, 1 H, C*H*₂-Ar, ²J = 10,6 Hz); 4,63 (d, 1H, C*H*₂-Ar, ²J = 12,0 Hz); 4,56 (d, 1H, C*H*₂-Ar, ²J = 10,5 Hz); 4,50 (d, 1H, C*H*₂-Ar, ²J = 12,1 Hz); 4,33-4,32 (m, 2H, H7a, H7b); 3,97 (t, 2H, H3, H4, J_{H3-H2} = J_{H3-H4} = J_{H4-H3} = J_{H4-H5} = 9,5 Hz); 3,84-3,78 (m, 2H, H5, H6a); 3,68 (dd, 1H, H6b, J_{H6b-H5} = 1,6 Hz, J_{H6b-H6a} = 11,1 Hz); 2,04 (s, 3H, CH₃).

### EXEMPLE 15

### Synthèse d'un synthon accepteur de glycosyle de formule générale (II) selon l'invention.

### Etape 15A

### Préparation du précurseur 2-O-acétyl-3-O-allyl-4,6-di-O-benzyl-β-D-glucopyranoside de benzyle.

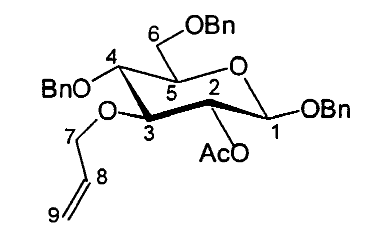

A 3,07 g (1 éq.) de 2-*O*-acétyl-3-*O*-allyl-4,6-di-*O*-benzyl-α-D-glucopyranosyl trichloroacétimidate (M = 586,5) dans 25 mL de dichlorométhane à 0°C en présence de 2,5 g de tamis moléculaire 4Å, sont additionnés 1,62 mL (3éq.) d'alcool benzylique et 130 µL (0,1 éq.) de TESOTf. Après 1 h de réaction à 0°C, le milieu est neutralisé avec de la triéthylamine, filtré et concentré. La purification sur gel de silice [flash; éluant: toluène/acétate d'éthyle (98/2; v/v)] permet d'isoler 2,52 g de 2-*O*-acétyl-3-*O*-allyl-4,6-di-*O*-benzyl-β-D-glucopyranoside de benzyle (M = 532).
CCM: Rf = 0,5 [toluène/acétate d'éthyle (9/1; v/v)].
Huile incolore.
Rendement (%) = 91.
RMN ¹³C (CDCl₃, 100 MHz) δ (ppm): 169,55 (C=O); 138,20, 137,99, 137,47 (C quat. arom.); 134,75 (C8); 128,51, 128,46, 128,41, 128,13, 128,01, 127,94, 127,83, 127,74, 127,71, 127,67, 127,64 (C arom.); 117,04 (C9); 99,72 (C1); 82,73 (C3); 77,78 (C4); 75,22 (C5); 75,09 (CH₂-Ar); 73,94 (C7); 73,58 (CH₂-Ar); 73,08 (C2); 70,36 (CH₂-Ar sur C1); 68,81 (C6); 21,13 (CH3).
RMN ¹H (CDCl₃, 400 MHz) δ (ppm): 7,36-7,20 (m, 15H, H arom.); 5,85 (ddt, 1H, H8, J_{H8-H7a} = J_{H8-H7b} = 5,7 Hz, J_{H8-H9a} = 17,2 Hz, J_{H8-H9b} = 10,4 Hz); 5,22 (ddt, 1H, H9a, J_{H9a-H7} = 1,6 Hz, J_{H9a-H8} = 17,2 Hz, J_{H9a-H9b} = 1,7 Hz); 5,13 (ddt, 1H, H9b, J_{H9b-H7} = 1,2 Hz, J_{H9b-H8} = 10,4 Hz, J_{H9b-H9a} = 1,5 Hz); 5,02 (dd, 1H, H2, J_{H2-H1} = 8,0 Hz, J_{H2-H3} = 9,4 Hz); 4,89 (d, 1H, C*H*₂-Ar, ²J = 12,4 Hz); 4,80 (d, 1H, C*H*₂-Ar, ²J = 10,7 Hz); 4,62 (d, 1H, C*H*₂-Ar, ²J = 12,0 Hz); 4,61 (d, 1H, C*H*₂-Ar sur C1, ²J = 11,3 Hz); 4,56 (d, 1H, C*H*₂-Ar, ²J = 12,2 Hz); 4,55 (d, 1H, C*H*₂-Ar, ²J = 10,7 Hz); 4,40 (d, 1H, H1, J_{H1-H2} = 8,0 Hz); 4,24 (ddt, 1H, H7a, J_{H7a-H7b} = 12,6 Hz, J_{H7a-H8} = 5,6 Hz, J_{H7a-H9} = 1,4 Hz); 4,12 (ddt, 1H, H7b, J_{H7b-H7a} = 12,6 Hz, J_{H7b-H8} = 5,9 Hz, J_{H7b-H9} = 1,4 Hz); 3,75 (dd, 1H, H6a, J_{H6a-H5} = 2,0 Hz, J_{H6a-H6b} = 11,0 Hz); 3,70 (dd, 1H, H6b, J_{H6b-H5} = 4,7 Hz, J_{H6b-H6a} = 10,9 Hz); 3,64 (t, 1H, H4, J_{H4-H3} = J_{H4-H5} = 9,4 Hz); 3,51 (t, 1H, H3, J_{H3-H2} = J_{H3-H4} = 9,3 Hz); 3,45 (ddd, 1H, H5, J_{H5-H4} = 9,6 Hz, J_{H5-H6a} = 2,0 Hz, J_{H5-H6b} = 4,6 Hz); 2,06 (s, 3H, CH₃).

### Etape 15B

### Préparation du synthon accepteur 2-O-acétyl-4,6-di-O-benzyl-β-D-glucopyranoside de benzyle (OH en position 3).

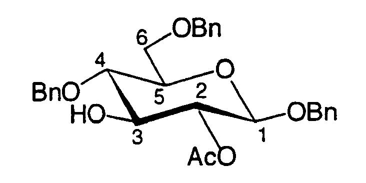

2,44 g (1 éq.) de 2-*O*-acétyl-3-*O*-allyl-4,6-di-*O*-benzyl-β-D-glucopyranoside de benzyle (M = 532) sont dissous dans 85 mL d'un mélange éthanol/toluène/eau (8/3/1; v/v/v) puis additionnés de 3,86 g (7,5 éq.) de Dabco et 640 mg (0,15 éq.) de catalyseur de Wilkinson. Le milieu est porté à reflux pendant 2 heures puis concentré. Le mélange est repris au dichlorométhane, lavé à l'eau glacée, avec HCl 5% glacé, NaHCO₃ 5% glacé, à l'eau glacée, séché (MgSO₄) et concentré.

Le résidu est alors dissous dans 48 mL d'un mélange acétone/HCl aq. 10% (l9/1; v/v) et la solution est porté à reflux pendant 8 min, puis refroidie à T.A., neutralisée avec NaHCO₃ 5% et concentrée. Après reprise au dichlorométhane et lavages à l'eau, la purification sur gel de silice [flash; éluant: toluène/acétate d'éthyle (85/15; v/v)] conduit à l'obtention de 1,32 g de 2-*O*-acétyl-4,6-di-*O-*benzyl-β-D-glucopyranoside de benzyle (M = 491).
CCM: Rf = 0,3 [toluène/acétate d'éthyle (8/2; v/v)].
Solide Blanc.
Rendement (%) = 59.
RMN ¹³C (CDCl₃, 100 MHz) δ (ppm): 170,96 (C=O); 138,12, 138,09, 137,37 (C quat. arom.); 128,63, 128,49, 128,44, 128,13, 128,07, 127,90, 127,82, 127,78, 127,69 (C arom.); 99,47 (C1); 78,34 (C3); 75,98 (C4); 74,98 (C5); 74,87 (CH₂-Ar);74,40 (C2); 73,64 (CH₂-Ar); 70,53 (CH₂-Ar sur Cl); 68,77 (C6); 21,03 (CH3).
RMN ¹H (CDCl₃, 400 MHz) δ (ppm): 7,38-7,23 (m, 15H, H arom.); 4,91 (d, 1H, C*H₂*-Ar, ²j = 12,4 Hz); 4,88 (dd, 1H, H2, J_{H2-H1}= 7,9 Hz, J_{H2-H3} = 9,4 Hz); 4,78 (d, 1 H, *CH*₂-Ar, ²J = 11,2 Hz); 4,66 (d, 1H, C*H₂*-Ar, ²J = 12,2 Hz); 4,62 (d, 1H, C*H*₂-Ar, ²J = 12,2 Hz); 4,62 (d, 1H, C*H*₂-Ar, ²J = 11,4 Hz); 4,58 (d, 1H, C*H₂*-Ar, ²J = 12,1 Hz); 4,46 (d, 1H, H1, J_{H1-H2} = 7,9 Hz); 3,79 (dd, 1H, H6a, J_{H6a-H5} = 2,2 Hz, J_{H6a-H6b} = 10,9 Hz); 3,74 (dd, 1H, H6b, J_{H6b-H5} = 4,4 Hz, J_{H6b-H6a} = 10,9 Hz); 3,72 (t, 1H, H3, J_{H3-H2} = J_{H3-H4} = 9,1 Hz); 3,60 (dd, 1H, H4, J_{H4-H3} = 8,8 Hz, J_{H4-H5} = 9,6 Hz); 3,45 (ddd, 1H, H5, J_{H5-H4} = 9,6 Hz, J_{H5-H6a} = 2,2 Hz, J_{H5-H6b} = 4,4 Hz); 2,39 (d, 1 H, OH, J = 4,1 Hz), 2,09 (s, 3H, CH₃).

### EXEMPLE 16

### Synthèse d'un disaccharide selon l'invention.

Cet exemple illustre l'intérêt du procédé conforme à l'invention pour la préparation d'un disaccharide par réaction de couplage entre deux monosaccharides, le disaccharide obtenu pouvant lui-même servir de précurseur d'un disaccharide accepteur de glycosyle (voir exemple 17) pour la synthèse d'un trisaccharide (voir exemple 18).

### Synthèse du 2-O-acétyl-3-O-allyl-4,6-di-O-benzyl-β-D-glucopyranosyl-(1→3)-2-O-acétyl-4,6-di-O-benzyl-β-D-glucopyranoside de benzyle.

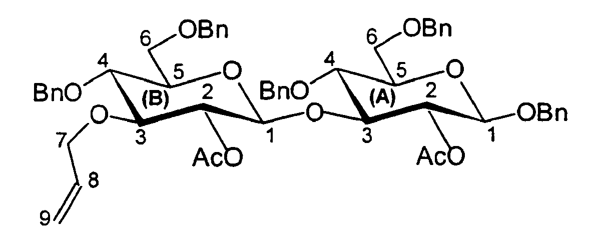

Dans 30 mL de dichlorométhane anhydre à 0°C en présence de tamis moléculaire 4Å (3 g) sont introduits 1,73 g (1,1 éq.) de 2-O-acétyl-3-O-allyl-4,6-di-*O*-benzyl-α-D-glucopyranosyl trichloroacétimidate préparé à l'exemple 14 (M = 586,5) et 1,32 g (1 éq.) de 2-*O*-acétyl-4,6-di-*O*-benzyl-β-D-glucopyranoside de benzyle préparé à l'exemple 15 (M = 491) puis 150 µL (0,25 éq.) de TESOTf. Après 1 h de réaction à 0°C, le milieu est neutralisé avec de la triéthylamine, filtré et concentré. Le produit est purifié sur gel de silice [flash; éluant: toluène/acétate d'éthyle (95/5; v/v)] et on recueille ainsi 1,87 g de 2-*O*-acétyl-3-*O*-allyl-4,6-di-*O-*benzyl-β-D-glucopyranosyl-(1→3)-2-*O*-acétyl-4,6-di-*O*-benzyl-[3-D-glucopyranoside de benzyle (M = 915).
CCM: Rf= 0,3 [toluène/acétate d'éthyle (9/1; v/v)].
Huile incolore.
Rendement (%) = 76.
RMN ¹³C (CDCl₃, 100 MHz) δ (ppm): 170,04, 169,12 (C=O); 138,46, 138,28, 138,22, 137,84, 137,38 (C quat. arom.); 134,64 (C8B); 128,54, 128,45, 128,44, 128,40, 128,36, 128,22, 128,17, 128,00, 127,83, 127,77, 127,69, 127,63, 127,56 (C arom.); 117,10 (C9B); 100,86 (C1B); 99,22 (C1A); 82,95 (C3B); 80,44 (C3A); 77,93 (C4B); 75,88 (C4A); 75,50 (C5B); 75,15 (C5A); 75,10 (CH₂-Ar); 74,98 (CH₂-Ar); 73,99 (C7B); 73,63 (C2A); 73,53 (CH₂-Ar); 73,48 (CH₂-Ar); 73,05 (C2B); 70,28 (CH₂-Ar sur C1A); 69,14, 69,04 (C6A, C6B); 21,07, 20,96 (CH3).
RMN ¹H (CDCl₃, 400 MHz) δ (ppm): 7,35-7,18 (m, 25H, H arom.); 5,85 (ddt, 1H, H8B,J_{H8B-H7aB} = J_{H8B-H7bB} = 5,7 Hz, J_{H8B-H9aB} = 17,2 Hz, J_{H8B-H9bB} = 10,4 Hz); 5,23 (ddt, 1H, H9aB, J_{H9aB-H7B} = 1,6 Hz, J_{H9aB-H8B} = 17,2 Hz, J_{H9aB-H9bB} = 1,7 Hz); 5,14 (ddt, 1H, H9bB, J_{H9bB-H7B} = 1,2 Hz, J_{H9bB-H8B} = 10,4 Hz, J_{H9bB-H9aB} = 1,5 Hz); 5,03 (d, 1H, C*H*₂-Ar, ²J = 11,0 Hz); 5,00 (dd, 1H, H2A, J_{H2A-H1A} = 8,0 Hz, J_{H2A-H3A} = 9,6 Hz); 4,95 (dd, 1H, H2B, J_{H2B-H1B} = 8,2 Hz, J_{H2B-H3B} = 9,6 Hz); 4,88 (d, 1H, C*H*₂-Ar sur C1, ²J = 12,4 Hz); 4,78 (d, 1H, C*H*₂-Ar, ²J = 10,8 Hz); 4,58 (d, 1H, C*H₂*-Ar, ²J = 12,1 Hz); 4,57 (d, 1H, H1B, J_{H1B-H2B} = 8,0 Hz); 4,57 (d, 1H, C*H*₂-Ar sur C1A, ²J = 13,4 Hz); 4,54 (d, 1H, C*H₂*-Ar, ²J = 12,2 Hz); 4,52 (d, 1H, C*H₂*-Ar, ²J = 10,7 Hz); 4,47 (d, 1H, C*H₂-*Ar, ²J = 10,9 Hz); 4,41 (d, 1H, C*H₂*-Ar, ²J = 12,1 Hz); 4,36 (d, 1H, C*H₂*-Ar, ²J = 14,0 Hz); 4,33 (d, 1H, H1A, J_{H1A-H2A} = 8,0 Hz); 4,25 (ddt, 1H, H7aB, J_{H7aB-H7bB} = 12,6 Hz, J_{H7aB-H8B} = 5,6 Hz, J_{H7aB-H9B} = 1,4 Hz); 4,11 (ddt, 1H, H7bB, J_{H7bB-H7aB =} 12,6 Hz, J_{H7bB-H8B} = 5,9 Hz, J_{H7bB-H9B} = 1,4 Hz); 3,94 (dd, 1H, H3A, J_{H3A-H2A} = 9,3 Hz, J_{H3A-H4A} = 8,8 Hz); 3,74 (dd, 1H, H6aA ou H6aB, J_{H6a-H5} = 2,0 Hz, J_{H6a-H6b} = 10,8 Hz); 3,73 (dd, 1H, H6aA ou H6aB, J_{H6a-H5} = 1,6 Hz, J_{H6a-H6b} = 10,7 Hz); 3,64 (dd, 1H, H6bA ou H6bB, J_{H6b-H5} = 5,1 Hz, J_{H6b-H6a} = 10,8 Hz); 3,61 (t, 1H, H4B, J_{H4B-H3B} = J_{H4B-H5B} = 9,2 Hz); 3,56 (dd, 1H, H4A, J_{H4A-H3A} =8,9 Hz, J_{H4A-H5A} = 9,4 Hz); 3,53 (dd, 1H, H6bA ou H6bB, J_{H6b}-_{H5} = 5,2 Hz, J_{H6b}-_{H6a} = 10,9 Hz); 3,47 (t, 1H, H3B, J_{H3B}-_{H2B} = J_{H3B-H4B} = 9,2 Hz); 3,47-3,41 (m, 2H, H5A, H5B), 2,10 (s, 3H, CH₃); 2,05 (s, 3H, CH₃).

### EXEMPLE 17

### Synthèse d'un disaccharide accepteur de glycosyle selon l'invention.

### Synthèse du 2-O-acétyl-4,6-di-O-benzyl-β-D-glucopyranosyl-(1→3)-2-O-acétyl-4,6-di-O-benzyl-β-D-glucopyranoside de benzyle.

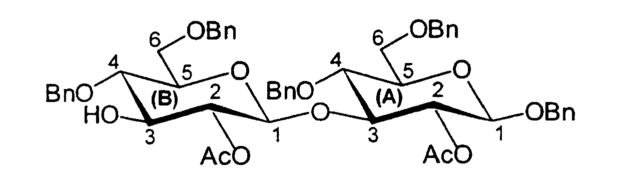

1,28 g ( 1 éq.) de 2-*O*-acétyl-3-*O*-allyl-4,6-di-*O*-benzyl-β-D-glucopyranosyl-(1→3)-2-*O*-acètyl-4,6-di-*O*-benzyl-β-D-glucopyranoside de benzyle (M = 915) et 1,18 g de Dabco sont dissous dans 50 ml d'un mélange éthanol/toluène/eau (8/3/1; v/v/v) puis additionnés de 390 mg (0,3 éq.) de catalyseur de Wilkinson. Le milieu réactionnel est chauffé à reflux pendant 1h30 puis mis à sec. Le mélange est alors repris au dichlorométhane et extrait à l'eau, avec HCl 1/2N glacé, avec NaHCO₃ 5% et encore à l'eau, séché et concentré. Le résidu est ensuite redissous dans 30 ml d'un mélange acétone/HCl aq. 10% (19/1; v/v) que l'on porte à reflux pendant 8 min. Après refroidissement rapide à T.A., la solution est neutralisée par quelques gouttes de NaHCO₃ 5% et concentrée. On dilue de nouveau au dichlorométhane et on extrait à l'eau. Le produit est finalement purifié sur gel de silice [flash; éluant: toluène/acétate d'éthyle (85/15; v/v)] ce qui permet d'isoler ainsi 600 mg de 2-*O*-acétyl-4,6-di-*O*-benzyl-β-D-glucopyranosyl-( 1→3)-2-*O*-acétyl-4,6-di-*O*-benzyl-β-D-glucopyranoside de benzyle (M = 875).
CCM: Rf = 0,3 [toluène/acétate d'éthyle (8/2; v/v)].
Huile incolore.
Rendement (%) = 49.
RMN ¹³C (CDCl₃, 100 MHz) δ (ppm): 171,65, 169,09 (C=O); 138,49, 138,24, 137,99, 137,37 (C quat. arom.); 128,61, 128,43, 128,39, 128,29, 128,23, 128,14, 128,07, 127,78, 127,76, 127,66, 127,61, 127,59, 127,57 (C arom.); 100,64 (C1B); 99,22 (C1A); 80,70 (C3A); 78,50 (C4B); 76,39 (C3B); 75,96 (C4A); 75,31, 75,17 (C5A, C5B); 74,97, 74,89 (CH₂-Ar); 74,31 (C2B); 73,53, 73,51, (C2A, 2 CH₂-Ar); 70,25 (CH₂-Ar sur C1A); 69,16, 69,01 (C6A, C6B); 21,07, 20,85 (CH3).
RMN ¹H (CDCl₃, 400 MHz) δ (ppm): 7,34-7,19 (m, 25H, H arom.); 5,04 (d, 1H, C*H*₂-Ar, ²J = 10,1 Hz); 5,03 (dd, 1H, H2A, J_{H2A-H1A} = 8,1 Hz, J_{H2A-H3A} = 9,8 Hz); 4,88 (d, 1H, C*H*₂-Ar sur C1A, ²J = 12,4 Hz); 4,80 (dd, 1H, H2B, J_{H2B-H1B} = 8,1 Hz, J_{H2B-H3B} = 9,4 Hz); 4,76 (d, 1H, C*H*₂-Ar, ²J = 11,1 Hz); 4,62 (d, 1H, H1B, J_{H1B-H2B} = 8,5 Hz); 4,60 (d, 1H, C*H*₂-Ar, ²J = 11,5 Hz); 4,58 (d, 1H, C*H*₂-Ar ²J = 12,2 Hz); 4,57 (d, 1H, C*H*₂-Ar sur C1A, ²J = 12,3 Hz); 4,53 (d, 1H, C*H*₂-Ar, ²J = 12,2 Hz); 4,47 (d, 1H, C*H*₂-Ar, ²J = 11,0 Hz); 4,40 (s, 2H, C*H*₂-Ar); 4,34 (d, 1H, H1A, J_{H1A-H2A} = 8,0 Hz); 3,95 (t, 1H, H3A, J_{H3A-H2A} = J_{H3A-H4A} = 9,1 Hz); 3,76 (dd, 1H, H6aA ou H6aB, J_{H6a-H5} = 1,6 Hz, J_{H6a-H6b} = 11,6 Hz); 3,74 (dd, 1H, H6aA ou H6aB, J_{H6a-H5} = 1,7 Hz, J_{H6a-H6b} = 11,8 Hz); 3,69 (m, 1H, H3B); 3,65 (dd, 1H, H6bA ou H6bB, J_{H6b-H5} = 5,0 Hz, J_{H6b-H6a} = 10,8 Hz); 3,57 (t, 1H, H4A, J_{H4A-H3A} = J_{H4A-H5A} = 9,3 Hz); 3,57 (dd, 1H, H6bA ou H6bB, J_{H6b-H5} = 5,4 Hz, J_{H6b-H6a} = 11,3 Hz); 3,55 (t, 1H, H4B, J_{H4B-H3B} = J_{H4B-H5B} = 8,6 Hz); 3,43 (m, 2H, H5A, H5B); 2,49 (d, 1H, OH, J =4,1 Hz); 2,13 (s, 3H, CH₃); 2,04 (s, 3H, CH₃).

### EXEMPLE 18

### Synthèse d'un trisaccharide selon l'invention.

Cet exemple illustre l'intérêt du procédé conforme à la présente invention pour la préparation d'un trisaccharide par réaction de couplage entre un monosaccharide et un disaccharide, le trisaccharide obtenu pouvant lui-même servir de précurseur d'accepteur de glycosyle pour la synthèse d'un tétrasaccharide, et ainsi de suite.

### Synthèse du 2-O-acétyl-3-O-allyl-4,6-di-O-benzyl-β-D-glucopyranosyl-(1→3)-2-O-acétyl-4,6-di-O-benzyl-β-D-glucopyranosyl-(1→3 )-2-O-acétyl-4,6-di-O-benzyl-β-D-glucopyranoside de benzyle.

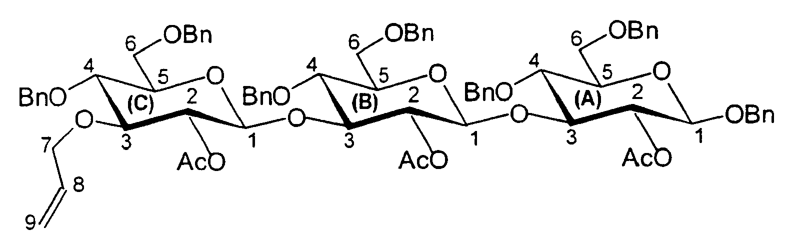

Dans 10 mL de dichlorométhane anhydre en présence de 1 g de tamis moléculaire 4Å à 0°C sont introduits 600 mg (1,1 éq.) de 2-*O*-acétyl-3-*O*-allyl-4,6-di-*O*-benzyl-α-D-glucopyranosyl trichloroacétimidate préparé à l'exemple 14 (M = 586,5) et 540 mg (1 éq.) de 2-*O*-acétyl-4,6-di-*O*-benzyl-β-D-glucopyranosyl-(1→3)-2-*O*-acétyl-4,6-di-*O*-benzyl-β-D-glucopyranoside de benzyle préparé à l'exemple 17 (M = 875) et additionnés ensuite de 35 µL (0,25 éq.) de TESOTf. Après 50 min de réaction à 0°C, le milieu est neutralisé avec de la triéthylamine, filtré et concentré. La purification sur gel de silice [flash; éluant: toluène/acétate d'éthyle (9/1; v/v)] conduit à l'obtention de 500 mg de 2-*O*-acétyl-3-*O*-allyl-4,6-di-*O*-benzyl-β-D-glucopyranosyl-(1→3)-2-*O*-acétyl-4,6-di-*O*-benzyl-β-D-glucopyranosyl-( 1→3)-2-*O*-acétyl-4,6-di-*O*-benzyl-β-D-glucopyranoside de benzyle (M = 1299).
CCM: Rf = 0,4 [toluène/acétate d'éthyle (9/1; v/v)].
Huile incolore.
Rendement (%) = 63.
RMN ¹³C (CDCl₃, 100 MHz) δ (ppm): 170,09, 169,44, 169,29 (C=O); 138,46, 138,43, 138,35, 138,20, 138,17, 137,85, 137,36 (C quat. arom.); 134,58 (C8C); 128,47-127,37 (C arom.); 117,13 (C9C); 101,13 (CIC); 100,43 (C1B); 99,21 (C1A); 82,92 (C3C); 80,96 (C3B); 80,23 (C3A); 77,88 (C4C); 76,13 (C4B); 75,89 (C4A); 75,45, 75,08, 75,02, 74,99, 74,96 (2 CH₂-Ar, C5A, C5B, C5C); 74,03 (C7C); 73,69, 73,52, 73,51, 73,46, 73,42, 72,95 (3 CH₂-Ar, C2A, C2B, C2C); 70,30 (CH₂-Ar sur C1A); 69,40, 69,18, 69,00 (C6A, C6B, C6C); 21,19, 21,02, 20,99 (CH3 ).
RMN ¹H (CDCl₃, 400 MHz) δ (ppm): 7,34-7,19 (m, 35H, H arom.); 5,85 (ddt, 1H, H8C, J_{H8C-H7aC} = J_{H8C-H7bC} = 5,7 Hz, J_{H8C-H9aC} = 17,2 Hz, J_{H8C-H9bC} = 10,4 Hz); 5,23 (ddt, 1H, H9aC, J_{H9aC-H7C} = 1,6 Hz, J_{H9aC-H8C} = 17,2 Hz, J_{H9aC-H9bC} = 1,7 Hz); 5,15 (ddt, 1H, H9bC, J_{H9bC-H7C} = 1,2 Hz, J_{H9bC-H8C} = 10,4 Hz, J_{H9bC-H9aC} = 1,5 Hz); 5,03 (d, 1H, C*H₂*-Ar, ²J = 10,7 Hz); 5,01 (d, 1H, C*H₂*-Ar, ²J = 10,1 Hz); 4,97 (dd, 1H, H2A, J_{H2A-H1A} = 8,0 Hz, J_{H2A-H3A} = 9,2 Hz); 4,96 (dd, 1H, H2C, J_{H2C-H1C} = 8,1 Hz, J_{H2C-H3C} = 9,4 Hz); 4,92 (dd, 1H, H2B, J_{H2B-H1B} = 8,2 Hz, J_{H2B-H3B} = 9,5 Hz); 4,87 (d, 1H, C*H₂*-Ar sur C1A, ²J = 12,4 Hz); 4,78 (d, 1H, C*H₂*-Ar, ²J = 10,6 Hz); 4,58-4,28 (m, 3H, C*H₂*-Ar); 4,56 (d, 1H, H1C, J_{H1C-H2C} = 8,1 Hz); 4,56 (d, 1H, C*H₂*-Ar sur C1A, ²J = 12,1 Hz); 4,52 (d, 1H, C*H₂*-Ar, ²J = 10,8 Hz); 4,49 (d, 1H, H1B, J_{H1B-H2B} = 8,0 Hz); 4,47 (d, 1H, *CH₂*-Ar, ²J = 10,8 Hz); 4,46 (d, 1H, C*H₂*-Ar, ²J = 11,2 Hz); 4,37 (d, 1H, C*H₂-*Ar*,* ²J = 12,3 Hz); 4,33 (d, 1H, C*H₂*-Ar, ²J = 11,9 Hz); 4,32 (d, 1H, H1A, J_{H1A-H2A} = 7,9 Hz); 4,30 (d, 1H, *CH₂-Ar,* ²J = 12,3 Hz); 4,24 (m, 1H, H7aC); 4,12 (ddt, 1H, H7bC, J_{H7bC-H7aC} = 12,6 Hz, J_{H7bC-H8C} = 5,9 Hz, J_{H7bC-H9C} = 1,4 Hz); 3,92 (t, 1H, H3A, J_{H3A-H2A} = J_{H3A-H4A} = 9,0 Hz); 3,89 (dd, 1H, H3B, J_{H3B-H2B} = 9,4 Hz, J_{H3B-H4B} = 8,6 Hz); 3,75-3,40 (m, 3H, H5A, H5B, H5C); 3,74-3,70 (m, 2H, H6); 3,64-3,59 (m, 2H, H6); 3,62 (t, 1H, H4C, J_{H4C-H3C} = J_{H4C-H5C} = 9,3 Hz); 3,53-3,40 (m, 2H, H6); 3,53 (t, 1H, H4A, J_{H4A-H3A} = J_{H4A-H5A} = 9,1 Hz); 3,51 (dd, 1H, H4B, J_{H4B-H3B} = 8,7 Hz, J_{H4B-H5B} = 9,4 Hz); 3,48 (t, 1H, H3C, J_{H3C-H2C} = J_{H3C-H4C} = 9,1 Hz); 2,12 (s, 3H, CH₃); 2,10 (s, 3H, CH₃); 2,02 (s, 3H, CH₃).

## Revendications

1. Procédé pour la préparation de dérivés fonctionalisés de β-(1,3)-glucanes comprenant une réaction entre un donneur de glycosyle et un accepteur de glycosyle, **caractérisé en ce que** le donneur de glycosyle est choisi dans le groupe constitué des composés de formules générales **Ia** et **Ib :** 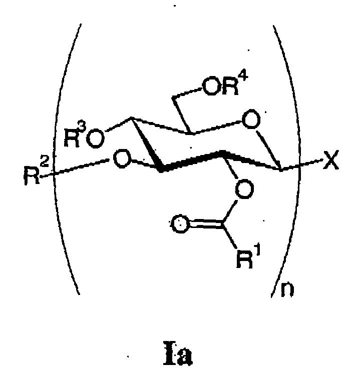 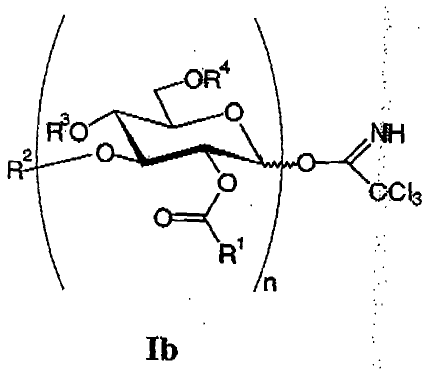 dans lesquelles :
X représente un groupe partant choisi parmi:
- un groupement de formule S(O)ₚRₐ, dans laquelle Rₐ représente un radical alkyle ayant 1 à 18 atomes de carbone, un radical 1,1-dicyclohexylméthyle, un radical aryle non substitué ou substitué par un groupement alkyle ou alcoxy ayant de 1 à 6 atomes de carbone, un groupement nitro ou acétamide et p est un nombre entier égal à 0 ou 1;
R¹ représente:
- un radical alkyle, halogénoalkyle ou cétoalkyle ayant de 1 à 6 atomes de carbone;
- un radical aryle non substitué ou substitué par un ou plusieurs groupements choisis parmi un atome d'halogène, un radical alcoxy ayant de 1 à 6 atomes de carbone ou un groupement nitro ;
R³ et R⁴, différents de -CO-R¹ et de R², représentent indépendamment un radical benzyle, chlorobenzyle, méthoxybenzyle, nitrobenzyle, allyle, méthylnaphtyle, chloroacétyle, trialkylsilyle ou triarylméthyle;
ou forment ensemble un radical éthylidyle, isopropylidyle, hexafluoroisopropylidyle, cyclopentylidyle, cyclohexylidyle, cycloheptylidyle, butylidyle, 1-tertiobutyléthylidyle, benzylidyle, méthoxybenzylidyle, 1-phénylbenzylidyle.
R² représente:
- un groupement différent de -COR¹ et choisi parmi un radical méthyle, allyle, méthylnaphthyle, benzyle, paraméthoxybenzyle ;
n est un nombre entier compris entre 1 et 4 ; étant précisé que dans le cas
où n est supérieur à 1, -COR¹, R³ et R⁴ peuvent être différents d'une unité glucosyle à l'autre ;
et **en ce que** l'accepteur de glycosyle est choisi dans le groupe constitué des composés de formule générale **II**: 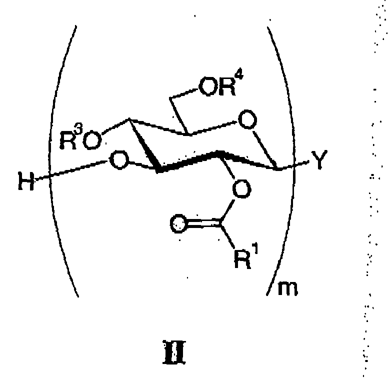 dans laquelle:
Y représente un groupe choisi parmi :
- un groupement de formule -O-R_{b} dans laquelle R_{b} représente un radical alkyle ayant de 1 à 24 atomes de carbone, alcényle ayant de 2 à 24 atomes de carbone ou un radical arylalkylaryle ou arylalkyle ayant de 6 à 18 atomes de carbone;
- un résidu de serine ou de thréonine ;
- un résidu de stérol ;
- un résidu de glycérolipide ;
- un groupement de formule -S- Rₐ dans laquelle Rₐ est tel que défini précédemment;
R¹, R³ et R⁴ sont tels que définis précédemment et ;
m est un nombre entier compris entre 1 et 8, étant précisé que dans le cas où m est supérieur à 1, -COR¹, R³ et R⁴ peuvent être différents d'une unité glucosyle à l'autre.

2. Procédé selon la revendication 1, **caractérisé en ce que** le donneur de glycosyle précité est choisi dans le groupe constitue des composés de formules générales (Ia) ou (Ib) précitées dans lesquelles :
X représente un groupe partant choisi parmi :
- un groupement de formule S(O)ₚRₐ, dans laquelle Rₐ représente un radical alkyle ayant 1 à 5 atomes de carbone, un radical aryle non substitué, de préférence un radical phényle ou un radial aryle substitué par un groupement alkyle ayant de 1 à 6 atomes de carbone; de préférence un radical toluyle, et p est un nombre entier égal à 0 ou 1;
R¹ représente :
- un radical alkyle ayant de 1 à 6 atomes de carbone, de préférence méthyle, ou un groupement lévulinyle;
- un radical aryle non substitué, de préférence un radical phényle;
R³ et R⁴, différents de -CO-R¹ et de R², représentent indépendamment un radical benzyle, chlorobenzyle, méthoxybenzyle, nitrobenzyle, allyle, méthylnaphtyle, chloroacétyle, trialkylsilyle ou triarylméthyle ;
ou forment ensemble un radical éthylidyle, isopropylidyle, benzylidyle ;
R² représente :
- un groupement différent de -COR¹ et choisi parmi un radical méthyle, allyle, méthylnaphthyle, benzyle, paraméthoxybenzyle ;
n est un nombre entier égal à 1, 2 ou 3; étant précisé que dans le cas où n est égal à 2 ou 3, -COR¹, R³ et R⁴ peuvent être différents d'une unité glucosyle à l'autre.

3. Procédé selon la revendication 2, **caractérisé en ce que** le donneur de glycosyle précité est choisi dans le groupe constitué des composés de formules générales (la) ou (Ib) précitées dans lesquelles :
X représente un groupe partant choisi parmi:
- un groupement de formule SRₐ dans laquelle Rₐ représente un radical éthyle, propyle, butyle ou phényle ou toluyle;
R¹ représente un radical méthyle ou phényle ;
R³ et R⁴ différents de -CO-R¹ et de R² représentent indépendamment un radical benzyle, méthoxybenzyle
ou forment ensemble un radical benzylidyle ;
R² représente un groupement différent de -COR¹ et choisi parmi un radical allyle, méthylnaphtyle ;
n est un nombre entier égal à 1 ou 2 ; étant précisé que dans le cas où n est égal à 2, -COR¹, R³ et R⁴ peuvent être différents d'une unité glucosyle à l'autre.

4. Procédé selon la revendication 3, **caractérisé en ce que** le donneur de glycosyle précité est choisi dans le groupe constitué des composés de formules générales (Ia) ou (1b) précitées dans lesquelles :
X représente un groupe partant choisi parmi :
- un groupement de formule SRₐ dans laquelle Rₐ représente un radical éthyle ou phényle ;
R¹ représente un radical méthyle ou phényle ;
R³ et R⁴ différents de -CO-R¹ et de R² représentent indépendamment un radical benzyle ou méthoxybenzyle,
ou forment ensemble un radical benzylidyle ;
R² représente un groupement différent de -COR¹ et choisi parmi un radical allyle, méthylnaphtyle ;
n est un nombre entier égal à 1.

5. Procédé selon l'une des revendications 1 à 4, **caractérisé en ce que** l'accepteur de glycosyle précité est choisi dans le groupe constitué des composés de formule générale (II) précitée dans laquelle:
Y représente un groupe choisi parmi :
un groupement de formule -O-R_{b} dans laquelle R_{b} représente un radical alkyle ayant de 1 à 24 atomes de carbone, alcényle ayant de 2 à 24 atomes de carbone ou un radical arylalkylaryle ou arylalkyle ayant de 6 à 18 atomes de carbone ;
- un résidu de sérine ou de thréonine ;
- un résidu de stérol ;
- un résidu de glycérolipide ;
- un groupement de formule -S- Rₐ dans laquelle Rₐ est tel que défini à la revendication 2;
R¹, R³ et R⁴ sont tels que définis à la revendication 2 et ;
m est un nombre entier compris entre 1 et 8, étant précisé que dans le cas où m est supérieur à 1, -COR¹, R³ et R⁴ peuvent être différents d'une unité glucosyle à l'autre.

6. Procédé selon la revendication 5, **caractérisé en ce que** l'accepteur de glycosyle précité est choisi dans le groupe constitué des composés de formule générale (II) précitée dans laquelle :
Y représente un groupe choisi parmi :
- un groupement de formule -O-R_{b} dans laquelle R_{b} représente un radical alkyle ayant de 1 à 24 atomes de carbone, alcényle ayant de 2 à 24 atomes de carbone ou un radical benzyle ;
R¹, R³ et R⁴ sont tels que définis à la revendication ;
m est un nombre entier compris entre 1 et 8, étant précisé que dans le cas
où m est supérieur à 1, -COR¹, R³ et R⁴ peuvent être différents d'une unité glucosyle à l'autre.

7. Procédé selon la revendication 6, **caractérisé en ce que** l'accepteur de glycosyle précité est choisi dans le groupe constitué des composés de formule générale (II) précitée dans laquelle :
R¹ représente un radical méthyle ou phényle ;
R³ et R⁴ différents de -CO-R¹ représentent indépendamment un radical benzyle, methoxybenzyle ou forment ensemble un radical benzylidyle.

8. Nouveau synthon accepteur de glycosyle pour la mise en oeuvre du procédé selon l'une des revendications 1 à 7, **caractérisé en ce qu'**il est choisi dans le groupe constitué des composés de formule générale **II**: 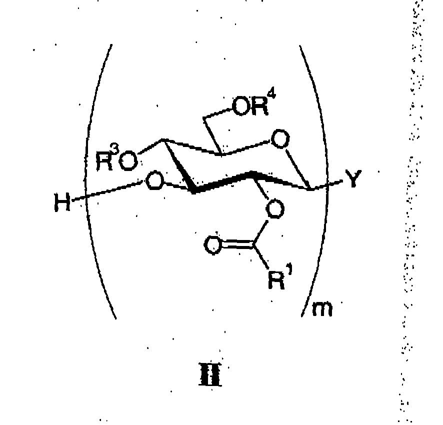 dans laquelle :
Y représente un groupe choisi parmi :
un groupement de formule -O-R_{b} dans laquelle R_{b} représente un radical alkyle ou alcényle ayant de 2 à 24 atomes de carbone ou un radical benzyle ;
R¹ représente:
- un radical alkyle ayant de 1 à 6 atomes de carbone, de préférence méthyle, ou un groupement lévulinyle ;
- un radical aryle non substitué, de préférence un radical phényle;
R³ et R⁴, différents de -CO-R¹ et de R², représentent indépendamment un radical benzyle, chlorobenzyle, méthoxybenzyle, nitrobenzyle, allyle, méthylnaphtyle, chloroacétyle, trialkylsilyle ou triarylméthyle;
ou forment ensemble un radical éthylidyle, isopropylidyle, benzylidyle ;
m est un nombre entier compris entre 1 et 8, étant précisé que dans le cas , où m est supérieur à 1, -COR¹, R³ et R⁴ peuvent être différents d'une unité glucosyle à l'autre.

9. Synthon accepteur de glycosyle selon la revendication 8, **caracterisé en ce qu**'il est choisi dans le groupe constitué des composés de formule générale (II) précitée dans laquelle :
Y représente un groupement --O-benzyle ;
R¹ représente un radical méthyle ou phényle ;
R³ et R⁴ différents de -CO-R¹ représentent indépendamment un radical benzyle, methoxybenzyle
ou forment ensemble un radical benzylidyle.

10. Nouveau synthon donneur de glycosyle pour la mise en oeuvre du procédé selon l'une des revendications 1 à 7, **caractérisé en ce qu'**il est choisi dans le groupe constitué :
a) des composés de formule générale la dans laquelle : 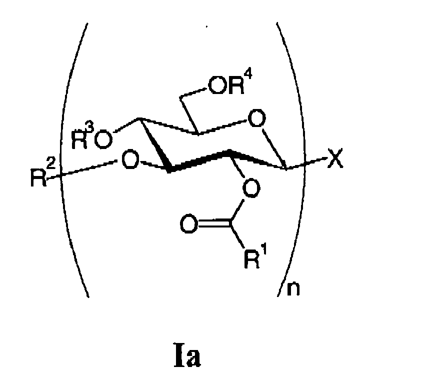
X représente un groupe partant choisi parmi :
- un groupement de formule SRₐ dans laquelle Rₐ représente un radical éthyle, propyle, butyle, phényle ou toluyle;
R¹ représente un radical méthyle ou phényle;
R³ et R⁴ différents de -CO-R¹ et de R² représentent indépendamment un radical benzyle, méthoxybenzyle
ou forment ensemble un radical benzylidyle ;
R² représente un groupement allyle ou méthylnaphtyle ;
n est un nombre entier égal à 1 ou 2 ; étant précisé que dans le cas où n est égal à 2, -COR¹, R³ et R⁴ peuvent être différents d'une unité glucosyle à l'autre ;
b) des composés de formule générale Ib 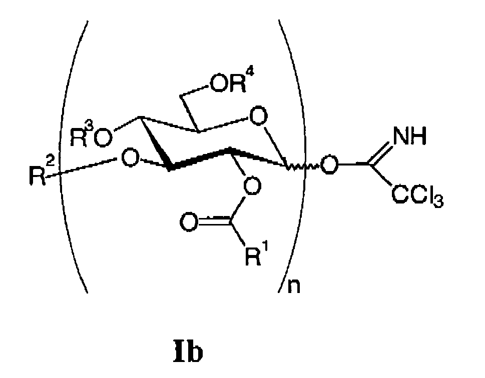
dans laquelle :
R¹, R³, R⁴ et n sont tels que définis ci-dessus et R² représente un groupement méthylnaphtyle.

11. Synthon donneur de glycosyle selon la revendication 10, **caractérisé en ce qu'**il est choisi dans le groupe constitué :
a) des composés de formule générale **Ia** précitée dans laquelle :
X représente un groupe partant choisi parmi :
- un groupement de formule SRₐ dans laquelle Rₐ représente un radical éthyle ou phényle ;
R¹ représente un radical méthyle ou phényle ;
R³ et R⁴ différents de -CO-R¹ et de R² représentent indépendamment un radical benzyle ou méthoxybenzyle,
ou forment ensemble un radical benzylidyle ;
R² représente un groupement allyle ou méthylnaphtyle ;
n est un nombre entier égal à 1 ;
b) des composés de formule générale Ib précitée dans laquelle :
R¹, R³, R⁴ et n sont tels que définis ci-dessus et R² représente un groupement méthylnaphtyle.

## Claims

1. A method of preparing functionalized β-(1,3)-glucans derivatives comprising a reaction between a glycosyl donor and a glycosyl acceptor, **characterised in that** the glycosyl donor is selected from the group consisting of the compounds of general formulae **Ia** and **Ib**: 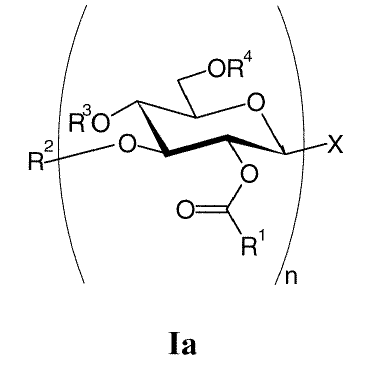 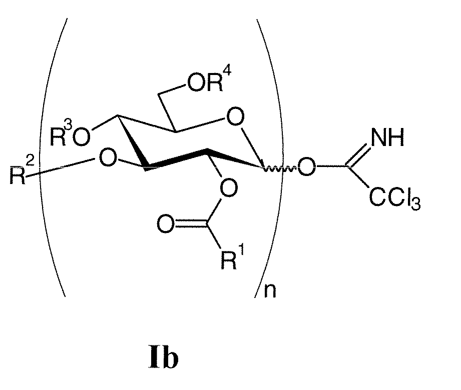 in which :
X represents a leaving group selected from:
- a group of formula S(O)ₚRₐ, in which Rₐ represents an alkyl radical having 1 to 18 carbon atoms, a 1,1-dicyclohexylmethyl radical, an aryl radical which is non-substituted or substituted by an alkyl or alkoxy group having 1 to 6 carbon atoms, a nitro or acetamide group, and p is an integer equal to 0 or 1 ;
R¹ represents:
- an alkyl, haloalkyl or ketoalkyl radical having 1 to 6 carbon atoms ;
- an aryl radical which is non-substituted or substituted with one or more groups selected from a halogen atom, an alkoxy radical having 1 to 6 carbon atoms or a nitro group;
R³ and R⁴, which are different from -CO-R¹ and from R², independently represent a benzyl, chlorobenzyl, methoxybenzyl, nitrobenzyl, allyl, methylnaphthyl, chloroacetyl, trialkylsilyl ou triarylmethyl radical ;
or together form an ethylidyl, isopropylidyl, hexafluoroisopropylidyl, cyclopentylidyl, cyclohexylidyl, cycloheptylidyl, butylidyl, 1-tert-butylethylidyl, benzylidyl, methoxybenzylidyl or 1-phenylbenzylidyl radical.
R² represents:
- a group which is different from -COR¹ and which is selected from a methyl, allyl, methylnaphthyl, benzyl or paramethoxybenzyl radical;
n is an integer of between 1 and 4 ; it being specified that in the case in which n is greater than 1, it is possible for -COR¹, R³ and R⁴ to be different from one glycosyl unit to another ;
and **in that** the glycosyl acceptor is selected from the group consisting of the compounds of general formula **II:** 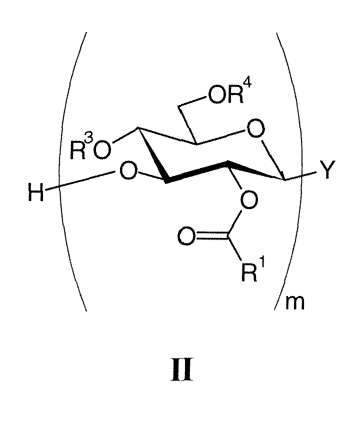 in which :
Y represents a group selected from:
- a group of formula -O-R_{b} in which R_{b} represents an alkyl radical having 1 to 24 carbon atoms, an alkenyl radical having 2 to 24 carbon atoms or an arylalkylaryl or arylalkyl radical having 6 to 18 carbon atoms;
- a serine or threonine residue ;
- a sterol residue ;
- a glycerolipid residue ;
- a group of formula -S-Rₐ in which Rₐ is as defined above;
R¹, R³ and R⁴ are as defined above and ;
m is an integer of between 1 and 8, it being specified that in the case in which m is greater than 1, it is possible for -COR¹, R³ and R⁴ to be different from one glycosyl unit to another.

2. The method according to claim 1, **characterised in that** the glycosyl donor cited above is selected from the group consisting of the compounds of general formulae (Ia) or (Ib) cited above in which :
X is a leaving group selected from:
- a group of formula S(O)ₚRₐ, in which Rₐ represents an alkyl radical having 1 to 5 carbon atoms, a non-substituted aryl radical, preferably a phenyl radical, or an aryl radical which is substituted with an alkyl group having 1 to 6 carbon atoms, preferably a toluyl radical, and p is an integer equal to 0 or 1 ;
R¹ represents :
- an alkyl radical having 1 to 6 carbon atoms, preferably a methyl radical or a levulinyl group;
- a non-substituted aryl radical, preferably a phenyl radical;
R³ and R⁴, which are different from -CO-R¹ and from R², independently represent a benzyl, chlorobenzyl, methoxybenzyl, nitrobenzyl, allyl, methylnaphthyl, chloroacetyl, trialkylsilyl ou triarylmethyl radical;
or together form an ethylidyl, isopropylidyl or benzylidyl radical ;
R² represents:
- a group which is different from -COR¹ and which is selected from a methyl, allyl, methylnaphthyl, benzyl or paramethoxybenzyl radical ;
n is an integer equal to 1, 2 or 3; it being specified that in the case in which n is equal to 2 or 3, it is possible for -COR¹, R³ and R⁴ to be different from one glycosyl unit to another.

3. The method according to claim 2, **characterised in that** the glycosyl donor cited above is selected from the group consisting of the compounds of general formulae (Ia) or (Ib) cited above in which:
X represents a leaving group selected from:
- a group of formula SRₐ, in which Rₐ represents an ethyl, propyl, butyl, phenyl or toluyl radical ;
R¹ represents a methyl or phenyl radical;
R³ and R⁴, which are different from -CO-R¹ and from R², independently represent a benzyl or methoxybenzyl radical
or together form a benzylidyl radical;
R² represents a group which is different from -CO-R¹ and which is selected from an allyl or methylnaphthyl radical;
n is an integer equal to 1 or 2 ; it being specified that in the case in which n is equal to 2, it is possible for -COR¹, R³ and R⁴ to be different from one glycosyl unit to another.

4. The method according to claim 3, **characterised in that** the glycosyl donor cited above is selected from the group consisting of the compounds of formulae (Ia) or (Ib) cited above in which:
X represents a leaving group selected from:
- a group of formula SRₐ, in which Rₐ represents an ethyl or phenyl radical;
R¹ represents a methyl or phenyl radical;
R³ and R⁴, which are different from -CO-R¹ and from R², independently represent a benzyl or methoxybenzyl radical,
or together form a benzylidyl radical;
R² represents a group which is different from -CO-R¹ and which is selected from an allyl or methylnaphthyl radical;
n is an integer equal to 1.

5. The method according to one of claims 1 to 4, **characterised in that** the glycosyl acceptor cited above is selected from the group consisting of the compounds of general formula (II) in which:
Y represents a group selected from:
- a group of formula -O-R_{b} in which R_{b} represents an alkyl radical having 1 to 24 carbon atoms, an alkenyl radical having 2 to 24 carbon atoms or an arylalkylaryl or arylalkyl radical having 6 to 18 carbon atoms;
- a serine or threonine residue;
- a sterol residue ;
- a glycerolipid residue ;
- a group of formula -S-Rₐ in which Rₐ is as defined in Claim 2 ;
R¹, R³ and R⁴ are as defined in Claim 2 and ;
m is an integer of between 1 and 8, it being specified that in the case in which m is greater than 1, it is possible for -COR¹, R³ and R⁴ to be different from one glycosyl unit to another.

6. The method according to claim 5 **characterised in that** the glycosyl acceptor is selected from the group consisting of the compounds of general formula (II) in which:
Y represents a group selected from:
- a group of formula -O-R_{b} in which R_{b} represents an alkyl radical having 1 to 24 carbon atoms, an alkenyl radical having 2 to 24 carbon atoms or a benzyl radical ;
R¹ , R³ and R⁴ are as defined in Claim 2;
m is an integer of between 1 and 8, it being specified that in the case in which m is greater than 1, it is possible for -COR¹, R³ and R⁴ to be different from one glycosyl unit to another.

7. The method according to claim 6, **characterised in that** the glycosyl acceptor is a compound of formula (II) in which :
R¹ is a methyl or phenyl radical;
R³ and R⁴, which are different from -CO-R¹ independently represent a benzyl or methoxybenzyl radical
or together form a benzylidyl radical.

8. A novel glycosyl acceptor synthon for implementing the method according to one of claims 1 to 7, **characterised in that** it is selected from the group consisting of the compounds of general formula II: 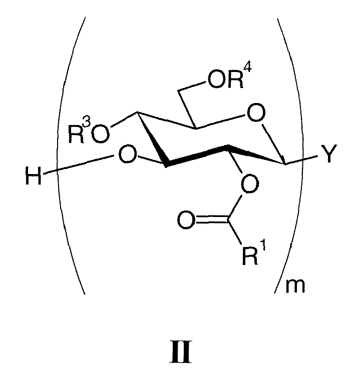 in which :
Y represents a group selected from:
- a group of formula -O-R_{b} in which R_{b} represents an alkyl radical or an alkenyl radical having 2 to 24 carbon atoms or a benzyl radical;
R¹ represents :
- an alkyl radical having 1 to 6 carbon atoms, preferably a methyl radical or a levulinyl group;
- an aryl radical which is non-substituted, preferably a phenyl radical;
R³ and R⁴, which are different from -CO-R¹ and from R², independently represent a benzyl, chlorobenzyl, methoxybenzyl, nitrobenzyl, allyl, methylnaphthyl, chloroacetyl, trialkylsilyl ou triarylmethyl radical ;
or together form an ethylidyl, isopropylidyl or benzylidyl radical ;
m is an integer of between 1 and 8, it being specified that in the case in which m is greater than 1, it is possible for -COR¹, R³ and R⁴ to be different from one glycosyl unit to another.

9. The glycosyl acceptor synthon according to claim 8, **characterised in that** it is selected from the group consisting of the compounds of general formula (II) in which :
Y represents an -O-benzyl group ;
R¹ represents a methyl or phenyl radical ;
R³ and R⁴, which are different from -CO-R¹, independently represent a benzyl or methoxybenzyl radical
or together form a benzylidyl radical.

10. A novel glycosyl donor synthon for implementing the method according to one of claims 1 to 7, **characterised in that** it is selected from the group consisting of:
a) the compounds of general formula **Ia:** 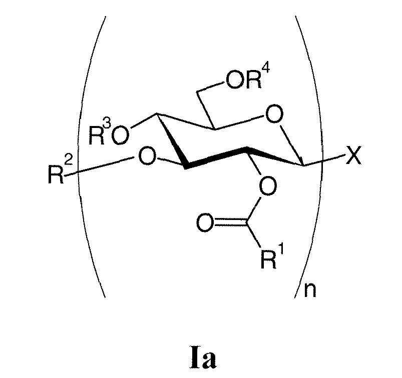 in which:
X is a leaving group selected from:
- a group of formula SRₐ, in which Rₐ represents an ethyl, propyl, butyl, phenyl or toluyl radical;
R¹ represents a methyl or phenyl radical ;
R³ and R⁴, which are different from -CO-R¹ and from R², independently represent a benzyl or methoxybenzyl radical
or together form a benzylidyl radical;
R² represents an allyl or methylnaphthyl radical ;
n is an integer equal to 1 or 2; it being specified that in the case in which n is equal to 2, it is possible for -COR¹, R³ and R⁴ to be different from one glycosyl unit to another;
b) the compounds of general formula **Ib**: 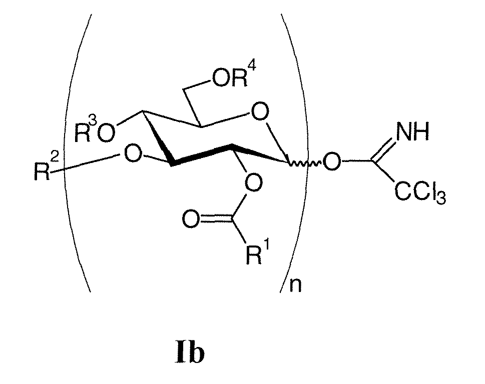
in which :
R¹, R³, R⁴ and n are as defined above and R² represents a methylnaphthyl group.

11. The glycosyl donor synthon according to claim 10 which is selected from:
a) the compounds of general formula **Ia** cited above in which:
X is a leaving group selected from:
- a group of formula SRₐ, in which Rₐ represents an ethyl or phenyl radical ;
R¹ represents a methyl or phenyl radical;
R³ and R⁴ which are different from -CO-R1 and from R2, independently represent a benzyl or methoxybenzyl radical,
or together form a benzylidyl radical;
R² represents an allyl or methylnaphthyl radical;
n is an integer equal to 1.
b) the compounds of general formula **Ib** cited above in which:
R¹, R³ and R⁴ and n are as defined above and R² represents a methylnaphthyl group.

## Patentansprüche

1. Verfahren zur Herstellung von funktionalisierten Derivaten von β-(1,3)-Glucanen, umfassend eine Reaktion unter einem Glycosyldonor und einem Glycosylakzeptor, **dadurch gekennzeichnet, daß** der Glycosyldonor gewählt ist aus der Gruppe, die besteht aus Verbindungen der allgemeinen Formeln la und Ib: 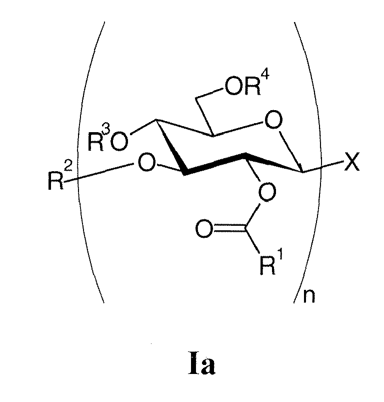 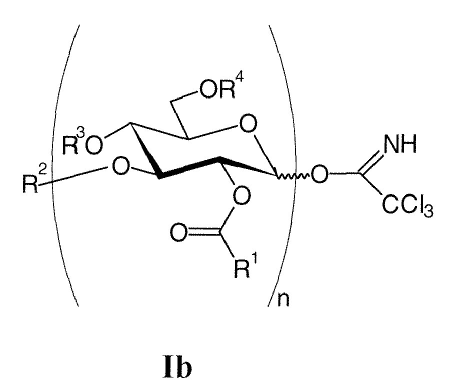 in denen:
X eine abgehende Gruppe ist, gewählt unter:
- einer Gruppe mit Formel S(O)_{P}Rₐ, in welcher Rₐ einen Alkylrest mit 1 bis 18 Kohlenstoffatomen, einen 1,1-Dicyclohexylmethylrest, einen Arylrest, der gegebenenfalls substituiert ist durch eine Alkyl- oder Alkoxygruppe mit 1 bis 6 Kohlenstoffatomen, eine Nitro- oder Acetamidgruppe darstellt und p eine ganze Zahl gleich 0 oder 1 darstellt;
R¹ darstellt:
- einen Alkyl-, Halogenalkyl- oder Ketoalkylrest mit 1 bis 6 Kohlenstoffatomen;
- einen Arylrest, der gegebenenfalls substituiert ist durch eine oder mehrere Gruppen, gewählt unter einem Halogenatom, einem Alkoxyrest mit 1 bis 6 Kohlenstoffatomen oder einer Nitrogruppe;
R³ und R⁴ verschieden von -CO-R¹ und R², unabhängig einen Benzyl-, Chlorbenzyl-, Methoxybenzyl-, Nitrobenzyl-, Allyl-, Methylnaphtyl-, Chloracetyl-, Trialkylsilyl- oder Triarylmethylrest darstellen; oder gemeinsam einen Ethidyl-, Isopropylidyl-, Hexafluorisopropylidyl-, Cyclopentylidyl-, Cyclohexylidyl-, Cycloheptylidyl-, Butylidyl-, 1-tert.-Butylethylidyl-, Benzylidyl-, Methoxybenzylidyl-, 1-Phenylbenzylidylrest bilden;
R² darstellt:
- eine Gruppe, die verschieden ist von -COR¹ und gewählt ist unter einem Methyl-, Allyl-, Methylnaphthyl-, Benzyl-, Paramethoxybenzylrest; n eine ganze Zahl zwischen 1 und 4 ist; wobei präzisiert ist, daß in dem Fall, wo n größer als 1 ist, -COR¹, R³ und R⁴ unterschiedlich von einer Glucosyleinheit zur anderen sein können;
und **dadurch**, daß der Glycosylakzeptor in der Gruppe gewählt ist, die besteht aus Verbindungen der allgemeinen Formel II: 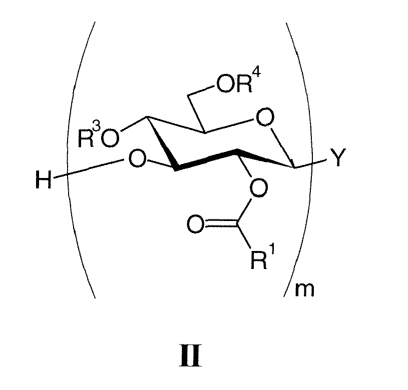 in welcher:
Y eine Gruppe darstellt, gewählt unter:
- einer Gruppe mit einer Formel -O-R_{b}, in der R_{b} einen Alkylrest mit 1 bis 24 Kohlenstoffatomen, Alkenylrest mit 2 bis 24 Kohlenstoffatomen oder einen Arylalkylaryl- oder Arylalkylrest darstellt mit 6 bis 18 Kohlenstoffatomen;
- einem Serin- oder Threoninrest;
- einem Sterolrest;
- einem Glycerolipidrest;
- einer Gruppe mit einer Formel -S-Rₐ, in der Rₐ wie oben definiert ist;
R¹, R³ und R⁴ wie oben definiert sind;
m eine ganze Zahl zwischen 1 und 8 ist, wobei präzisiert ist, daß in dem Fall, wo m größer als 1 ist, -COR¹, R³ und R⁴ unterschiedlich von einer Glucosyleinheit zur anderen sein können.

2. Verfahren nach Anspruch 1, **dadurch gekennzeichnet, daß** der genannte Glycosyldonor gewählt ist aus der Gruppe, die besteht aus Verbindungen mit vorgenannten allgemeinen Formeln (Ia) oder (Ib), in denen:
X eine abgehende Gruppe ist, gewählt unter:
- einer Gruppe mit Formel S(O)_{P}Rₐ, in welcher Rₐ einen Alkylrest mit 1 bis 5 Kohlenstoffatomen, einen Arylrest, der gegebenenfalls substituiert ist, vorzugsweise einen Phenylrest oder einen Arylrest, der substituiert ist durch eine Alkylgruppe mit 1 bis 6 Kohlenstoffatomen, vorzugsweise einen Toluylrest darstellt und p eine ganze gleich 0 oder 1 darstellt;
R¹ darstellt:
- einen Alkylrest mit 1 bis 6 Kohlenstoffatomen, vorzugsweise Methyl, oder eine Levulinylgruppe;
- einen Arylrest, der gegebenenfalls substituiert ist durch einen Phenylrest;
R³ und R⁴ verschieden von -CO-R¹ und R², unabhängig einen Benzyl-, Chlorbenzyl-, Methoxybenzyl-, Nitrobenzyl-, Allyl-, Methylnaphtyl-, Chloracetyl-, Trialkylsilyl- oder Triarylmethylrest darstellen; oder gemeinsam einen Ethidyl-, Isopropylidyl-, Benzylidylrest bilden;
R² darstellt:
- eine Gruppe, die verschieden ist von -COR¹ und gewählt ist unter einem Methyl-, Allyl-, Methylnaphthyl-, Benzyl-, Paramethoxybenzylrest; n eine ganze Zahl gleich 1, 2 oder 3 ist; wobei präzisiert ist, daß in dem Fall, wo n gleich 2 oder 3 ist, -COR¹, R³ und R⁴ unterschiedlich von einer Glucosyleinheit zur anderen sein können.

3. Verfahren nach Anspruch 2, **dadurch gekennzeichnet, daß** der genannte Glycosyldonor gewählt ist aus der Gruppe, die besteht aus den genannten Verbindungen der allgemeinen Formeln (Ia) oder (Ib), in denen:
X eine abgehende Gruppe darstellt, gewählt unter:
- einer Gruppe mit einer Formel SRₐ, in der Rₐ einen Ethyl-, Propyl-, Butyl- oder Phenyl- oder Toluylrest darstellt;
R¹ einen Methyl- oder Phenylrest darstellt;
R³ und R⁴ verschieden von -CO-R¹ und R² unabhängig einen Benzyl-, Methoxybenzylrest darstellen
R² eine Gruppe verschieden von -COR¹ ist und gewählt unter einem Allyl-, Methylnaphthylrest;
n eine ganze Zahl gleich 1 oder 2 ist; wobei präzisiert ist, daß in dem Fall, wo n gleich 2 ist, -COR¹, R³ und R⁴ verschieden von einer Glucosyleinheit zur anderen sein können.

4. Verfahren nach Anspruch 3, **dadurch gekennzeichnet, daß** der genannte Glycosyldonor gewählt ist aus der Gruppe, die besteht aus den genannten Verbindungen der allgemeinen Formeln (Ia) oder (Ib), in denen:
X eine abgehende Gruppe darstellt, gewählt unter:
- einer Gruppe mit einer Formel SRₐ, in der Rₐ einen Ethyl- oder Phenylrest darstellt;
R¹ einen Methyl- oder Phenylrest darstellt;
R³ und R⁴ verschieden von -CO-R¹ und R² unabhängig einen Benzyl-, Methoxybenzylrest darstellen
oder zusammen einen Benzylidylrest bilden;
R² eine Gruppe verschieden von -COR¹ ist und gewählt unter einem Allyl-, Methylnaphthylrest;
n eine ganze Zahl gleich 1 ist.

5. Verfahren nach einem der Ansprüche 1 bis 4, **dadurch gekennzeichnet, daß** der genannte Glycosylakzeptor gewählt ist aus der Gruppe, die besteht aus den genannten Verbindungen der allgemeinen Formel (II), in welcher:
Y eine Gruppe darstellt, gewählt unter:
- einer Gruppe mit einer Formel -O-R_{b}, in der R_{b} einen Alkylrest mit 1 bis 24 Kohlenstoffatomen, Alkenylrest mit 2 bis 24 Kohlenstoffatomen oder einen Arylalkylaryl- oder Arylalkylrest darstellt mit 6 bis 18 Kohlenstoffatomen;
- einem Serin- oder Threoninrest;
- einem Sterolrest;
- einem Glycerolipidrest;
- einer Gruppe mit einer Formel -S-Rₐ, in der Rₐ in Anspruch 2 definiert ist;
R¹, R³ und R⁴ wie in Anspruch 2 definiert sind und
m eine ganze Zahl zwischen 1 und 8 ist, wobei präzisiert ist, daß in dem Fall, wo m größer als 1 ist, -COR ¹ R³ und R⁴ unterschiedlich von einer Glucosyleinheit zur anderen sein können.

6. Verfahren nach Anspruch 5, **dadurch gekennzeichnet, daß** der genannte Glycosylakzeptor gewählt ist aus der Gruppe, die besteht aus den genannten Verbindungen der allgemeinen Formel (II), in der:
Y eine Gruppe darstellt, gewählt unter:
- einer Gruppe mit einer Formel -O-R_{b}, in der R_{b} einen Alkylrest mit 1 bis 24 Kohlenstoffatomen, Alkenylrest mit 2 bis 24 Kohlenstoffatomen oder einen Benzylrest darstellt;
R¹, R³ und R⁴ wie in Anspruch 2 definiert sind;
m ist eine ganze Zahl zwischen 1 und 8 ist, wobei präzisiert ist, daß in dem Fall, wo m größer als 1 ist, -COR¹, R³ und R⁴ unterschiedlich von einer Glucosyleinheit zur anderen sein können.

7. Verfahren nach Anspruch 6, **dadurch gekennzeichnet, daß** der genannte Glycosylakzeptor gewählt ist aus der Gruppe, die besteht aus den Verbindungen der genannten allgemeinen Formel (II), in der:
R¹ einen Methyl- oder Phenylrest darstellt;
R³ und R⁴ verschieden von -CO-R¹ unabhängig einen Benzyl-, Methoxybenzylrest darstellen oder gemeinsam einen Benzylidylrest bilden.

8. Neuer Synthonakzeptor von Glycosyl zum Ausführen des Verfahrens gemäß einem der Ansprüche 1 bis 7, **dadurch gekennzeichnet, daß** er aus der Gruppe gewählt ist, die besteht aus Verbindungen allgemeinen Formel II: 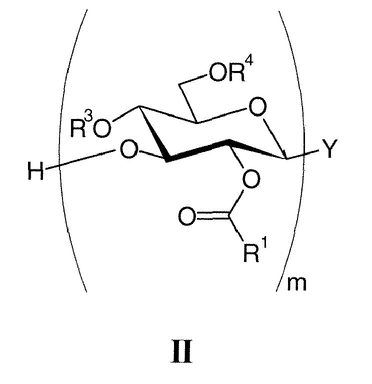 in welcher:
Y eine Gruppe darstellt, gewählt unter:
- einer Gruppe mit einer Formel -O-R_{b}, in der R_{b} einen Alkylrest mit 1 bis 24 Kohlenstoffatomen oder Alkenylrest mit 2 bis 24 Kohlenstoffatomen oder einen Benzylrest darstellt;
R¹ darstellt:
- einen Alkylrest mit 1 bis 6 Kohlenstoffatomen, vorzugsweise Methyl, oder eine Levurinylgruppe;
- einen Arylrest, der nicht substituiert ist, vorzugsweise einen Phenylrest;
R³ und R⁴ verschieden von -CO-R¹ und R², unabhängig einen Benzyl-, Chlorbenzyl-, Methoxybenzyl-, Nitrobenzyl-, Allyl-, Methylnaphtyl-, Chloracetyl-, Trialkylsilyl- oder Triarylmethylrest darstellen; oder gemeinsam einen Ethidyl-, Isopropylidyl-, Benzylidylrest bilden;
m eine ganze Zahl zwischen 1 und 8 ist, wobei präzisiert ist, daß in dem Fall, wo m größer als 1 ist, -COR¹, R³ und R⁴ unterschiedlich von einer Glucosyleinheit zur anderen sein können.

9. Synthonakzeptor von Glycosyl nach Anspruch 8, **dadurch gekennzeichnet, daß** er gewählt ist aus der Gruppe, die besteht aus Verbindungen der genannten allgemeinen Formel (II), in der:
Y eine -O-Benzylgruppe darstellt;
R¹ einen Methyl- oder Phenylrest darstellt;
R³ und R⁴ verschieden von -CO-R¹ unabhängig einen Benzyl-, Methoxybenzylrest darstellen
oder gemeinsam einen Benzylidylrest bilden.

10. Neuer Synthondonor von Glycosyl zur Ausführung des Verfahrens gemäß einem der Ansprüche 1 bis 7, **dadurch gekennzeichnet, daß** er gewählt ist aus der Gruppe, die besteht aus:
a) Verbindungen mit allgemeiner Formel la, in welcher: 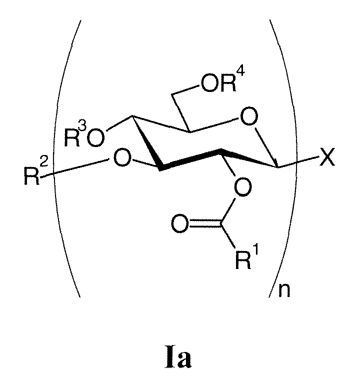
X eine abgehende Gruppe darstellt, gewählt unter:
- einer Gruppe mit einer Formel SRₐ, in der Rₐ einen Ethyl-, Propyl-, Butyl- oder Phenyl- oder Toluylrest darstellt;
R¹ einen Methyl- oder Phenylrest darstellt;
R³ und R⁴ verschieden von -CO-R¹ und R² unabhängig einen Benzyl-, Methoxybenzylrest darstellen
oder zusammen einen Benzylidylrest bilden;
R² eine Allyl- oder Methylnaphthylgruppe darstellt;
n eine ganze Zahl gleich 1 oder 2 ist; wobei präzisiert ist, daß in dem Fall, wo n gleich 2 ist, -COR¹, R³ und R⁴ verschieden von einer Glucosyleinheit zur anderen sein können;
b) Verbindungen der allgemeinen Formel Ib 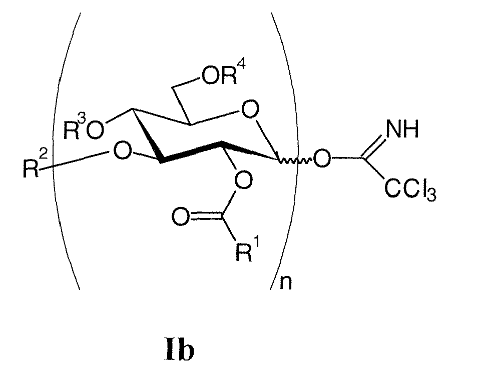
in welcher:
R¹, R³, R⁴ und n wie oben definiert sind und R² eine Methylnaphthylgruppe darstellt.

11. Synthondonor von Glycosyl nach Anspruch 10, **dadurch gekennzeichnet, daß** er gewählt ist aus der Gruppe, die besteht aus:
a) Verbindungen der genannten allgemeinen Formel la, in der:
X eine abgehende Gruppe darstellt, gewählt unter:
- einer Gruppe mit einer Formel SRₐ, in der Rₐ einen Ethyl- oder Phenylrest darstellt;
R¹ einen Methyl- oder Phenylrest darstellt;
R³ und R⁴ verschieden von -CO-R¹ und R² unabhängig einen Benzyl-, Methoxybenzylrest darstellen
oder zusammen einen Benzylidylrest bilden;
R² eine Allyl- oder Methylnaphthylgruppe darstellt;
n eine ganze Zahl gleich 1 ist;
b) Verbindungen der genannten allgemeinen Formel Ib, in der:
R¹, R³, R⁴ und n wie oben definiert sind und R² eine Methylnaphthylgruppe darstellt.
